# EUROPEAN PATENT SPECIFICATION

(11) **EP 1 660 500 B1**
(45) Date of publication and mention of the grant of the patent: **12.12.2007**
(21) Application number: 04763583.4
(22) Date of filing: 29.07.2004
(51) Int. Cl.: C07D 487/04, A61K 31/505, A61P 35/00

(54) **SUBSTITUTED IMIDAZOPYRIMIDINES FOR THE PREVENTION AND TREATMENT OF CANCER**
SUBSTITUIERTE IMIDAZOPYRIMIDINE ZUR PRÄVENTION UND BEHANDLUNG VON KREBS
IMIDAZOPYRIMIDINES SUBSTITUEES POUR LA PREVENTION ET LE TRAITEMENT DU CANCER

(30) Priority: 30.07.2003 ES 200301906
(43) Date of publication of application: 31.05.2006
(73) Proprietor: LABORATORIOS S.A.L.V.A.T., S.A., 08950 Esplugues de Llobregat (Barcelona) (ES)
(72) Inventor: CATENA RUIZ, Juan Lorenzo, E-08901 L'Hospitalet de Llobregat (ES); FARRERONS GALLEMI, Carles, E-08034 Mataro (ES); FERNANDEZ SERRAT, Anna, F-06800 Cagnes sur Mer (FR); SERRA COMAS, Carmen, E-08902 L'Hospitalet de Llobregat (ES); BALSA LÓPEZ, Dolors, E-08912 Badalona (ES); LAGUNAS ARNAL, Carmen, E-08903 L'Hospitalet de Llobregat (ES); SALCEDO ROCA, Carolina, E-08757 Corbera (ES); FERNÁNDEZ GARCÍA, Andrés, E-08034 Barcelona (ES)
(74) Representative: Rambelli, Paolo
(86) International application number: PCT/EP2004/008476
(87) International publication number: WO 2005/014598

(56) References cited:
- WO-A-00/08024
- US-A- 3 455 924
- BELL SC ET AL: "The synthesis and reactions of some imidazo[1,2-a]pyrimidines" JOURNAL OF THE AMERICAN CHEMICAL SOCIETY, vol. 82, 1960, pages 1469-1471, XP002308585 USA
- LE WANG ET AL: "Potent, orally, active heterocycle-based combretastatin A-4 analogues : synthesis, structure-activity relationship, pharmacokinetics, and in vivo antitumor activity evaluation" JOURNAL OF MEDICINAL CHEMISTRY, AMERICAN CHEMICAL SOCIETY. WASHINGTON, US, vol. 45, 2002, pages 1697-1711, XP002304485 ISSN: 0022-2623 cited in the application
- ALMANSA C ET AL: "Synthesis and SAR of a new series of cox-2 selective inhibitors: pyrazolo[1,5-a]pyrimidines" JOURNAL OF MEDICINAL CHEMISTRY, vol. 44, 2001, pages 350-361, XP002308586 cited in the application

## Description

The present invention relates to new compounds, and the use thereof for the chemoprevention and treatment of both precancerous lesions [e.g. familial adenomatous polyposis (FAP), and actinic keratoses (AKs)] and cancer (e.g. colorectal, prostate, breast, bladder or skin cancer).

### BACKGROUND ART

Colorectal cancer (CRC) is one of the most common cancers in the world, with an overall mortality exceeding 40%. About 15% of patients with CRC report a family history of the disease. Hereditary CRC generally develops from two syndromes: familial adenomatous polyposis (FAP) and hereditary nonpolyposis colorectal cancer (HNPCC). FAP is caused by germline mutations in the tumour suppressor gene adenomatous polyposis coli (APC) and is characterized by the early development of multiple adenomas in the large intestine. If these lesions are not removed, they may progress to carcinomas.

Approximately 90% of all CRC cases and deaths are thought to be preventable. The objective of chemopreventive strategies is to avoid the formation of adenomatous polyps and their subsequent progression to CRC. Chemopreventive agents can act at various levels, by increasing apoptosis, reducing cell proliferation, and/or decreasing carcinogen-induced DNA damage. A number of pharmacological agents have been studied for the prevention of CRC, including nonsteroidal antiinflammatory drugs (NSAIDs) and cyclooxygenase (COX)-2-selective inhibitors. The inhibition of COX-2 enzymatic activity underlies part of the preventative action of these compounds (there is an over expression of COX-2 in tumour cells), although COX-2-independent mechanisms have also been described (Hsu A.L. J. Biol. Chem. 2000, 275, 11397-403). Some NSAID derivatives that do not inhibit COX, retain their chemopreventive activity on precancerous and cancerous processes, thereby acting through a mechanism independent of COX inhibition (Piazza G.A. Cancer Res. 1997, 57, 2909-15).

Actinic keratosis (AK) and skin cancer are increasingly frequent dermatological diseases in the population. They appear in regions of chronic sun exposure such as the face and the back of the hands. Although the exact incidence of AK is unknown, 40-50% of Australians over 40 years of age harbour AK, and the incidence increases with age. There is strong evidence that AK can progress to squamous cell carcinoma (SCC), in fact, approximately 60% of SCC arise from pre-existing AK. The incidence of skin cancer is increasing due to many factors, including greater life expectancy of the population and increases in ambiental ultraviolet radiation. UV light induces molecular signalling pathways and results in specific genetic alterations (i.e. mutation of p53) that are likely critical to skin cancer development. It has been shown that celecoxib, a COX-2 inhibitor, reduces the development of murine UVB-induced skin tumours, although its precise mechanism of action has not been fully elucidated. Additionally, recent reports on cyclooxygenase knockout-fibroblasts confirm that some of the antiproliferative and antineoplastic effects of NSAIDs are independent of the inhibition of either COX-1 or COX-2 (Zhang X. J. Exp. Med. 1999, 190, 451-459).

The development of safe and effective NSAIDs for chemoprevention is complicated by the fact that severe toxicity may counteract the benefits of treatment with these drugs when administered to healthy individuals who have a low probability of developing the disease. Moreover, there is increasing concern due to the risk of serious gastrointestinal reactions, cardiovascular safety, and the potential for serious skin reactions and hypersensitivity reactions, related to the use of COX-2 inhibitors, that currently limits their clinical application to the prevention and/or treatment of precancerous lesions and cancer. Thus, the development of new, less toxic, and more efficient therapeutic agents for these pathologies is essential.

Among the many antiproliferative compounds proposed, there are some that are structurally similar to the present invention, such as those of the following formulae, described in documents WO 99/51590 and US 5700826, that are still in the development phase.

WO 00/08024 discloses some compounds that are structurally similar to those of the present invention. Such compounds are selective COX-2 inhibitors, and as such, are useful for the treatment of inflammation and cancer.

Wang and collaborators (J. Med. Chem. 2002, 45, 1697-1711) have described 4,5-diphenylimidazo derivatives having potent antitubulin and cytotoxic activity.

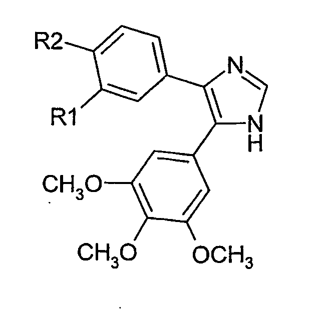

For the aforementioned reasons, it is necessary to provide new compounds for chemoprevention and treatment of both precancerous lesions and cancer.

### SUMMARY OF THE INVENTION

The compounds of the present invention inhibit the proliferation of, and induce apoptosis in cancer cell lines, through a COX-2 independent pathway, thereby minimizing the toxicity associated with COX-2 inhibition.

The present invention relates to a compound of general formula (I): 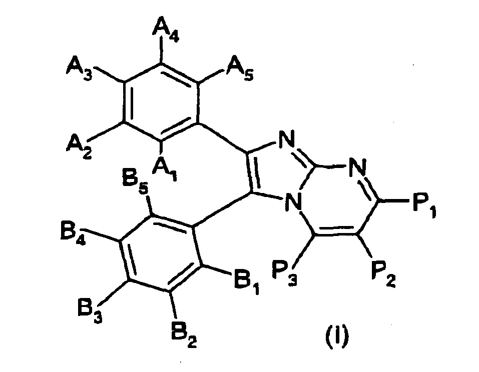 wherein:
A1, A2, A3, A4, A5, B1, B2, B3, B4 y B5 are radicals independently selected from the group consisting of H, (Cl-C4)-alkyl, (C3-C7)-cycloalkyl, CF3, OCF3, CN, (CH2)nOR1, (CH2)nNR1R2, CONR1R2, F, Cl, Br, I, NR1R2, NR2COR1, OR1, COR1, COOR1, COSR1, OCOR1, SR1, SOR1, S(O)OH, SO2R1, SO2NR2R3, SO2NHCOR1, and SCOR1; wherein n is an integer from 1 to 3; R1 is a radical selected from H, CH2OCOR2, CF3, (Cl-C4)-alkyl, and (C3-C7)-cycloalkylmethyl and (C3-C7)-cycloalkyl; R2 is a radical selected from H, and (C1-C4)-alkyl;
R3 is a radical selected from COR1, and SO2R1; alternatively, A2, A3, B2 or B3 may represent NO2; alternatively, either A2 and A3, or B2 and B3 may be forming a R4-(C1-C3)-alkyl-R5 biradical, wherein R4 and R5 are independently selected from CR1R2, O, NR1, S; P1, P2, and P3 are radicals independently selected from the group consisting of H, NR1R2, NR2COR1, CF3, F, Cl, Br, OH, SH, (C1-C4)-alkyl, (C1-C4)-alkoxyl and (C1-C4)-alkylsulfanyl.

In some documents of the prior art (cf. WO 00/08024; US 3455924; JP 01/43978; Almansa C. et al., J. Med. Chem. 2001, 44, 350-361; Bell S. C. et al., J. Amer. Chem. Soc. 1960, 82, 1469-1471; and Kaiser D. G. et al., J. Pharm. Sci 1975, 64, 2011-13, Kruglenko V.P. Chem. Heterocycl. Compounds, 1999, 35, 374; y Kruglenko V.P. et al. Ukr. Khim. Zh. 2001, 67, 108), some compounds included in the general formula (I) have been described chemically. Thus, compounds of formula (I) fulfilling any of the following circumstances are excluded from the scope of the protection of the present invention: simultaneously B3 is SO2NH2 or SO2CH3, A3, A4 or A5 are H, F, Cl, Br, (C1-C3)-alkyl, CF3, (C1-C3)-alkoxyl or OCF3, and P1 or P2 are H, CH3, Cl, Br or CH3O; or simultaneously B3 is CH3O or H, A3 is CH3O or H, and P1, P2 and P3 are H; or simultaneously B3 is F, A3 is SO2CH3, and P1 is methyl; or simultaneously A1, A2, A3, A4, A5, B1, B2, B3, B4, B5 are H, P1 is methyl, P2 is H and P3 is OH; or simultaneously A3 and B3 are CH30, A1, A2, A4, A5, B1, B2, B4 and B5 are H, and one of the groups P1, P2 or P3 are H, OH, (C1-C4)-alkyl or (C1-C4)-alkoxyl, being the remaining two groups P1, P2 or P3 representing H; or simultaneously A3 and B3 are CH3O, A1, A2, A4, A5, B1, B2, B4 and B5 are H,
P1 is OH or (C1-C4)-alkoxyl, P2 is H and P3 is (C1-C4)-alkyl.

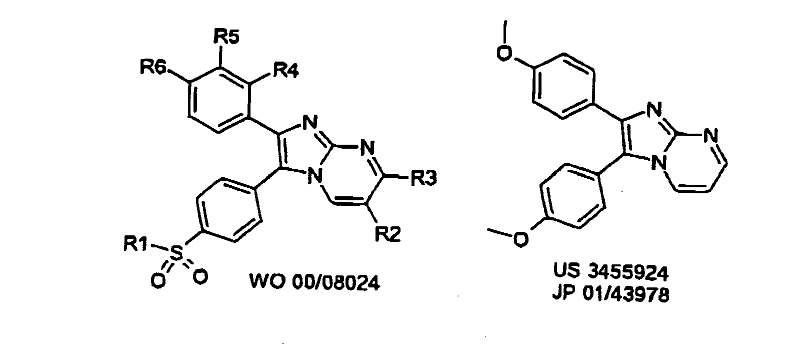

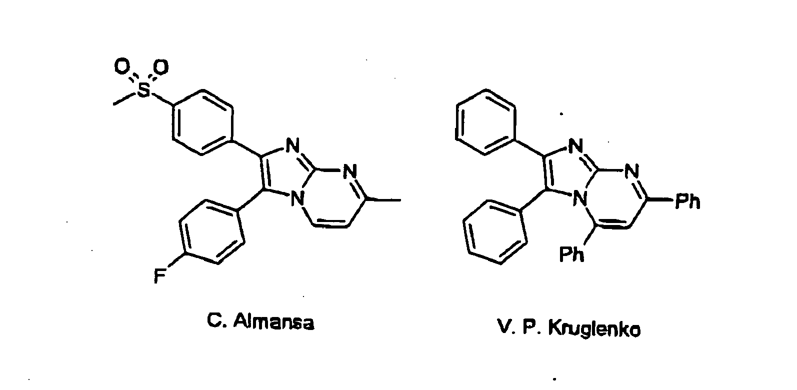

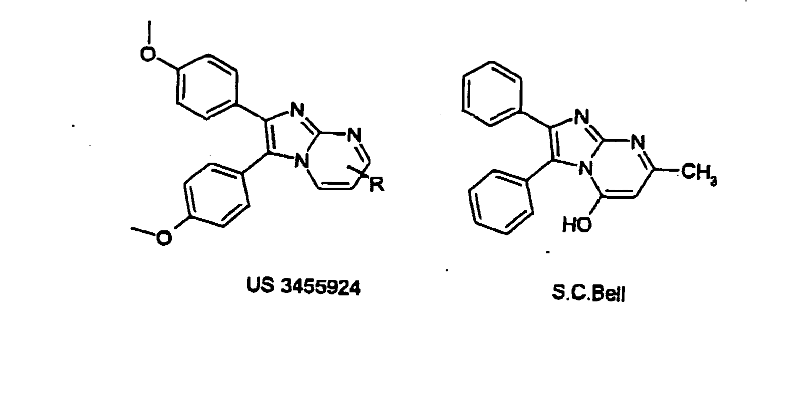

In a particular embodiment of this aspect of the invention, in the compounds of formula (I), A₃ and B₃ are radicals selected from H, (C₁-C₄)-alkyl, (C₃-C₇)-cycloalkyl, CF₃, OCF₃, CN, CONR1R2, F, Cl, Br, I, NR1R2, NR2COR1, OR1, COR1, COOR1, COSR1, OCOR1, SR1, SOR1, and SCOR1.In another particular embodiment B₃ is a radical selected from SR1 and SOR1.

Preferred compounds of the present invention include:
2-(4-methoxyphenyl)-3-(4-methylsulfanylphenyl)imidazo[1,2-a]pyrimidine;
2-(4-bromophenyl)-3-(4-methylsulfanylphenyl)imidazo[1,2-a]pyrimidine;
2-(4-methoxyphenyl)-7-methyl-3-(4-methylsulfanylphenyl)imidazo[1,2-a]pyrimidine;
2-(4-methanesulfonylphenyl)-7-methyl-3-p-tolylimidazo[1,2-a]pyrimidine;
3-(3-chloro-4-methylsulfanylphenyl)-2-(4-methylsulfanylphenyl)imidazo[1,2-a]pyrimidine;
3-(3-chloro-4-methylsulfanylphenyl)-2-(4-methoxyphenyl)imidazo[1,2-a]pyrimidine;
3-(3-methyl-4-methylsulfanylphenyl)-2-(4-methylsulfanylphenyl)imidazo[1,2-a]pyrimidine;
3-(3-chloro-4-methylsulfanylphenyl)-2-(4-methoxyphenyl)-7-methylimidazo[1,2-*a*]pyrimidine;
3-(3-bromo-4-methylsulfanylphenyl)-2-m-tolylimidazo[1,2-a]pyrimidine;
3-(3-bromo-4-methylsulfanylphenyl)-2-(4-chlorophenyl)imidazo[1,2-a]pyrimidine;
2-(4-methoxyphenyl)-3-(3-methyl-4-methylsulfanylphenyl)imidazo[1,2-a]pyrimidine;
3-(3-chloro-4-propylsulfanylphenyl)-2-(4-methoxyphenyl)imidazo[1,2-*a*]pyrimidine;
3-(4-isopropylsulfanylphenyl)-2-(4-methoxyphenyl)imidazo[1,2-a]pyrimidine;
3-(3-chloro-4-isopropylsulfanylphenyl)-2-p-tolylimidazo[1,2-a]pyrimidine;
3-(3-chloro-4-isopropylsulfanylphenyl)-2-(4-methylsulfanylphenyl)imidazo[1,2-a]pyrimidine; and
3-(3-chloro-4-methanesulfinylphenyl)-2-(4-methoxyphenyl)imidazo[1,2-a]pyrimidine.

Some of the compounds of formula (I) of the present invention may have one or more chiral centres. The present invention includes each one of the possible stereoisomers and mixtures thereof, particularly racemic mixtures thereof. A single enantiomer may be prepared by any of the commonly used processes, for example, by chromatographic separation of the racemic mixture on a stationary chiral phase, by resolution of the racemic mixture by fractional crystallization techniques of the diastereomeric salts, by chiral synthesis, by enzymatic resolution or by biotransformation.

Pharmaceutically acceptable salts include, among others, addition salts of inorganic acids such as hydrochloric, hydrobromic, nitric, sulphuric and phosphoric, as well as addition salts of organic acids such as acetic, benzenesulphonic, benzoic, camphorsulphonic, mandelic, methanesulphonic, oxalic, succinic, fumaric, tartaric, and maleic. Likewise, an acid proton in compounds of formula (I) may be substituted by a metallic ion, for example, an alkaline metal ion, an alkaline-earth metal ion or an aluminium ion; or may be coordinated with an organic or inorganic base. An acceptable organic base includes diethylamine and triethylamine. An acceptable inorganic base includes aluminium hydroxide, calcium hydroxide, potassium hydroxide, sodium carbonate, and sodium hydroxide. There may be more than one cation or anion depending on the number of functions with charge and on the valence of cations and anions.

Some of the compounds of formula (I) of the present invention may exist in unsolvated as well as solvated forms such as, for example, hydrates. The present invention encompasses all such above-mentioned forms that are pharmaceutically active. Some of the compounds of general formula (I) may exhibit polymorphism, encompassing the present invention all the possible polymorphic forms, and mixtures thereof.

Compounds of the present invention may be synthesized using the methods described below, as well as other processes known in the field of organic synthesis. Preferred methods include, but are not limited to, the general processes shown in the attached schemes.

According to Scheme 1, α-bromodesoxybenzoine (III) may be obtained by bromination of desoxybenzoine (II) by using Br₂/HBr/AcOH, Br₂/CCl₄, or CuBr₂ in ethyl acetate (EtOAc). The reaction of compound (III) with 2-aminopyrimidine (IV), in the presence of a base such as potassium carbonate or an excess of aminopyrimidine, gives a mixture of compounds (Ia) and (Ib).

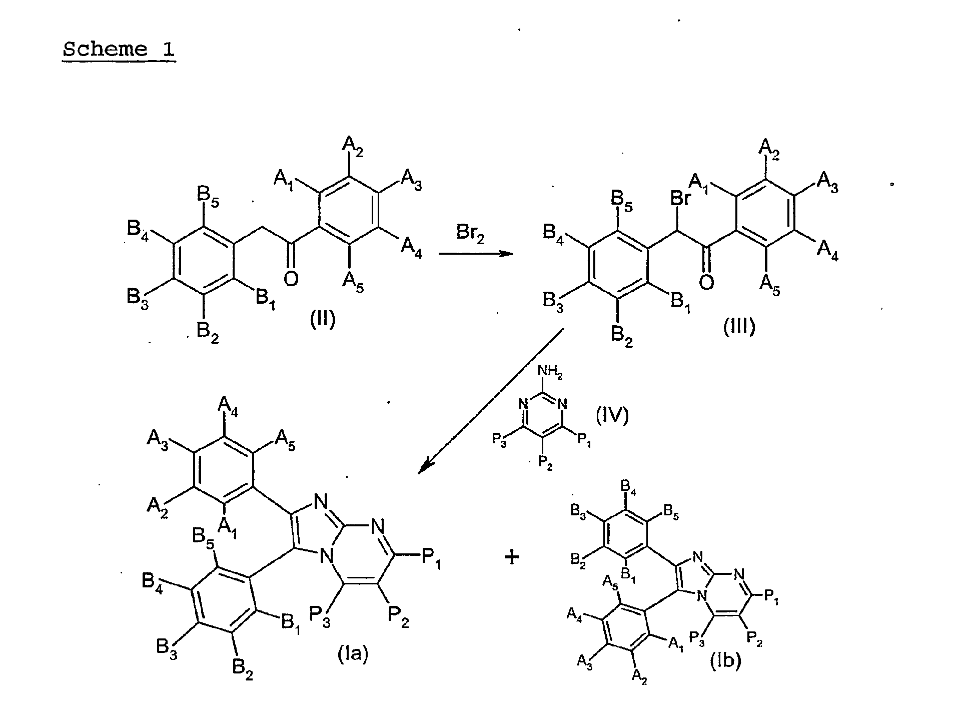

As shown in Scheme 2, the starting desoxybenzoine (II) may be prepared at least by one of these four different routes: route 1 consists in a Friedel-Crafts reaction of an aromatic substrate (VI) with a substituted phenacetyl chloride (V); route 2 consists in a Perkin condensation between a benzaldehyde (VIII) and a phenylacetic acid derivative (VII) to yield a 2,3-diphenylacrilic acid, followed by Curtius rearrangement, and subsequent hydrolytic treatment; route 3 consists in the addition of a benzylmagnesiane (IX) over benzaldehyde (VIII), and subsequent oxidation of the compound obtained; and route 4 consists in the addition of benzonitrile (X) to the benzylmagnesiane (IX).

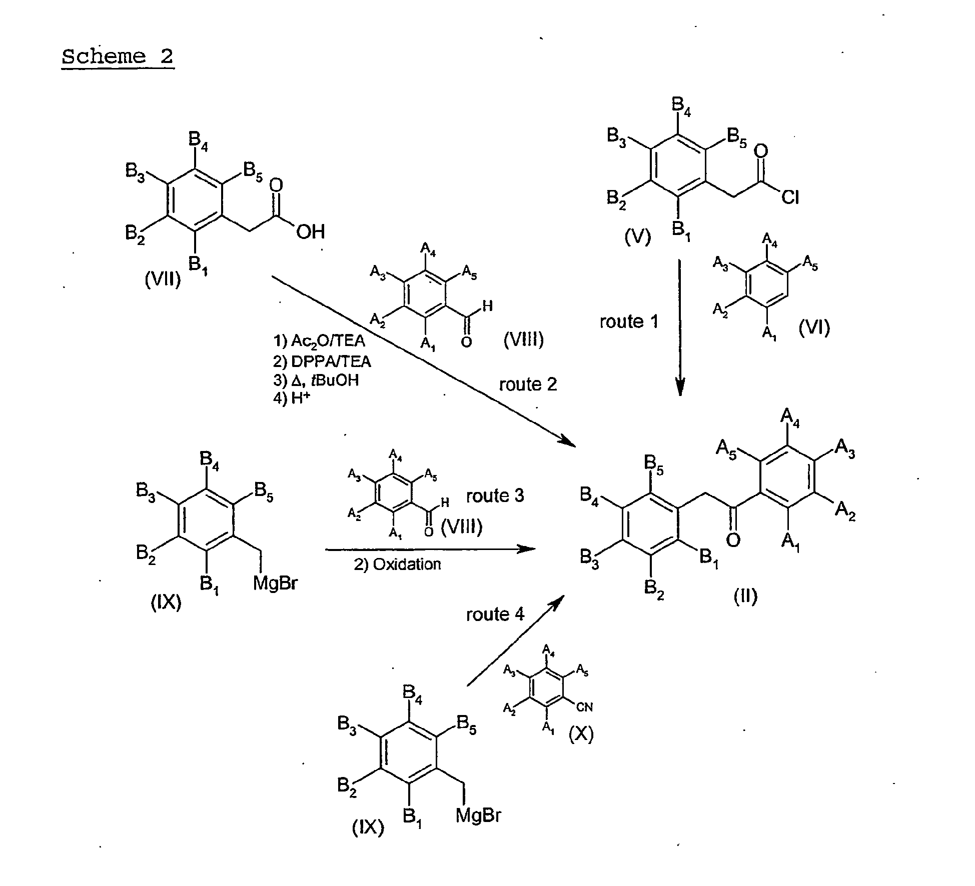

Alternatively, according to Scheme 3, compound (Ia) may be obtained by reaction of bromoacetophenone (XI) with 2-aminopyrimidine (IV), then selective bromination with *N-*bromosuccinimide (NBS) of the obtained compound (XII) to give compound (XIII), and subsequent Suzuki reaction with a suitable arylboronic acid (XIV) in the presence of Pd and a base.

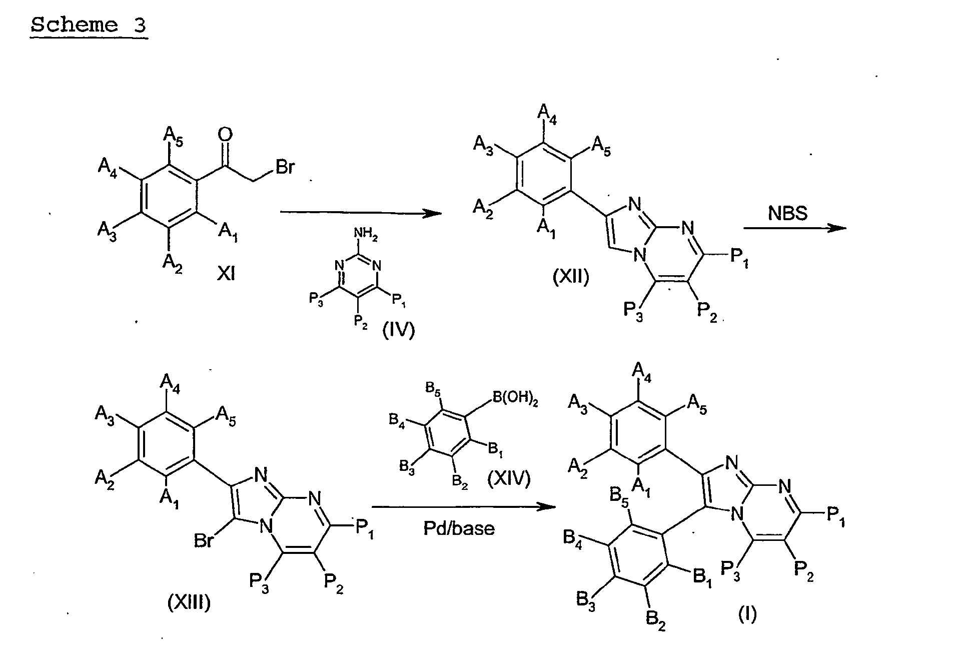

When in compound (I) either one of A₁ to A₅ or one of B₁ to B₅ (being the rest of them as defined above) is a methylsulfide, this may be transformed in the corresponding sulfonamide as shown in Scheme 4 (where only the sulfonamide substituent on ring A is represented, although the same applies for ring B, and where the rest of the positions on A and B ring may be substituted as defined above). Thus, the methylsulfide (XV) is oxidized with metachloroperbenzoic acid (*m*CPBA) to obtain the sulfoxide (XVI). Pummerer reaction of (XVI) affords the acetoxymethylthio (XVII), which can be oxidized with monoperoxyphthalic acid magnesium salt hexahydrate (MMPP) to give compound (XVIII). The treatment of (XVIII) with NaOH (1N) in MeOH allows to obtain the sulfinate (XIX). This is first treated with sulfuryl chloride in dichloromethane (DCM), and then with aqueous ammonium hydroxide in tetrahydrofurane (THF) to yield the sulfonamide (XX).

Compound (XVIII) may be also converted in the sulfonamide (XX) by treatment with NaOAc and K₂CO₃ followed by reaction with HOSA (hydroxylamina-*O*-sulfonic acid).

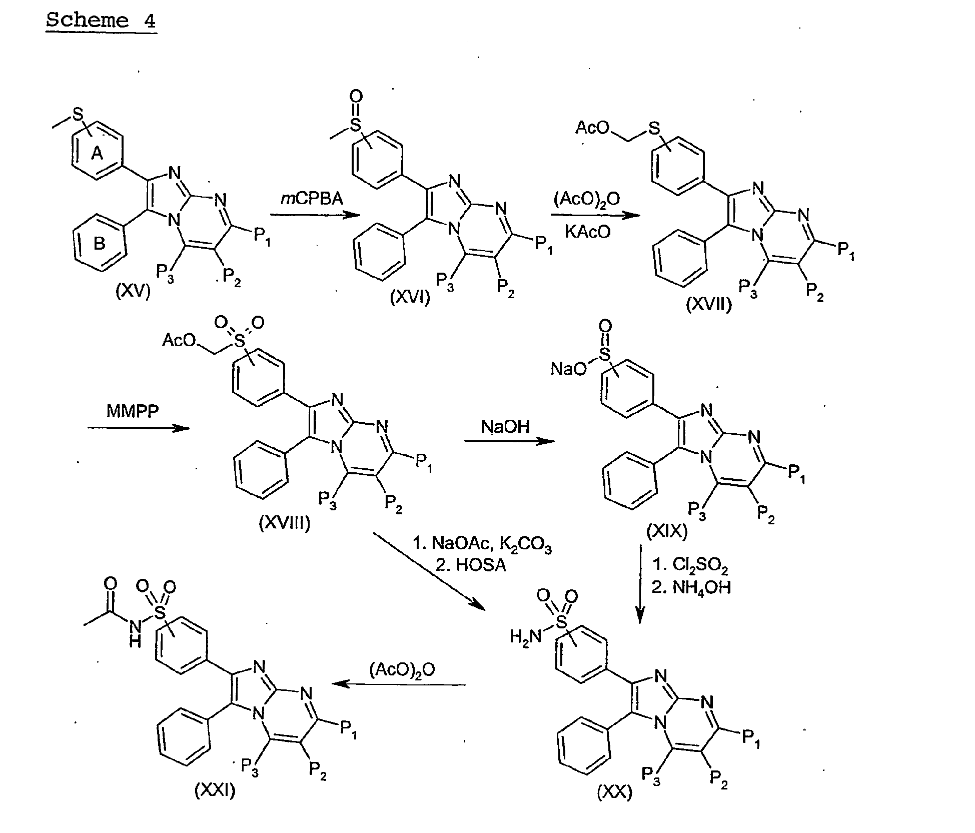

For purposes of simplicity, the possible substitituents in A and B rings in the scheme 4 above are not shown.

Alternatively, sulfonamide (XX) may be obtained starting from sulfoxide (XVI) by successive reactions with: a) TFAA (trifluoroacetic anhydride); b) triethylamine in MeOH; c) chlorine in acetic acid; and, finally, d) ammonium hydroxide.

N-acetylation of sulfamoyl group in (XX) with acetic anhydride in presence of triethylamine allows to obtain its corresponding acetyl derivative (XXI). On the other hand, the compound (XX) where B3 is a sulfonamide may also be obtained starting from compound (II) where B3 is H by chlorosulfonation and subsequent amination.

As shown in scheme 5, some substitutions on the pyrimidine ring (i.e. P1, P2 and P3), may be obtained by treatment of hydroxyimidazopyrimidine (XXII) with phosphorous oxychloride to yield the chloroderivative (XXIII), which then can be reacted either with thiourea to give the corresponding mercaptan (XXIV), or with an alcohol or amine to give the corresponding ether (XXV) or aminoderivative (XXVI), respectively.

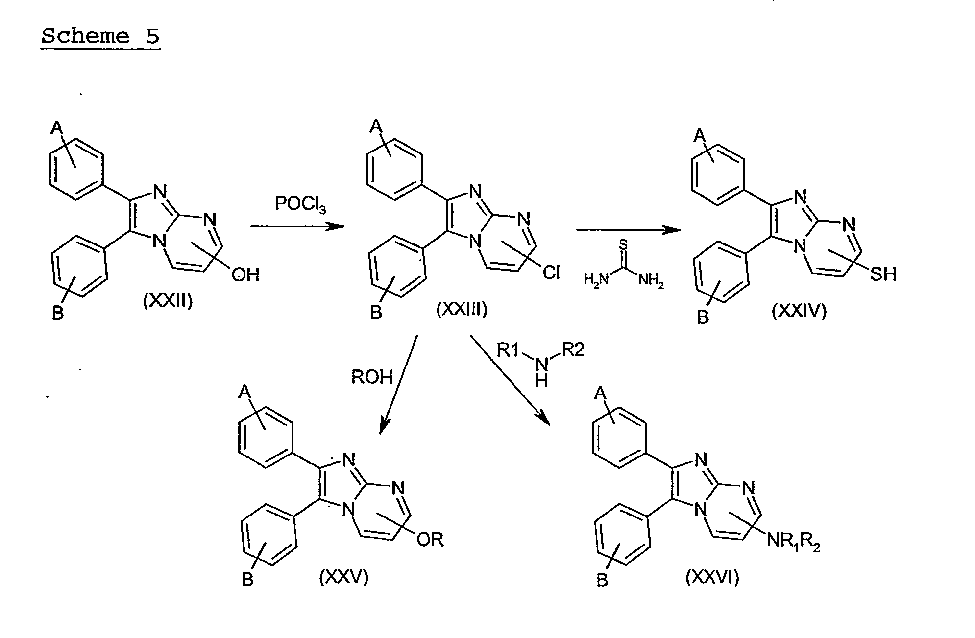

The compounds of the present invention may induce apoptosis of cancerous and/or precancerous cells. Thus, an aspect of the present invention relates to the use of said compounds for the manufacture of a medicament for the chemoprevention and treatment of both a precancerous lesion (such as, familial adenomatous polyposis, and actinic keratoses) and a cancer (particularly, colorectal, prostate, breast, bladder, or skin cancer). Therefore, this aspect of the invention is related to a method for the prophylactic and/or curative treatment of an animal, including a human, suffering from the above-mentioned pathologies, which comprises administering a therapeutically effective amount of a compound of formula (I) .

Another aspect of the invention relates to pharmaceutical compositions comprising a therapeutically effective amount of the compound (I), as an active ingredient, together with appropiate amounts of pharmaceutically acceptable excipients. Preferably, the compound is administered orally, parenterally or topically.

Throughout the description and claims the word "comprise" and variations of the word, such as "comprising", is not intended to exclude other additives, components, elements or steps. The disclosures in the abstract accompanying this application and in the application from which priority is claimed, are incorporated herein as reference. Throughout the description and claims, the terms "alkyl" and "alkoxyl" shall be construed as straight or branched. Additional aspects, advantages and novel features of the invention will be set forth in part in the description, and in part will become apparent to those skilled in the art upon examination of the description or may be learned by practice of the invention. The present invention will be further illustrated by the following examples. The examples are given by way of illustration only and are not to be construed as limiting.

### EXAMPLES

The structure of the different compounds of the present invention is confirmed either by ¹H-NMR (in CDCl₃, unless otherwise stated, and using a VARIAN UNITY-300 MHz equipment, wherein chemical shifts are expressed as ppm (δ) from the internal reference TMS) or by mass spectrometry obtaining the molecular ions by a electrospray probe of an Agilent 1100 VL. All of the reactions under microwave irradiation were conducted in heavy-walled Pyrex tubes. Microwave heating was carried out with a single mode cavity Discover Microwave Synthesizer (CEM corporation). The nomenclature used in the present document is based on the software AUTONOM (Automatic Nomenclature) from the Beilstein Institute, which uses the IUPAC systematic nomenclature. The following examples are given by way of illustration only and are not to be construed as limiting.

### General methods for bromination of desoxybenzoine

a) To a mixture of a desoxybenzoine derivative (50 mmol) in chloroform (210 mL) and CCl₄ (826 mL), bromine (50 mmol) dissolved in CCl₄ was added dropwise. When decolouration was complete, the organic layer was washed with 5% sodium bicarbonate and brine. Then, it was dried over anhydrous sodium sulfate and the solvent was evaporated to give the α-bromodesoxibenzoine of interest.
b) To a solution of a desoxybenzoine derivative (50 mmol) in EtOAc (210 mL), cooper (II) bromide (24, 5 g, 110 mmol) was added, and then the mixture was stirred at 60°C for 3 h. The reaction mixture was allowed to cool down and filtered through Celite, and the solvent was evaporated to give the α -bromodesoxibenzoine of interest.

### General method for 2-Phenylimidazo[1,2-a]pyrimidine derivatives

To a solution of a bromoacetofenone derivative (7.37 mmol) in dimethylformamide (DMF, 75 mL), a 2-aminopyrimidine derivative (18.4 mmol) was added. The mixture was stirred at 70°C for 12 h. Then, it was allowed to cool down and diluted with EtOAc. The solution was washed first with 5% sodium bicarbonate and then with water, dried over anhydrous sodium sulfate, and evaporated to dryness. The obtained residue was purified by column chromatography over silica gel (flash).

### General method for 3-bromo-2-phenylimidazo[1,2-a]pyrimidines

To a suspension of a 2-phenylimidazo[1,2-a]pyrimidine derivative (4.60 mmol) in acetonitrile (50 mL), NBS (750 mg, 4.20 mmol) was added in one portion at 0°C. The resulting solution was stirred at the same temperature for 1h, whereupon the reaction mixture was concentrated under reduced pressure. The obtained residue was purified by column chromatography over silica gel (flash).

### Examples 1 and 2: 2-(4-Ethoxyphenyl)-3-(4-methylsulfanylphenyl)imidazo[1,2-a]pyrimidine and 3-(4-ethoxyphenyl)-2-(4-methylsulfanylphenyl)imidazo[1,2-a]pyrimidine, respectively

To a solution of 1.1 g (2.9 mmol) of 2-bromo-2-(4-ethoxyphenyl)-1-(4-methylsulfanylphenyl)ethanone in 30 mL of DMF, 0.7 g (7.4 mmol) of 2-aminopyrimidine were added. The mixture was heated at 70°C with stirring for 12 h. Then, it was allowed to cool down and diluted with EtOAc. The solution was washed first with 5% sodium bicarbonate and then with water, dried over anhydrous sodium sulfate, and evaporated to dryness. The obtained residue was purified by column chromatography over silica gel (flash), using EtOAc as eluent, to give 300 mg of Example 1, and 70 mg of Example 2.

### Example 3: 2-(4-Methoxyphenyl)-5,7-dimethyl-3-(4-methylsulfanylphenyl)imidazo-[1,2-a]pyrimidine

To a solution of 750 mg of 2-bromo-1-(4-methoxyphenyl)-2-(4-methylsulfanylphenyl)ethanone (2.1 mmol) in 21 mL of acetonitrile, 660 mg of 2-amino-4,6-dimethylpyrimidine (5.3 mmol) were added. The reaction mixture was refluxed with stirring for 72 h, then, it was allowed to cool down and diluted with EtOAc. The solution was washed, first with 5% sodium bicarbonate and then with water, dried over anhydrous sodium sulfate, and evaporated to dryness. The obtained residue was purified by column chromatography over silica gel (flash), using EtOAc as eluent, to give 30 mg of the title compound.

### Example 4: 3-(4-Fluorophenyl)-2-p-tolylimidazo[1,2-a]pyrimidine

A solution of 150 mg of 3-bromo-2-p-tolylimidazo[1,2-a]pyrimidine (0.55 mmol), 92 mg of 4-fluoroboronic acid (0.66 mmol), 120 mg of Na₂CO₃ (2.10 mmol), and 0,5 mg of tetrakis(triphenylphosphine) (0.005 mmol) in 2 mL of THF and 2 mL of water was exposed to microwave irradiation (60 W) at a temperature of 170°C for 20 min. The pressure in the closed reaction vessel was comprised between 140-150 psi. After irradiation, the solution was diluted with EtOAc, and washed with water. The organic layer was dried over anhydrous sodium sulfate, and evaporated to dryness. The obtained residue was purified by column chromatography over silica gel (flash), using EtOAc:DCM (1:1) as eluent, to give 100 mg of the title compound.

### Example 73: 7-Chloro-3-(4-methoxyphenyl)-2-(4-methylsulfanylphenyl)imidazo[1,2-a]pyrimidine

A solution of 200 mg of Example 72 (0.55 mmol) in 5 mL of POCl₃ was refluxed with stirring for 3 h and then allowed to cool down. The solvent was evaporated, the residue obtained was diluted with water, and ammonia was added to basic pH. The solution was extracted with EtOAc, and the organic extracts were dried over anhydrous sodium sulfate, and evaporated to dryness. The obtained residue was purified by column chromatography over silica gel (flash), using EtOAc as eluent, to obtain 80 mg of the title compound.

### Example 102: 5-Methoxy-2-(4-methoxyphenyl)-3-(4-methylsulfanylphenyl)imidazo[1,2-a]pyrimidin-7-ol

To a solution of 750 mg (2.1 mmol) of 2-bromo-1-(4-methoxyphenyl)-2-(4-methylsulfanylphenyl)ethanone in 21 mL of acetonitrile, 826 mg (5.3 mmol) of 2-amino-4,6-dimethoxypyrimidine were added. The mixture was refluxed for 72 h, and allowed to cool down. Then, it was diluted with EtOAc, and 5% sodium bicarbonate was added. The organic layer was dried over anhydrous sodium sulfate, and evaporated to dryness. The obtained residue was purified by column chromatography over silica gel (flash), using EtOAc as eluent, to give 40 mg of the title compound.

### Example 220: Acetic acid 4-(7-Methyl-3-phenylimidazo[1,2-a]pyrimidin-2-yl)phenylsulfanylmethyl ester

To a solution of 550 mg of Example 166 (1.58 mmol) in 7 mL of acetic anhydride, 700 mg of potassium acetate (7.12 mmol) were added. The mixture was refluxed for 10 h, and then allowed to cool down. The solvent was evaporated, the residue obtained was diluted with EtOAc, and the solution was washed with a saturated solution of NH₄Cl and brine. The organic layer was dried over anhydrous sodium sulfate, and evaporated to dryness to obtain 610 mg of the title compound.

### Example 221: Acetic acid 4-(7-Methyl-3-phenylimidazo[1,2-a]pyrimidin-2-yl)benzenesulfonylmethyl ester

To a solution of 610 mg of Example 220 (1.56 mmol) in 20 mL of DCM:MeOH (3:1), 1,06 g of MMPP (1.72 mmol) were added. The mixture was stirred at room temperature for 10 h. The reaction mixture was neutralized with saturated bicarbonate and the solvent was evaporated. The residue obtained was diluted with DCM and the solution was washed with 5% sodium bicarbonate. The organic layer was dried over anhydrous sodium sulfate, and evaporated to dryness. The obtained residue was purified by column chromatography over silica gel (flash), using EtOAc as eluent, to give 260 mg of the title compound.

### Example 216: 4-(7-Methyl-3-phenylimidazo[1,2-a]pyrimidin-2-yl)benzenesulfonamide

To a solution of 250 mg of Example 221 (0.60 mmol) in 6 mL of MeOH, 460 mg of potassium acetate (4.74 mmol) were added. The mixture was stirred at room temperature for 10 min. Then, 170 mg of potassium carbonate (1.18 mmol) were added and the reaction mixture was stirred for 1.5 h. Then, 270 mg of HOSA (2.37 mmol) were added and the solution was further stirred for 2 h. The solvent was evaporated and the residue obtained was diluted with EtOAc. The solution was washed with 5% sodium bicarbonate. The organic layer was dried over anhydrous sodium sulfate, and evaporated to dryness. The obtained residue was washed with MeOH to give 90 mg of the title compound.

The following examples were prepared using some of the methods previously described.

**TABLE 1**

| Ex. | |
|---|---|
| 1 | 2-(4-Ethoxyphenyl)-3-(4-methylsulfanylphenyl)imidazo[1,2-a]pyrimidine; ¹H-NMR: 1.49 (t, 3H, *J*=7), 2.47 (s, 3H), 4.13 (q, 2H, *J*=7), 6.80 (dd, 1H, *J*=6.5, *J*=4), 7.07 (d, 2H, *J*=9), 7.17 (d, 2H, *J*=8.5), 7.35 (d, 2H, *J*=8.5), 7.71 (d, 2H, *J*=9), 8.19 (dd, 1H, *J*=6.5, *J*=2), 8.53 (dd, 1H, *J*=6.5, *J*=2); MS [M+1]⁺: 362 |
| 2 | 3-(4-Ethoxyphenyl)-2-(4-methylsulfanylphenyl)imidazo[1,2-a]pyrimidine; ¹H-NMR: 1.41 (t, 3H, *J*=7), 2.57 (s, 3H), 4.04 (q, 2H, *J*=7), 6.80 (dd, 1H, *J*=6.5, *J*=4), 6.84 (d, 2H, *J*=8.5), 7.36 (d, 2H, *J*=8.5), 7.40 (d, 2H, *J*=8.5), 7.69 (d, 2H, *J*=8.5), 8.22 (dd, 1H, *J*=6.5, *J*=2), 8.52 (dd, 1H, *J*=6.5, *J*=2); MS [M+1]⁺: 362 |
| 3 | 2-(4-Methoxyphenyl)-5,7-dimethyl-3-(4-methylsulfanylphenyl)imidazo[1,2-a]pyrimidine; ¹H-NMR: 2.08 (s, 3H), 2.56 (s, 3H), 2.57 (s, 3H), 3.78 (s, 3H), 6.39 (s, 1H), 6.78 (d, 2H, *J*=9), 7.30 (d, 2H, *J*=8.5), 7.38 (d, 2H, *J*=8.5), 7.59 (d, 2H, *J*=9); MS [M+1]⁺: 376 |
| 4 | 3-(4-Fluorophenyl)-2-*p*-tolylimidazo[1,2-*a*]pyrimidine; ¹H-NMR: 2.34 (s, 3H), 6.83 (dd, 1H, *J*=7, *J*=4), 7.13 (d, 2H, *J*=8), 7.27 (t, 2H, *J*=8.5), 7.46 (d, 1H, *J*=8.5), 7.47 (d, 1H, *J*=8.5), 7.64 (d, 2H, *J*=8.5), 8.22 (dd, *J*=6.5, *J*=1), 8.61 (dd, 1H, *J*=4, *J*=1); MS [M+1]⁺: 304 |
| 5 | 2,3-Bis-(4-methylsulfanylphenyl)imidazo[1,2-*a*]pyrimidine; ¹H-NMR: 2.49 (s, 3H), 2.57 (s, 3H), 6.82 (dd, 1H, *J*=6.5, *J*=4), 7.18 (d, 2H, *J*=8.5), 7.36 (d, 2H, *J*=8.5), 7.41 (d, 2H, *J*=8.5), 7.70 (d, 2H, *J*=9), 8.22 (dd, 1H, *J*=6.5, *J*=2), 8.55 (dd, 1H, J=6.5, *J*=2); MS [M+1]⁺: 364 |
| 6 | 3-Phenyl-2-(4-propylsulfanylphenyl)imidazo[1,2-*a*]pyrimidine; ¹H-NMR: 1.24 (t, 3H, *J*=7.5), 1.69 (m, 2H, *J*=7.5), 2.91 (t, 2H, *J*=7.5), 6.82 (dd, 1H, *J*=6.5, *J*=4), 7.22 (d, 2H, *J*=8.5), 7.44-7.48 (m, 2H), 7.54-7.59 (m, 3H), 7.68 (d, 2H, *J*=8.5), 8.24 (dd, 1H, *J*=6.5, *J*=2), 8.56 (dd, 1H, *J*=6.5, *J*=2); MS [M+1]⁺: 346 |
| 7 | 2-(3-Methoxy-4-methylphenyl)-3-(4-methylsulfanylphenyl)imidazo[1,2-*a*]pyrimidine; ¹H-NMR: 2.28 (s, 3H), 2.48 (s, 3H), 3.94 (s, 3H), 6.79 (dd, 1H, *J*=7, *J*=4), 6.98 (d, 1H, *J*=8), 7.18 (d, 2H, *J*=8.5), 7.23 (dd, 2H, *J*=8, *J*=2), 7.36 (d, 2H, *J*=8.5), 7.75 (s, 1H), 8.18 (dd, 1H, *J*=7, *J*=2), 8.52 (dd, 1H, *J*=7, *J*=2) ; MS [M+1]⁺: 362 |
| 8 | 2-(4-Methoxyphenyl)-3-(4-methylsulfanylphenyl)imidazo[1,2-a]pyrimidine; ¹H-NMR: 2.56 (s, 3H), 3.80 (s, 3H), 6.77 (dd, 1H, *J*=7, *J*=4), 6.83 (d, 2H, *J*=9), 7.33 (d, 2H, *J*=9), 7.38 (d, 2H, *J*=9), 7.71 (d, 2H, *J*=9), 8.21 (dd, 1H, *J*=7, *J=*2), 8.52 (dd, 1H, *J*=4, *J*=2); MS [M+1]⁺: 348 |
| 9 | 3-(4-Methoxyphenyl)-2-(3-nitrophenyl)imidazo[1,2-*a*]pyrimidine; ¹H-NMR: 3.92 (s, 3H), 6.86 (dd, 1H, *J*=7, *J*=4), 7.12 (d, 2H, *J*=9), 7.38 (d, 2H, *J*=8.5), 7.47 (t, 1H, *J*=8), 8.09-8.15 (m, 2H), 8.22 (dd, 1H, *J*=7, *J*=2), 8.61 (dd, 1H, *J*=4, *J*=2), 8.64 (t, 1H, *J*=1.5); MS [M+1]⁺: 347 |
| 10 | 3-(4-Methoxyphenyl)-2-(3-trifluoromethylphenyl)imidazo[1,2-a]pyrimidine; ¹H-NMR: 3.91 (s, 3H), 6.84 (dd, 1H, *J*=7, *J*=4), 7.10 (d, 2H, *J*=9), 7.36 (d, 2H, *J*=9), 7.38 (t, 1H, *J*=8), 7.51 (d, 1H, *J*=8), 7.87 (d, 1H, *J*=8), 8.12 (s, 1H), 8.21 (dd, 1H, *J*=7, *J*=2), 8.57 (dd, 1H, *J*=4, *J*=2); MS [M+1]⁺: 370 |
| 11. | 2-(4-Methylsulfanylphenyl)-3-phenylimidazo[1,2-*a*]pyrimidine; ¹H-NMR: 2.45 (s, 3H), 6.79 (dd, 1H, *J*=6.5, *J*=4), 7.14 (d, 2H, *J*=8.5), 7.42-7.45 (m, 2H), 7.50-7.55 (m, 3H), 7.66 (d, 2H, *J*=8.5), 8.21 (dd, 1H, *J*=6.5, *J*=2), 8.53 (dd, 1H, *J*=4, *J*=2) |
| 12 | 2-(4-Fluorophenyl)-3-(4-metliylsulfanylphenyl)imidazo[1,2-a]pyrimidine; ¹H-NMR: 2.57 (s, 3H), 6.85 (dd, 1H, *J*=7, *J*=4), 7.01 (d, 1H, *J*=8.5), 7.03 (d, 1H, *J*=9), 7.37 (d, 2H, *J*=9), 7.42 (d, 2H, *J*=9), 7.76 (d, 1H, *J*=9), 7.77 (d, 1H, *J*=9), 8.25 (dd, 1H, *J*=7, *J*=2), 8.58 (dd, 1H, *J*=4, *J*=2) |
| 13 | 2-(4-Bromophenyl)-3-(4-methylsulfanylphenyl)imidazo[1,2-*a*]pyrimidine; ¹H-NMR: 2.57 (s, 3H), 6.83 (dd, 1H, *J*=7, *J*=4), 7.34 (d, 2H, *J*=8.5), 7.37 (d, 1H, *J*=8.5), 7.41 (d, 2H, *J*=8.5), 7.43 (d, 2H, *J*=9), 7.64 (d, 1H, *J*=8.5), 8.22 (dd, 1H, *J*=7, *J*=2), 8.57 (dd, 1H, *J*=4, *J*=2); MS [M/M+2]⁺: 396/398 |
| 14 | 3-(4-Bromophenyl)-2-(4-methylsulfanylphenyl)imidazo[1,2-*a*]pyrimidine; ¹H-NMR: 2.45 (s, 3H), 6.80 (dd, 1H, *J*=7, *J*=4), 7.14 (d, 2H, *J*=8.5), 7.30 (d, 2H, *J*=8.5), 7.60 (d, 2H, *J*=8.5), 7.66 (d, 2H, *J*=8.5), 8.19 (dd, 1H, *J*=7, *J*=2), 8.50 (dd, 1H, *J*=4, *J*=2); MS [M/M+2]⁺: 396/398 |
| 15 | 3-(3,4-Difluorophenyl)-2-(4-methanesulfonylphenyl)imidazo[1,2-a]pyrimidine; ¹H-NMR: 3.19 (s, 3H), 7.09 (dd, 1H, *J*=7, *J*=4), 7.38-7.43 (m, 1H), 7.68 (dt, 1H, *J*=11, *J*=8.5) 7.80 (tdd, 1H, *J*=11, *J*=8, *J*=2), 7.86 (d, 2H, *J*=9), 7.91 (d, 2H, *J*=9), 8.57 (dd, 1H, *J*=7, *J*=2), 8.66 (dd, 1H, *J*=4, J=2) |
| 16 | 3-(4-Chlorophenyl)-2-(4-methanesulfonylphenyl)imidazo[1,2-a]pyrimidine; ¹H-NMR: 3.04 (s, 3H), 6.88 (dd, 1H, *J*=7, *J*=4), 6.38 (d, 2H, *J*=8.5), 7.56 (d, 2H, *J*=8.5) 7.85 (d, 2H, *J*=8.5), 7.91 (d, 2H, *J*=8.5), 8.21 (dd, 1H, *J*=7, *J*=2), 8.60 (dd, 1H, *J*=4, *J*=2) |
| 17 | 7-Methyl-2- (4-methylsulfanylphenyl) -3-phenylimidazo[1,2-a]pyrimidine; ¹H-NMR: 2.46 (s, 3H), 2.62 (s, 3H), 6.66 (d, 1H, *J*=7), 7.15 (d, 2H, *J*=8.5), 7.42-7.44 (m, 2H), 7.49-7.55 (m, 3H), 7.67 (d, 2H, *J*=8.5), 8.07 (d, 1H, *J*=7); MS [M+1]⁺: 332 |
| 18 | 3-(4-Fluorophenyl)-7-methyl-2-(4-methylsulfanylphenyl)imidazo[1,2-a]pyrimidine; ¹H-NMR: 2.57 (s, 3H), 2.61 (s, 3H), 6.69 (d, 1H, *J*=7), 6.98 (d, 1H, *J*=8.5), 7.01 (d, 1H, *J*=8.5), 7.34 (d, 2H, *J*=8.5), 7.40 (d, 2H, *J*=8.5), 7.72-7.77 (m, 2H), 8.08 (d, 1H, *J*=7); MS [M+1]⁺: 350 |
| 19 | 3-(4-Bromophenyl)-7-methyl-2-(4-methylsulfanylphenyl)imidazo[1,2-a]pyrimidine; ¹H-NMR: 2.46 (s, 3H), 2.61 (s, 3H), 6.68 (d, 1H, *J*=7), 7.14 (d, 2H, *J*=8.5), 7.30 (d, 2H, *J*=8.5), 7.62 (d, 2H, *J*=9), 7.65 (d, 2H, *J*=9), 8.04 (d, 1H, *J*=7); MS [M/M+2]⁺: 410/412 |
| 20 | 3-(4-Ethoxyphenyl)-7-methyl-2-(4-methylsulfanylphenyl)imidazo[1,2-*a*]pyrimidine; ¹H-NMR: 1.40 (t, 3H, *J*=7), 2.56 (s, 3H), 2.62 (s, 3H), 4.03 (q, 2H, *J*=7), 6.65 (d, 1H, *J*=7), 6.82 (d, 2H, *J*=9), 7.33 (d, 2H, *J*=9), 7.38 (d, 2H, *J*=9), 7.70 (d, 2H, *J*=9), 8.06 (d, 1H, *J*=7) |
| 21 | 7-Methyl-2,3-bis-(4-methylsulfanylphenyl)imidazo[1,2-a]pyrimidine; ¹H-NMR: 2.47 (s, 1H), 2.56 (s, 3H), 2.63 (s, 3H), 6.67 (d, 1H, *J*=7), 7.17 (d, 2H, *J*=8.5), 7.33 (d, 2H, *J*=8.5), 7.39 (d, 2H, *J*=8.5), 7.69 (d, 2H, *J*=8.5), 8.06 (d, 1H, *J*=7); MS [M+1]⁺: 365 |
| 22 | 7-Methyl-3-phenyl-2-(4-propylsulfanylphenyl)imidazo[1,2-a]pyrimidine; ¹H-NMR: 1.01 (t, 3H, *J*=7), 1.66 (m, 2H, *J*=7), 2.62 (s, 3H), 2.88 (t, 2H, *J*=7), 6.66 (d, 1H, *J*=7), 7.20 (d, 2H, *J*=8.5), 7.40-7.44 (m, 2H), 7.48-7.57 (m, 3H), 7.66 (d, 2H, *J*=8.5), 8.06 (d, 1H, *J*=7); MS [M+1]⁺: 360 |
| 23 | 7-Methyl-3-(4-methylsulfanylphenyl)-2-phenylimidazo[1,2-*a*]pyrimidine; ¹H-NMR: 2.57 (s, 3H), 2.64 (s, 3H), 6.68 (d, 1H, *J*=7), 7.26-7.33 (m, 3H), 7.34 (d, 2H, *J*=8.5), 7.38 (d, 2H, *J*=8.5), 7.75-7.79 (m, 2H), 8.08 (d, 1H, *J*=7) |
| 24 | 2-(4-Methoxyphenyl)-7-methyl-3-(4-methylsulfanylphenyl)imidazo[1,2-*a*]pyrimidine; ¹H-NMR: 2.56 (s, 3H), 2.62 (s, 3H), 3.80 (s, 3H), 6.65 (d, 1H, *J*=7), 6.83 (d, 2H, *J*=9), 7.33 (d, 2H, *J*=9), 7.38 (d, 2H, *J*=9), 7.71 (d, 2H, *J*=9), 8.05 (d, 1H, *J*=7); MS [M+1]⁺: 396 |
| 25 | 2-(3-Methoxy-4-methylphenyl)-7-methyl-3-(4-methylsulfanylphenyl)imidazo[1,2-*a*]pyrimidine;¹H-NMR: 2.00 (s, 3H), 2.21 (s, 3H), 2.40 (s, 3H), 3.85 (s, 3H), 6.57-6.63 (m, 2H), 6.89 (d, 1H, *J*=8.5), 7.09 (d, 2H, *J*=8.5), 7.12 (d, 2H, *J*=8.5), 7.69 (s, 1H), 7.95 (d, 1H, *J*=7); MS [M+1]⁺: 376 |
| 26 | 2-(4-Methoxy-3-methylphenyl)-7-methyl-3-(4-methylsulfanylphenyl)imidazo[1,2-*a*]pyrimidine; ¹H-NMR: 2.13 (s, 3H), 2.48 (s, 3H), 2.53 (s, 3H), 3.74 (s, 3H), 6.57 (d, 1H, *J*=7), 6.63 (d, 1H, *J*=8.5), 7.25 (d, 2H, *J*=8.5), 7.30 (d, 2H, *J*=8.5), 7.37 (dd, 1H, *J*=8.5, *J*=1), 7.66 (s, 1H) 7.98 (d, 1H, *J*=7); MS [M+1]⁺: 376 |
| 27 | 2-(4-Methanesulfonylphenyl)-7-methyl-3-phenylimidazo[1,2-a]pyrimidine; ¹H-NMR: 2.63 (s, 3H), 3.02 (s, 3H), 6.71 (d, 1H, *J*=7), 7.39-7.42 (m, 2H), 7.53-7.56 (m, 3H), 7.79 (d, 2H, *J*=9), 7.91 (d, 2H, *J*=9), 8.06 (d, 1H, *J*=7) |
| 28 | 2-(4-Methanesulfonylphenyl)-7-methyl-3-p-tolylimidazo[1,2-*a*]pyrimidine; ¹H-NMR: 2.48 (s, 3H), 2.66 (s, 3H), 3.04 (s, 3H), 6.72 (d, 1H, *J*=7), 7.31 (d, 2H, *J*=8), 7.38 (d, 2H, *J*=8), 7.83 (d, 2H, *J*=8.5), 7.96 (d, 2H, *J*=8.5), 8.07 (d, 1H, *J*=7) |
| 29 | 2-(4-Methanesulfonylphenyl)-5,7-dimethyl-3-phenylimidazo[1,2-*a*]pyrimidine; ¹H-NMR: 2.05 (d, 3H, *J*=0.5), 2.58 (s, 3H), 3.00 (s, 3H), 6.46 (d, 1H, *J*=0.5), 7.19 (d, 1H, *J*=8.5), 7.49-7.61 (m, 5H), 7.76 (d, 2H, *J*=8.5), 7.81 (d, 2H, *J*=8.5) |
| 30 | 3-(4-Fluorophenyl)-2-(4-methanesulfonylphenyl)-5,7-dimethylimidazo[1,2-*a*]pyrimidine; ¹H-NMR: 2.07 (s, 3H), 2.58 (s, 3H), 3.01 (s, 3H), 6.46 (s, 1H), 7.19 (d, 1H, *J*=8.5), 7.22 (d, 1H, *J*=8.5), 7.46 (d, 1H, *J*=8.5), 7.48 (d, 1H, *J*=8.5), 7.77 (s, 4H) |
| 31 | 3-(3-Fluorophenyl)-2-(4-methanesulfonylphenyl)imidazo[1,2-*a*]pyrimidine; ¹H-NMR (d₆-DMSO): 3.19 (s, 3H), 7.09 (dd, 1H, *J*=7, *J*=4), 7.38 (dt, 1H, *J*=8, *J*=1), 7.45 (tdd, 1H, *J*=9, J=2.5, *J*=1), 7.52 (ddd, 1H, *J*=9.5, *J*=2.5, *J*=1), 7.66 (td, 1H, *J*=8, *J*=6), 7.84 (d, 2H, *J*=8.5), 7.91 (d, 2H, *J*=8.5), 8.56 (ddd, 1H, *J*=6.5, *J*=2, *J*=1), 8.66 (dd, 1H, *J*=4, *J*=2) |
| 32 | 2-(4-Methoxy-3-methylphenyl)-3-(4-methylsulfanylphenyl)imidazo[1,2-*a*]pyrimidine; ¹H-NMR: 2.19 (s, 3H), 2.57 (s, 3H), 3.82 (s, 3H), 6.73 (d, 1H, *J*=8.5), 6.79 (dd, 1H, *J*=7, *J*=4), 7.37 (d, 2H, *J*=8.5), 7.41 (d, 2H, *J*=8.5), 7.45 (dd, 1H, *J*=8.5, *J*=2), 7.72 (s, 1H), 8.21 (dd, 1H, *J*=7, *J*=2), 8.53 (dd, 1H, *J*=4, *J*=2); MS [M+1]⁺: 362 |
| 33 | 2-(4-Methanesulfonylphenyl)-3-phenylimidazo[1,2-*a*]pyrimidine; ¹H-NMR: 3.06 (s, 3H), 6.88 (dd, 1H, *J*=7, *J*=4), 7.43-7.47 (m, 2H), 7.57-7.62 (m, 3H), 7.85 (d, 2H, *J*=8.5), 7.96 (d, 2H, *J*=8.5), 8.26 (dd, 1H, *J*=7, *J*=2), 8.62 (dd, 1H, *J*=4, *J*=2) |
| 34 | 3-(4-Fluorophenyl)-2-(4-methanesulfonylphenyl)imidazo[1,2-a]pyrimidine; ¹H-NMR (d₆-DMSO): 3.19 (s, 3H), 7.08 (dd, 1H, *J*=7, *J*=4), 7.45 (d, 1H, *J*=9), 7.48 (d, 1H, *J*=9) 7.63 (d, 1H, *J*=9), 7.65 (d, 1H, *J=*9), 7.84 (d, 2H, *J*=8.5), 7.90 (d, 2H, *J*=8.5), 8.48 (dd, 1H, *J*=7, *J*=2), 8.65 (dd, 1H, *J*=4, *J*=2) |
| 35 | 2-(4-Ethoxy-3-fluorophenyl)-7-methyl-3-phenylimidazo[1,2-*a*]pyrimidine; ¹H-NMR: 1.43 (t, 3H, *J*=7), 2.63 (s, 3H), 4.09 (q, 2H, *J*=7), 6.67 (d, 1H, *J*=7), 6.86 (t, 1H, *J*=8.5), 7.41-7.58 (m, 5H), 8.05 (d, 1H, *J*=7); MS [M+1]⁺: 348 |
| 36 | 2-(4-Ethoxy-3-fluorophenyl)-3-(4-methylsulfanylphenyl)imidazo[1,2-a]pyrimidine; ¹H-NMR: 1.44 (t, 3H, *J*=7), 2.57 (s, 3H), 4.10 (q, 2H, *J*=7), 6.80 (dd, 1H, *J*=7, *J*=4), 6.88 (t, 1H, *J*=8.5), 7.34 (d, 2H, *J*=9), 7.40 (d, 2H, *J*=9), 7.47-7.53 (m, 2H), 8.19 (dd, 1H, *J*=4, *J*=2), 8.54 (dd, 1H, J=7, *J*=2) |
| 37 | 2-(4-Ethoxy-3-fluorophenyl)-7-methyl-3-(4-methylsulfanylphenyl)imidazo[1,2-*a*]pyrimidine; ¹H-NMR: 1.43 (t, 3H, *J*=7), 2.57 (s, 3H), 2.63 (s, 3H), 4.10 (q, 2H, *J*=7), 6.66 (d, 1H, *J*=7), 6.87 (t, 1H, *J*=8.5), 7.33 (d, 2H, *J*=8.5), 7.39 (d, 2H, *J*=8.5), 7.48-7.51 (m, 2H), 8.04 (d, 1H, *J*=7) |
| 38 | 2-(4-Cyclopropylsulfanylphenyl)-3-(4-methylsulfanylphenyl)imidazo[1,2-a]pyrimidine; ¹H-NMR: 0.60-0.75 (m, 2H), 1.06-1.09 (m, 2H), 2.16-2.18 (m, 1H), 2.57 (s, 3H), 6.81 (dd, 1H, *J*=7, *J*=4), 7.29 (d, 2H, *J*=8.5), 7.37 (d, 2H, *J*=8), 7.38 (d, 2H, *J*=8), 7.70 (d, 2H, *J*=8.5), 8.22 (dd, 1H, *J*=7, *J*=2), 8.55 (dd, 1H, *J*=4, *J*=2) |
| 39 | 2-(4-Cyclopropylsulfanylphenyl)-7-methyl-3-(4-methylsulfanylphenyl)imidazo[1,2-*a*]pyrimidine; ¹H-NMR: 0.65-0.72 (m, 2H), 1.06-1.09 (m, 2H), 2.17-2.19 (m, 1H), 2.56 (s, 3H), 2.63 (s, 1H), 6.66 (d, 1H, *J*=7), 7.28 (d, 2H, *J*=8.5), 7.34 (d, 2H, *J*=8), 7.38 (d, 2H, *J*=8), 7.69 (d, 2H, *J*=8.5), 8.05 (dd, 1H, *J*=7) |
| 40 | 2-(3-Chloro-4-ethylsulfanylphenyl)-7-methyl-3-(4-methylsulfanylphenyl)imidazo[1,2-a]pyrimidine; ¹H-NMR: 1.36 (t, 3H, *J*=7), 2.57 (s, 3H), 2.64 (s, 3H), 2.96 (q, 2H, *J*=7), 6.68 (d, 1H, *J*=7), 7.13 (d, 1H, *J*=8), 7.33 (d, 2H, *J*=8.5), 7.40 (d, 2H, *J*=8.5), 7.55 (dd, 1H, *J*=8.5, *J*=2), 7.87 (d, 1H, *J*=2), 8.05 (d, 1H, *J*=7) |
| 41 | N-[5-Dimethylamino-2-(4-methoxyphenyl)-3-(4-methylsulfanylphenyl)imidazo[1,2-*a*]pyrimidin-7-yl]-2,2,2-trifluoroacetamide; MS [M+1]⁺: 406 |
| 42 | 2-(3-Chloro-4-isopropylsulfanylphenyl)-3-(4-methylsulfanylphenyl)imidazo[1,2-*a*]pyrimidine; ¹H-NMR: 1.35 (d, 6H, *J*=6.5), 2.58 (s, 3H), 3.49 (m, 1H), 6.85 (dd, 1H, *J*=7, *J*=4.5), 7.25 (d, 1H, *J*=8.5), 7.35 (d, 2H, *J*=8.5), 7.42 (d, 2H, *J*=8.5), 7.53 (dd, 1H, *J*=8.5, *J*=1.5), 7.88 (d, 1H, *J*=1.5), 8.22 (dd, 1H, *J*=7, *J*=2), 8.59 (dd, 1H, *J*=7, *J*=2) ; MS [M/M+2]⁺: 426/428 |
| 43 | 2-(3-Chloro-4-isopropylsulfanylphenyl)-7-methyl-3-(4-methylsulfanylphenyl)imidazo[1,2-a]pyrimidine; ¹H-NMR: 1.32 (d, 6H, *J*=6.5), 2.55 (s, 3H), 2.61 (s, 3H), 3.47 (m, 1H), 6.67 (d, 1H, *J*=7), 7.21 (d, 1H, *J*=8.5), 7.35 (d, 2H, *J*=8.5), 7.38 (d, 2H, *J*=8.5), 7.50 (dd, 1H, *J*=8.5, *J*=2), 7.88 (d, 1H, *J*=2), 8.03 (dd, 1H, *J*=7) |
| 44 | 3-(4-Methoxyphenyl)-7-methyl-2-(3-trifluoromethylphenyl)imidazo[1,2-*a*]pyrimidine; ¹H-NMR: 2.64 (s, 3H), 3.91 (s, 3H), 6.79 (d, 1H, *J*=7), 7.08 (d, 2H, *J*=9), 7.35 (d, 2H, *J*=9), 7.35 (t, 1H, J=7.5), 7.48 (d, 1H, *J*=7.5), 7.86 (d, 1H, *J*=7.5), 8.05 (d, 1H, *J*=7), 8.14 (s, 1H); MS [M+1]⁺: 384 |
| 45 | 2-(3-Chloro-4-methoxyphenyl)-3-(4-methoxyphenyl)imidazo[1,2-a]pyrimidine; ¹H-NMR: 3.89 (s, 3H), 3.91 (s, 3H), 6.80 (dd, 1H, *J*=7, *J*=4), 6.86 (d, 1H, *J*=8.5), 7.08 (d, 2H, *J*=9), 7.36 (d, 2H, *J*=9), 7.60 (dd, 1H, *J*=8.5, *J*=2), 7.85 (d, 1H, *J*=2), 8.17 (dd, 1H, *J*=7, *J*=2), 8.53 (dd, 1H, *J*=4, *J*=2); MS [M/M+2]⁺: 366/368 |
| 46 | 3-(2-Bromo-5-methoxyphenyl)-2-(4-methoxyphenyl)imidazo[1,2-*a*]pyrimidine; ¹H-NMR: 3.75 (s, 3H), 3.80 (s, 3H), 6.83 (dd, 1H, *J*=7, *J*=4), 6.85 (d, 2H, *J*=8.5), 6.89 (d, 1H, *J*=3), 6.99 (dd, 1H, *J*=8.5, *J*=3), 7.68 (d, 2H, *J=*8.5), 7.71 (d, 1H, *J*=8.5), 7.89 (dd, 1H, *J*=7, *J*=2), 8.56 (dd, 1H, *J*=4, *J*=2); MS [M/M+2]⁺: 410/412 |
| 47 | 3-(2-Bromo-5-methoxyphenyl)-2-(4-methoxyphenyl)-7-methylimidazo[1,2-*a*]pyrimidine; ¹H-NMR: 2.65 (s, 3H), 3.75 (s, 3H), 3.80 (s, 3H), 6.69 (d, 1H, *J*=7), 6.83 (d, 2H, *J*=8.5), 6.89 (d, 1H, *J*=3), 6.97 (dd, 1H, *J*=8.5, *J*=3), 7.67 (d, 2H, *J*=8.5), 7.70 (d, 1H, *J*=8.5), 7.75 (d, 1H, *J*=7); MS [M/M+2]⁺: 424/426 |
| 48 | 3-(3-Chloro-4-methoxyphenyl)-2-(2,4-dichloro-5-methoxyphenyl)imidazo[1,2-*a*]pyrimidine; ¹H-NMR: 3.79 (s, 3H), 3.90 (s, 3H), 6.86 (d, 1H, *J*=9), 6.88 (dd, 1H, *J*=7, *J*=4), 6.90 (s, 1H), 7.48 (dd, 1H, *J*=9, *J*=2), 7.68 (s, 1H), 7.90-7.93 (m, 2H), 8.61 (dd, 1H, *J*=4, *J*=2); MS [M/M+2/M+4/M+6]⁺: 434/436/438/440 |
| 49 | 3-(3-Chloro-4-methoxyphenyl)-2-(2,4-dichloro-5-methoxyphenyl)-7-methylimidazo[1,2-*a*]pyrimidine; ¹H-NMR: 2.66 (s, 3H), 3.78 (s, 3H), 3.89 (s, 3H), 6.74 (d, 1H, *J*=7), 6.85 (d, 1H, *J*=9), 6.89 (s, 1H), 7.48 (dd, 1H, *J*=9, *J*=2), 7.67 (s, 1H), 7.76 (d, 1H, *J*=7), 7.88 (d, 1H, *J*=2) |
| 50 | 3-(3-Fluorophenyl)-2-(4-methoxyphenyl)imidazo[1,2-*a]*pyrimidine; ¹H-NMR: 3.81 (s, 3H), 6.83 (dd, 1H, *J*=7, *J*=4), 6.85 (d, 2H, *J*=9), 7.15-7.25 (m, 3H), 7.53 (td, 1H, *J*=8, *J*=6), 7.68 (d, 2H, *J*=9), 8.24 (dd, 1H, *J*=7, *J*=2), 8.55 (d, 1H, *J*=4, *J*=2); MS [M+1]⁺: 320 |
| 51 | 3-(3-Fluorophenyl)-2-(4-methoxyphenyl)-7-methylimidazo[1,2-a]pyrimidine; ¹H-NMR: 2.63 (s, 3H), 3.81 (s, 3H), 6.68 (d, 1H, *J*=7), 6.84 (d, 2H, *J*=9), 7.13-7.19 (m, 2H), 7.23 (d, 1H, *J*=8), 7.51 (td, 1H, *J*=8, *J*=6), 7.67 (d, 2H, *J*=9), 8.09 (d, 1H, *J*=7) |
| 52 | 3-(3-Chlorophenyl)-2-(4-methoxyphenyl)imidazo[1,2-*a*]pyrimidine; ¹H-NMR: 3.81 (s, 3H), 6.83 (dd, 1H, *J*=7, *J*=4), 6.86 (d, 2H, *J*=9), 7.32-7.37 (m, 1H), 7.46-7.50 (m, 3H), 7.68 (d, 2H, *J*=9), 8.22 (dd, 1H, *J*=7, *J*=2), 8.55 (d, 1H, *J*=4, *J*=2); MS [M/M+2]⁺: 336/338 |
| 53 | 2-(4-Methoxyphenyl)-3-(3-trifluoromethylphenyl)imidazo[1,2-*a*]pyrimidine; ¹H-NMR: 3.81 (s, 3H), 6.84 (dd, 1H, *J*=7, *J*=4), 6.85 (d, 2H, *J*=9), 7.64 (d, 2H, *J*=9), 7.62-7.78 (m, 3H), 8.01 (s, 1H), 8.21 (dd, 1H, *J*=7, *J*=2), 8.57 (d, 1H, *J*=4, *J*=2); MS [M+1]⁺: 370 |
| 54 | 3-(3-Methoxyphenyl)-2-(4-methoxyphenyl)imidazo[1,2-*a*]pyrimidine; ¹H-NMR: 3.79 (s, 3H), 3.80 (s, 3H), 6.78 (dd, 1H, *J*=7, *J*=4), 6.83 (d, 2H, *J*=9), 6.96-6.97 (m, 1H), 7.01-7.06 (m, 2H), 7.46 (t, 1H, *J*=8), 7.72 (d, 2H, *J*=9), 8.23 (dd, 1H, *J*=7, *J*=2), 8.51 (d, 1H, *J*=4, *J*=2); MS [M+1]⁺: 332 |
| 55 | 2-(4-Methoxyphenyl)-3-(3-nitrophenyl)imidazo[1,2-*a*]pyrimidine; ¹H-NMR: 3.79 (s, 3H), 6.83 (d, 2H, *J*=9), 6.88 (dd, 1H, *J*=7, *J*=4), 7.59 (d, 2H, *J*=9), 7.71 (td, 1H, *J*=7.5, *J*=1), 7.76 (dt, 1H, *J*=7.5, *J*=1), 7.99 (s, 1H), 8.25 (dd, 1H, *J*=7, *J*=2), 8.34 (dt, 1H, *J*=7.5, *J*=1), 8.60 (dd, 1H, *J*=4, *J*=2); MS [M+1] ⁺: 347 |
| 56 | 2-(4-Methoxyphenyl)-3-p-tolylimidazo[1,2-a]pyrimidine; ¹H-NMR: 2.48 (s, 3H), 3.80 (s, 3H), 6.78 (dd, 1H, *J*=7, *J*=4), 6.84 (d, 2H, *J*=9), 7.33 (d, 2H, *J*=8.5), 7.36 (d, 2H, *J*=8.5), 7.72 (d, 2H, *J*=9), 8.20 (dd, 1H, *J*=7, *J*=2), 8.52 (dd, 1H, *J*=4, *J*=2); MS [M+1]⁺: 315 |
| 57 | 3-(4-Isopropylphenyl)-2-(4-methoxyphenyl)-7-methylimidazo[1,2-*a*]pyrimidine; ¹H-NMR: 1.33 (d, 6H, *J*=7), 2.62 (s, 3H), 3.00 (m, 1H), 3.80 (s, 3H), 6.63 (d, 1H, *J*=7), 6.82 (d, 2H, *J*=8.5), 7.34 (d, 2H, J=8.5), 7.39 (d, 2H, *J*=8.5), 7.71 (d, 2H, *J*=9), 8.08 (d, 1H, *J*=7); MS [M+1]⁺: 358 |
| 58 | 3-(4-*tert*-Butylphenyl) -2- (4-methoxyphenyl)imidazo[1,2-*a*]pyrimidine; ¹H-NMR: 1.40 (s, 9H), 3.80 (s, 3H), 6.78 (dd, 1H, *J*=7, *J*=4), 6.84 (d, 2H, *J*=9), 7.37 (d, 2H, *J*=8.5), 7.55 (d, 2H, *J*=8.5), 7.72 (d, 2H, *J*=9), 8.24 (dd, 1H, *J*=7, *J*=2), 8.52 (dd, 1H, *J*=4, *J*=2); MS [M+1]⁺: 358 |
| 59 | 3-(4-*tert*-Butylphenyl)-2-(4-inethoxyphenyl)-7-methylimidazo[1,2-*a*]pyrimidine; ¹H-NMR: 1.40 (s, 9H), 2.63 (s, 3H), 3.80 (s, 3H), 6.63 (d, 1H, *J*=7), 6.83 (d, 2H, *J*=9), 7.36 (d, 2H, *J*=8), 7.53 (d, 2H, *J*=8), 7.72 (d, 2H, *J*=9), 8.09 (d, 1H, *J*=7); MS [M+1]⁺: 372 |
| 60 | 3-(4-Bromophenyl)-2-(4-methoxyphenyl)imidazo[1,2-*a*]pyrimidine; ¹H-NMR: 3.81 (s, 3H), 6.82 (dd, 1H, *J*=6.5, *J*=4), 6.85 (d, 2H, *J*=9), 7.33 (d, 2H, *J*=8), 7.67 (d, 2H, *J*=8), 7.69 (d, 2H, *J*=7.5), 8.21 (dd, 1H, *J*=6.5, *J*=2), 8.55 (dd, 1H, *J*=4, *J*=2); MS [M/M+2]⁺: 380/382 |
| 61 | 3-(4-Bromophenyl)-2-(4-methoxyphenyl)-7-methylimidazo[1,2-*a*]pyrimidine; ¹H-NMR: 2.63 (s, 3H), 3.81 (s, 3H), 6.67 (d, 1H, *J*=7), 6.84 (d, 2H, *J*=8.5), 7.31 (d, 2H, *J*=7.5), 7.68 (d, 4H, *J*=8.5), 8.05 (d, 1H, *J*=7); MS [M/M+2]⁺: 394/396 |
| 62 | 2-(4-Methoxyphenyl)-3-(4-nitrophenyl)imidazo[1,2-*a*]pyrimidine; ¹H-NMR: 3.82 (s, 3H), 6.86 (d, 2H, *J*=8.5), 6.90 (dd, 1H, *J*=7, *J*=4), 7.60 (d, 2H, *J*=8.5), 7.66 (d, 2H, *J*=9), 8.35 (dd, 1H, *J*=7, *J*=2), 8.38 (d, 2H, *J*=9), 8.60 (dd, 1H, *J*=4, *J*=2); MS [M+1]⁺: 347 |
| 63 | 2-(4-Methoxyphenyl)-7-methyl-3-(4-nitrophenyl)imidazo[1,2-*a*]pyrimidine; ¹H-NMR: 2.67 (s, 3H), 3.82 (s, 3H), 6.76 (d, 1H, *J*=7), 6.86 (d, 2H, *J*=9), 7.60 (d, 2H, *J*=9), 7.64 (d, 2H, *J*=9), 8.19 (d, 1H, *J*=7), 8.37 (d, 2H, *J*=9); MS [M+1]⁺: 361 |
| 64 | 4-[2-(4-Methoxyphenyl)imidazo[1,2-*a]*pyrimidin-3-yl]phenylamine; ¹H-NMR: 3.80 (s, 3H), 6.76 (dd, 1H, *J*=7, *J*=4), 6.83 (d, 2H, *J*=8.5), 6.84 (d, 2H, *J*=9), 7.21 (d, 2H, *J*=8.5), 7.76 (d, 2H, *J*=8.5), 8.19 (dd, 1H, *J*=7, *J*=2), 8.50 (dd, 1H, *J*=4, *J*=2); MS [M+1]⁺: 317 |
| 65 | 4-[2-(4-Methoxyphenyl)-7-methylimidazo[1,2-*a*]pyrimidin-3-yl]phenylamine; ¹H-NMR: 2.61 (s, 3H), 3.79 (s, 3H), 6.62 (d, 1H, *J*=7), 6.81 (d, 2H, *J*=8.5), 6.82 (d, 2H, *J*=9), 7.19 (d, 2H, *J*=8.5), 7.75 (d, 2H, *J*=9), 8.02 (d, 1H, *J*=7); MS [M+1]⁺: 331 |
| 66 | 3-(3-Chloro-4-methoxyphenyl)-2-(4-methoxyphenyl)-7-methylimidazo[1,2-*a*]pyrimidine; ¹H-NMR: 2.62 (s, 3H), 3.89 (s, 3H), 3.91 (s, 3H), 6.80 (d, 1H, *J*=7), 6.84 (d, 2H, *J*=9), 7.08 (d, 1H, *J*=8.5), 7.28 (dd, 1H, *J*=8.5, *J*=2), 7.45 (d, 1H, *J*=2), 7.69 (d, 2H, *J*=9), 8.01 (d, 1H, *J*=7); MS [M/M+2]⁺: 380/382 |
| 67 | 3-(3-Methoxyphenyl)-2-(4-methylsulfanylphenyl)imidazo[1,2-*a*]pyrimidine; ¹H-NMR: 2.47 (s, 3H), 3.81 (s, 3H), 6.80 (d, 1H, *J*=7), 6.96 (dd, 1H, *J*=2, *J*=1), 7.03 (tdd, 1H, *J*=9, *J*=2, *J*=1), 7.17 (d, 2H, *J*=8.5), 7.52 (t, 1H, *J*=8), 7.71 (d, 2H, *J*=8.5), 8.24 (dd, 1H, *J*=7, *J*=2), 8.54 (dd, 1H, *J*=4, *J*=2); MS [M+1]⁺: 348 |
| 68 | 3-(3-Methoxyphenyl)-7-methyl-2-(4-methylsulfanylphenyl)imidazo[1,2-*a*]pyrimidine; ¹H-NMR: 2.47 (s, 3H), 2.64 (s, 3H), 3.81 (s, 3H), 6.66 (d, 1H, *J*=7), 6.95 (dd, 1H, *J*=2.5, *J*=2), 7.02 (tdd, 1H, *J*=9, *J*=2, *J*=1), 7.17 (d, 2H, *J*=8.5), 7.45 (t, 1H, *J*=9), 7.71 (d, 2H, *J*=8.5), 8.08 (d, 1H, *J*=7); MS [M+1]⁺: 362 |
| 69 | 3-(4-Chlorophenyl)-2-(4-methylsulfanylphenyl)imidazo[1,2-a]pyrimidine; ¹H-NMR: 2.48 (s, 3H), 6.83 (dd, 1H, *J*=7, *J*=4), 7.18 (d, 2H, *J*=8.5), 7.39 (d, 2H, *J*=8.5), 7.54 (d, 2H, *J*=8), 7.63 (d, 2H, *J*=8), 8.20 (dd, 1H, *J*=7, *J*=2), 8.56 (dd, 1H, *J*=4, *J*=2); MS [M/M+2]⁺: 352/354 |
| 70 | 3-(4-Chlorophenyl)-7-methyl-2-(4-methylsulfanylphenyl)imidazo[1,2-*a*]pyrimidine; ¹H-NMR: 2.47 (s, 3H), 2.64 (s, 3H), 6.68 (d, 1H, *J*=7), 7.17 (d, 2H, *J*=8), 7.37 (d, 2H, *J*=8), 7.52 (d, 2H, *J*=8), 7.64 (d, 2H, *J*=8), 8.05 (dd, 1H, *J*=7); MS [M/M+2]⁺: 366/368 |
| 71 | 4-[2-(4-Methylsulfanylphenyl)imidazo[1,2-a]pyrimidin-3-yl]benzonitrile; ¹H-NMR: 2.48 (s, 3H), 6.89 (dd, 1H, *J*=7, *J*=4), 7.18 (d, 2H, *J*=8.5), 7.58 (d, 2H, *J*=8.5), 7.59 (d, 2H, *J*=8.5), 7.83 (d, 2H, *J*=8.5), 8.29 (dd, 1H, *J*=7, *J*=2), 8.60 (dd, 1H, *J*=4, *J*=2); MS [M+1]⁺: 343 |
| 72 | 3-(4-Methoxyphenyl)-2-(4-methylsulfanylphenyl)imidazo[1,2-*a*]pyrimidin-7-ol; ¹H-NMR: 2.30-2.50 (br, 1H), 2.43 (s, 3H), 3.87 (s, 3H), 6.08 (d, 1H, *J*=8), 7.02 (d, 2H, *J*=8.5), 7.12 (d, 2H, *J*=8.5), 7.28 (d, 2H, *J*=8.5), 7.41 (d, 2H, *J*=8.5), 7.59 (d, 1H, *J*=8); MS [M+1]⁺: 364 |
| 73 | 7-Chloro-3-(4-methoxyphenyl)-2-(4-methylsulfanylphenyl)imidazo[1,2-*a*]pyrimidine; ¹H-NMR: 2.46 (s, 3H), 3.90 (s, 3H), 6.79 (d, 1H, *J*=7), 7.07 (d, 2H, *J*=8.5), 7.15 (d, 2H, *J*=8.5), 7.34 (d, 2H, *J*=8.5), 7.66 (d, 2H, *J*=8.5), 8.06 (d, 1H, *J*=7); MS [M/M+2]⁺: 382/384 |
| 74 | 5-Chloro-3-(4-methoxyphenyl)-2-(4-methylsulfanylphenyl)imidazo[1,2-*a*]pyrimidine; ¹H-NMR: 2.45 (s, 3H), 3.91 (s, 3H), 6.82 (d, 1H, *J*=4.5), 6.98 (d, 2H, *J*=8.5), 7.12 (d, 2H, *J*=8.5), 7.36 (d, 2H, *J*=8.5), 7.59 (d, 2H, *J*=8.5), 8.39 (d, 1H, *J*=4.5); MS [M/M+2]⁺: 382/384 |
| 75 | 5,7-Dichloro-3-(4-methoxyphenyl)-2-(4-methylsulfanylphenyl)imidazo[1,2-*a*]pyrimidine; ¹H-NMR: 2.45 (s, 3H), 3.89 (s, 3H), 5.67 (s, 1H), 6.88 (d, 2H, *J*=9), 7.07 (d, 2H, *J*=8.5), 7.14 (d, 2H, *J*=8.5), 7.83 (d, 2H, *J*=9); MS [M/M+2/M+4]⁺:416/418/420 |
| 76 | 7-Chloro-5-methoxy-3-(4-methoxyphenyl)-2-(4-methylsulfanylphenyl)imidazo[1,2-*a*]pyrimidine; ¹H-NMR: 2.42 (s, 3H), 3.81 (s, 3H), 3.84 (s, 3H), 5.60 (s, 1H), 6.85 (d, 2H, *J*=9), 7.10 (d, 2H, *J*=8.5), 7.15 (d, 2H, *J*=8.5), 7.84 (d, 2H, *J*=9); MS [M/M+2]⁺: 412/414 |
| 77 | 7-Methoxy-3-(4-methoxyphenyl)-5-methyl-2-(4-methylsulfanylphenyl)imidazo[1,2-*a*]pyrimidine; ¹H-NMR: 2.36 (s, 3H), 2.45 (s, 3H), 3.58 (s, 3H), 3.82 (s, 3H), 5.85 (s, 1H), 6.90 (d, 2H, *J*=8.5), 7.10 (d, 2H, *J*=8.5), 7.15 (d, 2H, *J*=8.5), 7.19 (d, 2H, *J*=8.5); MS [M+1]⁺: 392 |
| 78 | 6-Bromo-3-(4-methoxyphenyl)-2-(4-methylsulfanylphenyl)imidazo[1,2-*a*]pyrimidine; ¹H-NMR: 2.47 (s, 3H), 3.92 (s, 3H), 7.10 (d, 2H, *J*=8.5), 7.16 (d, 2H, *J*=8.5), 7.36 (d, 2H, *J*=8.5), 7.68 (d, 2H, *J*=8.5), 8.25 (d, 1H, *J*=2), 8.49 (d, 1H, *J*=2); MS [M/M+2]⁺: 426/428 |
| 79 | 3-(3-Fluoro-4-methoxyphenyl)-2-(4-methylsulfanylphenyl)imidazo[1,2-a]pyrimidine; ¹H-NMR: 2.47 (s, 3H), 3.98 (s, 3H), 6.82 (dd, 1H, *J*=7, *J*=4), 7.09-7.15 (m, 3H), 7.17 (d, 2H, *J*=8.5), 7.67 (d, 2H, *J*=8.5), 8.18 (dd, 1H, *J*=7, *J*=2), 8.54 (dd, 1H, *J*=4, *J*=2); MS [M+1]⁺: 366 |
| 80 | 3-(3-Fluoro-4-methoxyphenyl)-7-methyl-2-(4-methylsulfanylphenyl)imidazo[1,2-*a]*pyrimidine; ¹H-NMR: 2.48 (s, 3H), 2.64 (s, 3H), 3.99 (s, 3H), 6.68 (d, 1H, *J*=7), 7.09-7.19 (m, 3H), 7.17 (d. 2H, *J*=9), 7.68 (d, 2H, *J*=9), 8.03 (d, 1H, *J*=7); MS [M+1]⁺: 380 |
| 81 | 3-(3-Chloro-4-methoxyphenyl)-2-(4-methylsulfanylphenyl)imidazo[1,2-*a*]pyrimidine; ¹H-NMR: 2.47 (s, 3H), 4.00 (s, 3H), 6.82 (dd, 1H, *J*=7, *J*=4), 7.08 (d, 1H, *J*=8.5), 7.17 (d, 2H, *J*=9), 7.29 (dd, 1H, *J*=8.5, *J*=2.5), 7.47 (d, 1H, *J*=2.5), 7.67 (d, 2H, *J*=9), 8.17 (dd, 1H, *J*=7, *J*=2), 8.54 (dd, 1H, *J*=4, *J*=2); MS [M/M+2]⁺: 382/384 |
| 82 | 3-(3-Chloro-4-methoxyphenyl)-7-methyl-2-(4-methylsulfanylphenyl)imidazo[1,2-*a*]pyrimidine; ¹H-NMR: 2.47 (s, 3H), 2.63 (s, 3H), 4.00 (s, 3H), 6.68 (d, 1H, *J*=7), 7.08 (d, 1H, *J*=8.5), 7.17 (d. 2H, *J*=9), 7.28 (dd, 1H, *J*=8.5, *J*=2), 7.45 (d, 1H, *J*=2), 7.67 (d, 2H, *J*=9), 8.01 (d, 1H, *J*=7); MS [M/M+2]⁺: 382/383 |
| 83 | 3-(2-Fluoro-4-methoxyphenyl)-2-(4-methylsulfanylphenyl)imidazo[1,2-*a*]pyrimidine; ¹H-NMR: 2.48 (s, 3H), 3.91 (s, 3H), 6.82-6.88 (m, 3H), 7.19 (d, 2H, *J*=8.5), 7.27 (t, 1H, *J*=8.5), 7.70 (d, 2H, *J*=8.5), 8.04 (dt, 1H, *J*=6.5, *J*=2), 8.57 (dd, 1H, *J*=4, *J*=2); MS [M+1]⁺: 366 |
| 84 | 3-(3-Fluoro-4-methylsulfanylphenyl)-2-(4-methylsulfanylphenyl)imidazo[1,2-*a*]pyrimidine; ¹H-NMR: 2.49 (s, 3H), 2.56 (s, 3H), 6.84 (dd, 1H, *J*=7, *J*=4), 7.13 (dd, 1H, *J*=10.5, *J*=2), 7.19 (d, 2H, *J*=8.5), 7.20 (dd, 1H, *J*=8, *J*=2), 7.38 (t, 1H, *J*=8), 7.67 (d, 2H, *J*=8.5), 8.24 (dd, 1H, *J*=7, *J*=2), 8.56 (dd, 1H, *J*=4, *J*=2); MS [M+1]⁺: 382 |
| 85 | 3-(3-Fluoro-4-methylsulfanylphenyl)-7-methyl-2-(4-methylsulfanylphenyl)imidazo[1,2-*a*]pyrimidine; ¹H-NMR: 2.48 (s, 3H), 2.55 (s, 3H), 2.64 (s, 3H), 6.69 (d, 1H, *J*=7), 7.11 (dd, 1H, *J*=10, *J*=2), 7.17 (d, 2H, *J*=9), 7.18 (dd, 1H, *J*=8, *J*=2), 7.35 (t, 1H, *J*=8), 7.66 (d, 2H, *J*=9), 8.08 (d, 2H, *J*=7); MS [M+1]⁺: 396 |
| 86 | 3-(3-Chloro-4-methylsulfanylphenyl)-2-(4-methylsulfanylphenyl)imidazo[1,2-*a*]pyrimidine; ¹H-NMR: 2.49 (s, 3H), 2.56 (s, 3H), 6.84 (dd, 1H, *J*=7, *J*=4), 7.19 (d, 2H, *J*=8.5), 7.29 (d, 1H, *J*=8), 7.32 (dd, 1H, *J*=8, *J*=1.5), 7.46 (d, 1H, *J=*1.5), 7.68 (d, 2H, *J*=8.5), 8.21 (dd, 1H, *J*=7, *J=*2), 8.56 (dd, 2H, *J*=4, *J*=2); MS [M/M+2]⁺: 398/400 |
| 87 | 3-(3-Chloro-4-methylsulfanylphenyl)-7-methyl-2-(4-methylsulfanylphenyl)imidazo[1,2-a]pyrimidine; ¹H-NMR: 2.48 (s, 3H), 2.55 (s, 3H), 2.64 (s, 3H), 6.69 (d, 1H, *J*=7), 7.18 (d, 2H, *J*=8.5), 7.27 (d, 1H, *J*=8.5), 7.30 (dd, 1H, *J*=8.5, *J*=1.5), 7.43 (d, 1H, *J*=1.5), 7.67 (d, 2H, *J*=8.5), 8.06 (d, 2H, *J*=7); MS [M/M+2]⁺. 412/414 |
| 88 | 3-(3-Methyl-4-methylsulfanylphenyl)-2-(4-methylsulfanylphenyl)imidazo[1,2-*a*]pyrimidine; ¹H-NMR: 2.37 (s, 3H), 2.48 (s, 3H), 2.56 (s, 3H), 6.79 (dd, 1H, *J*=7, *J*=4), 7.18 (d, 2H, *J*=8.5), 7.20-7.35 (m, 3H), 7.72 (d, 2H, *J*=8.5), 8.21 (dd, 1H, *J*=7, *J*=2), 8.53 (dd, 2H, *J*=4, *J*=2); MS [M+1]⁺: 378 |
| 89 | 7-Methyl-3-(3-methyl-4-methylsulfanylphenyl)-2-(4-methylsulfanylphenyl)imidazo[1,2-*a*]pyrimidine; ¹H-NMR: 2.37 (s, 3H), 2.47 (s, 3H), 2.55 (s, 3H), 2.63 (s, 3H), 6.65 (d, 1H, *J*=7), 7.17 (d, 2H, *J*=8.5), 7.18-7.35 (m, 3H), 7.71 (d, 2H, *J*=8.5), 8.04 (d, 1H, *J*=7); MS [M+1]⁺: 392 |
| 90 | 2-(3-Bromo-4-methylsulfanylphenyl)-3-(4-methoxyphenyl)imidazo[1,2-*a*]pyrimidine; ¹H-NMR: 2.45 (s, 3H), 3.91 (s, 3H), 6.80 (dd, 1H, *J*=7, *J*=2), 7.00 (d, 1H, *J*=8), 7.09 (d, 2H, *J*=8), 7.35 (d, 2H, *J*=8), 7.61 (d, 1H, *J*=8), 8.06 (s, 1H), 8.17 (dd, 1H, *J*=7, *J*=4), 8.53 (dd, 1H, *J*=4, *J*=2); MS [M/M+2]⁺: 426/428 |
| 91 | 2-(3-Chloro-4-ethylsulfanylphenyl)-3-(4-methylsulfanylphenyl)imidazo[1,2-*a*]pyrimidine; ¹H-NMR: 1.37 (t, 3H, *J*=7.5), 2.58 (s, 3H), 2.96 (q, 2H, *J*=7.5), 6.84 (dd, 1H, *J*=7, *J*=4), 7.14 (d, 1H, *J*=8.5), 7.35 (d, 2H, *J*=8.5), 7.42 (d, 2H, *J*=8.5), 7.55 (dd, 1H, *J*=8.5, *J*=2), 7.87 (d, 1H, *J*=2), 8.22 (dd, 1H, *J*=7, *J*=2), 8.58 (dd, 1H, *J*=4, *J*=2) |
| 92 | 2-(3-Chloro-4-isopropylsulfanylphenyl)-3-p-tolylimidazo[1,2-*a*]pyrimidine; ¹H-NMR: 1.34 (d, 6H, *J*=7), 2.48 (s, 3H), 3.44-3.55 (m, 1H), 6.80 (dd, 1H, *J*=7, *J*=4), 7.24 (d, 1H, *J*=8.5), 7.32 (d, 2H, *J*=8), 7.38 (d, 2H, *J*=8), 7.55 (dd, 1H, *J*=8.5, *J*=2), 7.89 (d, 1H, *J*=2); 8.19 (dd, 1H, *J*=7, *J*=2), 8.53 (dd, 1H, *J*=4, *J*=2); MS [M/M+2]⁺: 394/396 |
| 93 | 2-(3-Chloro-4-propylsulfanylphenyl)-3-p-tolylimidazo[1,2-a]pyrimidine; ¹H-NMR: 1.06 (t, 3H, *J*=7.5), 1.66-1.78 (m, 2H), 2.48 (s, 3H), 2.90 (t, 2H, *J*=7.5), 6.80 (dd, 1H, *J*=7, *J*=4), 7.13 (d, 1H, 93 *J*=8.5), 7.32 (d, 2H, *J*=8), 7.38 (d, 2H, *J*=8), 7.56 (dd, 1H, *J*=8, *J*=2), 7.86 (d, 1H, *J*=2), 8.19 (dd, 1H, *J*=7, *J*=2), 8.55 (dd, 1H, *J*=4, *J*=2); MS [M/M+2]⁺: 394/396 |
| 94 | 2-(3-fluorophenyl)-3-(4-methylsulfanylphenyl)imidazo[1,2-*a*]pyrimidine; ¹H-NMR: 2.57 (s, 3H), 6.82 (dd, 1H, *J*=7, *J*=4), 6.97 (tdd, 1H, *J*=8.5, *J*=2.5, *J*=1), 7.23 (td, 1H, *J*=8.5, *J*=6), 7.35 (d, 2H, *J*=8.5), 7.41 (d, 2H, *J*=8.5), 7.51-7.55 (m, 2H), 8.22 (dd, 1H, *J*=7, *J*=4), 8.57 (dd, 1H, *J*=4, *J*=2); MS [M+1]⁺: 336 |
| 95 | 2-(3-Fluorophenyl)-7-methyl-3-(4-methylsulfanylphenyl) imidazo [1,2-*a*] pyrimidine; ¹H-NMR: 2.57 (s, 3H), 2.64 (S, 3H), 6.68 (d, 1H, *J*=7), 6.94 (tdd, 1H, *J*=8.5, *J*=2.5, *J*=1), 7.22 (td, 1H, *J*=8.5, *J*=6), 7.33 (d, 2H, *J*=8.5), 7.40 (d, 2H, *J*=8.5), 7.49-7.55 (m, 2H), 8.05 (dd, 1H, *J*=7); MS [M+1]⁺: 366 |
| 96 | 2-(3-Chlorophenyl)-3-(4-methylsulfanylphenyl)imidazo[1,2-*a*]pyrimidine; ¹H-NMR: 2.57 (s, 3H), 6.84 (dd, 1H, *J*=7, *J*=4), 7.19 (t, 1H, *J*=8), 7.26 (d, 1H, *J*=8), 7.35 (d, 2H, *J*=8.5), 7.41 (d, 2H, *J*=8.5), 7.54 (d, 1H, *J*=7.5), 7.89 (s, 1H), 8.23 (dd, 1H, *J*=7, *J*=4), 8.57 (dd, 1H, *J*=4, *J*=2); MS [M/M+2]⁺: 352/354 |
| 97 | 2-(3-Chlorophenyl)-7-methyl-3-(4-methylsulfanylphenyl)imidazo[1,2-*a*]pyrimidine; ¹H-NMR: 2.57 (s, 3H), 2.64 (s, 3H), 6.69 (d, 1H, *J*=7), 7.18 (t, 1H, *J*=7.5), 7.22 (dd, 1H, *J*=7, *J*=1.5), 7.33 (d, 2H, *J*=8.5), 7.40 (d, 2H, *J*=8.5), 7.54 (dt, 1H, *J*=7, *J*=1.5), 7.87 (t, 1H. *J*=1.5), 8.06 (d, 1H, *J*=7); MS [M/M+2]⁺: 366/368 |
| 98 | 4-[3-(4-Methylsulfanylphenyl)imidazo[1,2-*a*]pyrimidin-2-yl]benzonitrile; ¹H-NMR: 2.57 (s, 3H), 6.86 (dd, 1H, *J*=7, *J*=4), 7.33 (d, 2H, *J*=8), 7.41 (d, 2H, *J*=8), 7.58 (d, 2H, *J*=8.5), 7.88 (d, 2H, *J*=8.5), 8.21 (dd, 1H, *J*=7, *J*=2), 8.59 (dd, 1H, *J*=4, *J*=2); MS [M+1]⁺: 343 |
| 99 | 2-(3-Methoxyphenyl)-3-(4-methylsulfanylphenyl)imidazo[1,2-*a*]pyrimidine; ¹H-NMR: 2.56 (s, 3H), 3.76 (s, 3H), 6.81 (dd, 1H, *J*=7, *J*=4), 6.82 (ddd, 1H, *J*=7.5, *J*=2.5, *J*=1), 7.16 (t, 1H, *J*=7.5), 7.24 (dt, 1H, *J*=7.5, *J*=1), 7.36 (d, 2H, *J*=9), 7.37 (d, 2H, *J*=9), 7.47 (dd, 1H, *J*=2.5, *J*=1), 8.22 (dd, 1H, *J*=7, *J*=2), 8.55 (dd, 1H, *J*=4, *J*=2) |
| 100 | 2-(3-Methoxyphenyl)-7-methyl-3-(4-methylsulfanylphenyl)imidazo[1,2-*a*]pyrimidine; ¹H-NMR: 2.56 (s, 3H), 2.64 (s, 3H), 3.76 (s, 3H), 6.67 (d, 1H, *J*=7), 6.81 (ddd, 1H, *J*=8, *J*=2.5, *J*=1.5), 7.15 (t, 1H, *J*=8), 7.23 (dt, 1H, *J*=8, *J*=1.5), 7.35 (d, 2H, *J*=8.5), 7.39 (d, 2H, *J*=8.5), 7.50 (dd, 1H, *J*=2.5, *J*=1.5), 8.05 (d, 1H, *J*=7); MS [M+1]⁺: 362 |
| 101 | 2-(4-Methoxyphenyl)-3-(4-methylsulfanylphenyl)imidazo[1,2-*a*]pyrimidin-7-ol; ¹H-NMR: 2.30-2.50 (br, 1H), 2.43 (s, 3H), 3.87 (s, 3H), 6.08 (d, 1H, *J*=8), 7.02 (d, 2H, *J*=8.5), 7.12 (d, 2H, *J*=8.5), 7.28 (d, 2H, *J*=8.5), 7.41 (d, 2H, *J*=8.5), 7.59 (d, 1H, *J*=8); MS [M+1]⁺: 364 |
| 102 | 5-Methoxy-2-(4-methoxyphenyl)-3-(4-methylsulfanylphenyl)imidazo[1,2-*a*]pyrimidin-7-ol; ¹H-NMR: 2.51 (s, 3H), 3.80 (s, 3H), 3.87 (s, 3H), 5.32 (s, 1H), 6.83 (d, 2H, *J*=9), 7.20 (d, 2H, *J*=9), 7.22 (d, 2H, *J*=8.5), 7.32 (d, 2H, *J*=8.5); MS [M+1]⁺: 393 |
| 103 | 3-(4-Methoxyphenyl)-2-(4-methylsulfanylphenyl)imidazo[1,2-*a*]pyrimidine; ¹H-NMR: 2.47 (s, 3H), 3.90 (s, 3H), 6.81 (dd, 1H, *J*=7, *J*=4), 7.07 (d, 2H, *J*=9), 7.16 (d, 2H, *J*=9), 7.36 (d, 2H, *J*=9), 7.70 (d, 2H, *J*=9), 8.18 (dd, 1H, *J*=6.5, *J*=2), 8.54 (dd, 1H, *J*=4, *J*=2); MS [M+1]⁺: 348 |
| 104 | 2-(4-Methoxyphenyl)-3-(4-methylsulfanylphenyl)imidazo[1,2-*a*]pyrimidin-7-ylamine; ¹H-NMR (d₆-DMSO): 2.47 (s, 3H), 3.75 (s, 3H), 6.12 (s, 1H), 6.91 (d, 2H, *J*=8.5), 7.25 (s, 4H), 7.34 (d, 2H, *J*=8.5), 8.37 (s, 1H), 11.49 (s, 2H) ; MS [M+1]⁺: 363 |
| 105 | 2-(4-Methoxyphenyl)-5-methyl-3-(4-methylsulfanylphenyl)imidazo[1,2-*a*]pyrimidin-7-ol; ¹H-NMR: 1.90 (s, 3H), 2.51 (s, 3H), 3.77 (s, 3H), 5.55 (s, 1H), 6.74 (d, 2H, *J*=9), 7.22 (d, 2H, *J*=8.5), 7.29 (d, 2H, *J*=8.5), 7.32 (d, 2H, *J*=8.5); MS [M+1]⁺: 378 |
| 106 | 2-(4-Methoxyphenyl)-5-methyl-3-(4-methylsulfanylphenyl)imidazo[1,2-*a*]pyrimidine-7-thiol; ¹H-NMR: 2.02 (s, 3H), 2.17 (s, 1H), 2.53 (s, 3H), 3.77 (s, 3H), 6.73 (d, 2H, *J*=9), 6.91 (s, 1H), 7.17 (d, 2H, *J*=9), 7.18 (d, 2H, *J*=8), 7.30 (d, 2H, *J*=8); MS [M+1]⁺: 394 |
| 107 | 7-Methoxy-2-(4-methoxyphenyl)-5-methyl-3-(4-methylsulfanylphenyl)imidazo[1,2-*a*]pyrimidine; ¹H-NMR: 2.40 (s, 3H), 2.52 (s, 3H), 3.78 (s, 3H), 3.88 (s, 3H), 5.59 (s, 1H), 6.79(d, 2H, *J*=9), 7.23 (d, 2H, *J*=8.5), 7.32 (d, 2H, *J*=8.5), 7.45 (d, 2H, *J*=9); MS [M+1]⁺: 392 |
| 108 | 2-(4-Methoxyphenyl)-3-(4-methylsulfanylphenyl)-6-nitroimidazo[1,2-a]pyrimidine; ¹H-NMR: 2.48 (s, 3H), 3.95 (s, 3H), 7.15 (d, 2H, *J*=8.5), 7.18 (d, 2H, *J*=8.5), 7.39 (d, 2H, *J*=8.5), 7.72 (d, 2H, *J*=8.5), 9.09 (d, 1H, *J*=2), 9.27 (d, 1H, *J*=2); MS [M+1]⁺: 393 |
| 109 | 5-Chloro-2-(4-methoxyphenyl)-3-(4-methylsulfanylphenyl)imidazo[1,2-*a*]pyrimidin-7-ylamine; ¹H-NMR (d₆-DMSO): 2.48 (s, 3H), 3.72 (s, 3H), 6.47 (s, 2H), 6.84 (d, 2H, *J*=9), 7.20 (s, 4H), 7.23 (d, 2H, *J*=9); MS [M/M+2]⁺: 397/399 |
| 110 | 2- (4-Methoxyphenyl) -*N*⁵,*N*⁵-dimethyl-3-(4-methylsulfanylphenyl)imidazo[1,2-*a*]pyrimidine-5,7-diamine; ¹H-NMR (d₆-DMSO): 2.47 (s, 3H), 2.93 (s, 6H), 3.71 (s, 3H), 5.62 (s, 2H), 5.94 (s, 1H), 6.82 (d, 2H, *J*=9), 7.11 (d, 4H, *J*=8.5), 7.20 (d, 2H, *J*=8.5); MS [M+1]⁺: 406 |
| 111 | 7-Chloro-2-(4-methoxyphenyl)-5-methyl-3-(4-methylsulfanylphenyl)imidazo[1,2-*a*]pyrimidine; ¹H-NMR: 2.56 (s, 3H), 3.78 (s, 3H), 6.70 (s, 1H), 6.79 (d, 2H, *J*=9), 7.29 (d, 2H, *J*=8.5), 7.35 (d, 2H, *J*=8.5), 7.60 (d, 2H, *J*=9); MS [M/M+2]⁺: 396/398 |
| 112 | 6-Bromo-2-(4-methoxyphenyl)-3-(4-methylsulfanylphenyl) imidazo [1,2-*a*] pyrimidine; ¹H-NMR: 2.58 (s, 3H), 3.81 (s, 3H), 6.85 (d, 2H, *J*=9), 7.35 (d, 2H, *J*=8.5), 7.42 (d, 2H, *J*=8.5), 7.69 (d, 2H, *J*=9), 8.28 (d, 1H, *J*=2), 8.48 (d, 1H, *J*=2); MS [M/M+2]⁺: 426/428 |
| 113 | 2-(3-*tert*-Butyl-4-methoxyphenyl)-3-(4-methylsulfanylphenyl)imidazo[1,2-*a*]pyrimidine; ¹H-NMR: 1.24 (s, 9H), 2.56 (s, 3H), 3.83 (s, 3H), 6.79 (dd, 1H, *J*=7, *J*=4), 6.83 (d, 1H, *J*=9), 7.37 (d, 2H, *J*=8.5), 7.41 (d, 2H, *J*=8.5), 7.66 (dd, 1H, *J*=8.5, *J*=2.5), 7.66 (d, 1H, *J*=2.5), 8.20 (dd, 1H, *J*=7, *J*=2), 8.52 (dd, 1H, *J*=4, *J*=2); MS [M+1]⁺: 404 |
| 114 | 2-(3-*tert*-Butyl-4-methoxyphenyl)-7-methyl-3-(4-methylsulfanylphenyl)imidazo[1,2-*a*]pyrimidine; ¹H-NMR: 1.25 (s, 9H), 2.55 (s, 3H), 2.63 (s, 3H), 3.82 (s, 3H), 6.64 (d, 1H, *J*=7), 6.81 (d, 1H, *J*=8.5), 7.35 (d, 2H, *J*=8.5), 7.40 (d, 2H, *J*=8.5), 7.63 (dd, 1H, *J*=8.5, *J*=2.5), 7.70 (d, 1H, *J*=2.5), 8.04 (d, 1H, *J*=7); MS [M+1]⁺: 418 |
| 115 | 2-(6-Methoxybiphenyl-3-yl)-3-(4-methylsulfanylphenyl)imidazo[1,2-*a*]pyrimidine; ¹H-NMR: 2.57 (s, 3H), 3.82 (s, 3H), 6.80 (dd, 1H, *J*=7, *J*=4), 6.93 (d, 1H, *J*=8.5), 7.26-7.44 (m, 9H), 7.72 (dd, 1H, *J*=8.5, *J*=2.5), 7.76 (d, 1H, *J*=2.5), 8.24 (dd, 1H, *J*=7, *J*=2), 8.53 (dd, 1H, *J*=4, *J*=2); MS [M+1]⁺: 424 |
| 116 | 2-(6-Methoxybiphenyl-3-yl)-7-methyl-3-(4-methylsulfanylphenyl)imidazo[1,2-*a*]pyrimidine; ¹H-NMR: 2.57 (s, 3H), 2.63 (s, 3H), 3.81 (s, 3H), 6.66 (d, 1H, *J*=7), 6.92 (d, 1H, *J*=8.5), 7.26-7.44 (m, 9H), 7.70 (dd, 1H, *J*=8.5, *J*=2.5), 7.76 (d, 1H, *J*=2.5), 8.08 (d, 1H, *J*=7); MS [M+1]⁺: 438 |
| 117 | 2-(4-Methoxy-2-methylphenyl)-3-(4-methylsulfanylphenyl)imidazo[1,2-*a*]pyrimidine; ¹H-NMR: 2.17 (s, 3H), 2.51 (s, 3H), 3.80 (s, 3H), 6.69 (dd, 1H, *J*=8.5, *J*=2.5), 6.74 (d, 1H, *J*=2.5), 6.85 (dd, 1H, *J*=7, *J*=4), 7.20 (d, 2H, *J*=8.5), 7.25 (d, 1H, *J*=8.5), 7.27 (d, 2H, *J*=8.5), 8.52 (dd, 1H, *J*=7, *J*=2), 8.57 (dd, 1H, *J*=4, *J*=2); MS [M+1]⁺: 362 |
| 118 | 2-(4-Methoxy-2-methylphenyl)-7-methyl-3-(4-methylsulfanylphenyl)imidazo[1,2-*a*]pyrimidine; ¹H-NMR: 2.20 (s, 3H), 2.50 (s, 3H), 2.65 (s, 3H), 3.80 (s, 3H), 6.66 (dd, 1H, *J*=8.5, *J*=3), 6.72 (d, 1H, *J*=7), 6.73 (d, 1H, *J*=3), 7.18 (d, 2H, *J*=8.5), 7.20 (d, 1H, *J*=8.5), 7.26 (d, 2H, *J*=8.5), 8.36 (d, 1H, *J*=7); MS [M+1]⁺: 376 |
| 119 | 2-(2-Fluoro-4-methoxyphenyl)-3-(4-methylsulfanylphenyl)imidazo[1,2-*a*]pyrimidine; ¹H-NMR: 2.53 (s, 3H), 3.81 (s, 3H), 6.55 (dd, 1H, *J*=12, *J*=2.5), 6.77 (dd, 1H, *J*=8.5, *J*=2.5), 6.85 (dd, 1H, *J*=7, *J*=4), 7.27 (d, 2H, *J*=8.5), 7.32 (d, 2H, *J*=8.5), 7.70 (t, 1H, *J*=8.5), 8.42 (dd, 1H, *J*=7, *J*=2), 8.56 (dd, 1H, *J*=4, *J*=2); MS [M+1]⁺: 366 |
| 120 | 2-(2-Fluoro-4-methoxyphenyl)-7-methyl-3-(4-methylsulfanylphenyl)imidazo[1,2-*a*]pyrimidine; ¹H-NMR: 2.52 (s, 3H), 2.64 (s, 3H), 3.80 (s, 3H), 6.53 (dd, 1H, *J*=12, *J*=2.5), 6.71 (d, 1H, *J*=7), 6.76 (dd, 1H, *J*=8.5, *J*=2.5), 7.25 (d, 2H, *J*=8.5), 7.31 (d, 2H, *J*=8.5), 7.70 (t, 1H, *J*=8.5), 8.27 (d, 1H, *J*=7); MS [M+1]⁺: 380 |
| 121 | 3-(2-Chloro-4-methylsulfanylphenyl)-2-(4-methoxyphenyl)imidazo[1,2-*a*]pyrimidine; ¹H-NMR: 2.57 (s, 3H), 3.81 (s, 3H), 6.83 (dd, 1H, *J*=7, *J*=4), 6.85 (d, 2H, *J*=9), 7.22 (dd, 1H, *J*=8.5, *J*=2), 7.28 (d, 1H, *J*=8.5), 7.46 (d, 1H, *J*=2), 7.68 (d, 2H, *J*=9), 7.90 (dd, 1H, *J*=7, *J*=2), 8.57 (dd, 2H, *J*=4, *J*=2); MS [M/M+2]⁺: 382/384 |
| 122 | 3-(3-Fluoro-4-methylsulfanylphenyl)-2-(4-methoxyphenyl)imidazo[1,2-*a*]pyrimidine; ¹H-NMR: 2.55 (s, 3H), 3.74 (s, 3H), 6.92 (d, 2H, *J*=9), 7.00 (dd, 1H, *J*=7, *J*=4), 7.32 (dd, 1H, *J*=8, *J*=2), 7.42 (dd, 1H, *J*=10.5, *J*=2), 7.51 (t, 1H, *J*=8), 7.55 (d, 2H, *J*=9), 8.51 (dd, 1H, *J*=7, *J*=2), 8.55 (dd, 2H, *J*=4, *J*=2); MS [M+1]⁺: 366 |
| 123 | 3-(3-Fluoro-4-methylsulfanylphenyl)-2-(4-methoxyphenyl)-7-methylimidazo[1,2-*a*]pyrimidine; ¹H-NMR: 2.55 (s, 3H), 2.64 (s, 3H), 3.81 (s, 3H), 6.69 (d, 1H, *J*=7), 6.85 (d, 2H, *J*=9), 7.12 (dd, 2H, *J*=10.5, *J*=1.5), 7.19 (dd, 1H, *J*=8, *J*=1.5), 7.36 (t, 1H, *J*=8), 7.68 (d, 2H, *J*=9), 8.08 (d, 2H, *J*=7); MS [M+1] ⁺: 380 |
| 124 | 3-(3-Chloro-4-methylsulfanylphenyl)-2-(4-methoxyphenyl)imidazo[1,2-*a*]pyrimidine; ¹H-NMR: 2.56 (s, 3H), 3.81 (s, 3H), 6.83 (dd, 1H, *J*=7, *J*=4), 6.86 (d, 2H, *J*=9), 7.29 (d, 1H, *J*=8), 7.32 (dd, 1H, *J*=8, *J*=1.5), 7.46 (d, 1H, *J*=1.5), 7.70 (d, 2H, *J*=9), 8.21 (dd, 1H, *J*=7, *J*=2), 8.55 (dd, 2H, *J*=4, *J*=2); MS [M/M+2]⁺: 382/384 |
| 125 | 3-(3-Chloro-4-methylsulfanylphenyl)-2-(4-methoxyphenyl)-7-methylimidazo[1,2-*a*]pyrimidine; ¹H-NMR: 2.55 (s, 3H), 2.64 (s, 3H), 3.81 (s, 3H), 6.69 (d, 1H, *J*=7), 6.85 (d, 2H, *J*=9), 7.27 (d, 1H, *J*=8), 7.31 (dd, 1H, *J*=8, *J*=1.5), 7.44 (d, 1H, *J*=1.5), 7.69 (d, 2H, *J*=9), 8.06 (d, 2H, *J*=7); MS [M/M+2]⁺: 396/398 |
| 126 | 3-(3-Chloro-4-methylsulfanylphenyl)-2-(3-fluoro-4-methoxyphenyl)imidazo[1,2-*a*]pyrimidine; ¹H-NMR: 2.50 (s, 3H), 3.83 (s, 3H), 6.85 (dd, 1H, *J*=7, *J*=4), 6.89 . (d, 1H, *J*=8.5), 7.25 (d, 2H, *J*=1), 7.34-7.39 (m, 3H), 8.19 (dd, 1H, *J*=7, *J*=2), 8.50 (dd, 1H, *J*=4, *J*=2); MS [M/M+2]⁺: 400/402 |
| 127 | 3-(3-Chloro-4-methylsulfanylphenyl)-2-(3-fluoro-4-methoxyphenyl)-7-methylimidazo[1,2-*a*]pyrimidine; ¹H-NMR: 2.56 (s, 3H), 2.64 (s, 3H), 3.89 (s, 3H), 6.70 (d, 1H, *J*=7), 6.91 (d, 1H, *J*=8.5), 7.30 (d, 2H, *J*=1), 7.42-7.43 (m, 1H), 7.46-7.49 (m, 1H), 7.54 (d, 1H, *J*=2), 8.03 (d, 1H, *J*=7); MS [M/M+2]⁺: 414/416 |
| 128 | 2-(3-Chloro-4-methoxyphenyl)-3-(3-chloro-4-methylsulfanylphenyl)imidazo[1,2-*a*]pyrimidine; ¹H-NMR: 2.57 (s, 3H), 3.91 (s, 3H), 6.85 (dd, 1H, *J*=7, *J*=4), 6.86 (d, 1H, *J*=8.5), 7.32 (d, 2H, *J*=1), 7.45-7.46 (m, 1H), 7.53 (dd, 1H, *J*=8.5, *J*=2), 7.91 (d, 1H, *J*=2), 8.21 (dd, 1H, *J*=7, *J*=2), 8.57 (dd, 2H, *J*=4, *J*=2); MS [M/M+2/M+4]⁺ 416/418/420 |
| 129 | 2-(3-Chloro-4-methoxyphenyl)-3-(3-chloro-4-methylsulfanylphenyl)-7-methylimidazo[1,2-*a*]pyrimidine; ¹H-NMR: 2.56 (s, 3H), 2.65 (s, 3H), 3.90 (s, 3H), 6.71 (d, 1H, *J*=7), 6.85 (d, 1H, *J*=8.5), 7.29-7.30 (m 2H), 7.42-7.43 (m, 1H), 7.54 (dd, 1H, *J*=8.5, *J*=2), 7.89 (d, 1H, *J*=2), 8.05 (d, 1H, *J*=7); MS [M/M+2/M+4]⁺: 430/432/434 |
| 130 | 3-(3-Chloro-4-methylsulfanylphenyl)-2-(2-fluoro-4-methoxyphenyl)imidazo[1,2-*a*]pyrimidine; ¹H-NMR: 2.52 (s, 3H), 3.82 (s, 3H), 6.55 (dd, 1H, *J*=12, *J*=2.5), 6.79 (dd, 1H, *J*=8.5, *J*=2.5), 6.89 (dd, 1H, *J*=7, *J*=4), 7.22 (s, 2H), 7.37 (s, 1H), 7.72 (t, 1H, *J*=8.5), 8.41 (dd, 1H, *J*=7, *J*=2), 8.59 (dd, 2H, *J*=4, *J*=2); MS [M/M+2]⁺: 400/402 |
| 131 | 3-(3-Chloro-4-methylsulfanylphenyl)-2-(2-fluoro-4-methoxyphenyl)-7-methylimidazo[1,2-*a*]pyrimidine; ¹H-NMR: 2.52 (s, 3H), 2.66 (s, 3H), 3.82 (s, 3H), 6.55 (dd, 1H, *J*=12, *J*=2.5), 6.74 (d, 1H, *J*=7), 6.78 (dd, 1H, *J*=8.5, *J*=2.5), 7.21 (s, 2H), 7.36 (s, 1H), 7.74 (t, 1H, *J*=8.5), 8.25 (d, 1H, *J*=7, *J*=2); MS [M/M+2]⁺: 414/416 |
| 132 | 3-(3-Bromo-4-methylsulfanylphenyl)-2-m-tolylimidazo[1,2-*a*]pyrimidine; ¹H-NMR: 2.35 (s, 3H), 2.56 (s, 3H), 6.85 (dd, 1H, *J*=7, *J*=4), 7.12 (d, 1H, *J*=8), 7.17 (t, 1H, *J*=7.5), 7.25 (d, 1H, *J*=8), 7.35-7.39 (m, 2H), 7.64 (d, 1H, *J*=2), 7.79 (s, 1H), 8.21 (dd, 1H, *J*=6.5, *J*=2), 8.55 (dd, 1H, *J*=4, *J*=2); MS [M/M+2]⁺ 410/412 |
| 133 | 3-(3-Bromo-4-methylsulfanylphenyl)-7-methyl-2-m-tolylimidazo[1,2-*a*]pyrimidine; ¹H-NMR: 2.34 (s, 3H), 2.55 (s, 3H), 2.64 (s, 3H), 6.70 (d, 1H, *J*=7), 7.09 (d, 1H, *J*=8), 7.15 (t, 1H, *J*=8), 7.24 (d, 1H, *J*=8.5), 7.35 (dd, 1H, *J*=8.5, *J*=2), 7.38 (d, 1H, *J*=8), 7.62 (d, 1H, *J*=2), 7.78 (s, 1H), 8.07 (d, 1H, *J*=7); MS [M/M+2]⁺: 424/426 |
| 134 | 3-(3-Bromo-4-methylsulfanylphenyl)-2-(4-methoxyphenyl)imidazo[1,2-*a*]pyrimidine; ¹H-NMR: 2.56 (s, 3H), 3.82 (s, 3H), 6.83 (dd, 1H, *J*=6.5, *J*=4), 6.87 (d, 2H, *J*=9), 7.26 (d, 1H, *J*=8), 7.37 (dd, 1H, *J*=8, *J*=2), 7.64 (d, 1H, *J*=2), 7.71 (d, 2H, *J*=9), 8.21 (dd, 1H, *J*=6.5, *J*=2), 8.55 (dd, 1H, *J*=4, *J*=2); MS [M/M+2]⁺: 3426/428 |
| 135 | 3-(3-Bromo-4-methylsulfanylphenyl)-2-(4-methoxyphenyl)-7-methylimidazo[1,2-*a*]pyrimidine; ¹H-NMR: 2.56 (s, 3H), 2.64 (s, 3H), 3.82 (s, 3H), 6.69 (d, 1H, *J*=7), 6.86 (d, 2H, *J*=8.5), 7.26 (d, 1H, *J*=8), 7.36 (dd, 1H, *J*=8, *J*=1.5), 7.62 (d, 1H, *J*=1.5), 7.70 (d, 2H, *J*=8.5), 8.06 (d, 1H, *J*=7); MS [M/M+2]⁺: 440/442 |
| 136 | 3-(3-Bromo-4-methylsulfanylphenyl)-2-(4-chlorophenyl)imidazo[1,2-*a*]pyrimidine; ¹H-NMR: 2.56 (s, 3H), 6.87 (dd, 1H, *J*=7, *J*=4), 7.26 (d, 1H, *J*=8), 7.30 (d, 2H, *J*=9), 7.34 (dd, 1H, *J*=8, *J*=2), 7.63 (d, 1H, *J*=2), 7.70 (d, 2H, *J*=9), 8.23 (dd, 1H, *J*=7, *J*=2), 8.59 (dd, 1H, *J*=4, *J*=2); MS [M/M+2/M+4]⁺: 430/432/434 |
| 137 | 3-(3-Bromo-4-methylsulfanylphenyl)-2-(4-chlorophenyl)-7-methylimidazo[1,2-*a*]pyrimidine; ¹H-NMR: 2.56 (s, 3H), 2.65 (s, 3H), 6.72 (d, 1H, *J*=7), 7.25 (d, 1H, *J*=8), 7.28 (d, 2H, *J*=8.5), 7.33 (dd, 1H, *J*=8, *J*=2), 7.61 (d, 1H, *J*=2), 7.69 (d, 2H, *J*=8.5), 8.06 (d, 1H, *J*=7); MS [M/M+2/M+4]⁺: 444/446/448 |
| 138 | 2-(4-Methoxyphenyl)-3-(3-methyl-4-methylsulfanylphenyl)imidazo[1,2-*a*]pyrimidine; ¹H-NMR: 2.37 (s, 3H), 2.56 (s, 3H), 3.81 (s, 3H), 6.82 (dd, 1H, *J*=7, *J*=4), 6.85 (d, 2H, *J*=9), 7.21 (s, 1H), 7.25 (d, 1H, *J*=8), 7.28 (d, 1H, *J*=8), 7.74 (d, 2H, *J*=9), 8.21(dd, 1H, *J*=7, *J*=2), 8.54 (dd, 1H, *J*=4, *J*=2); MS [M+1]⁺: 362 |
| 139 | 2-(4-Methoxyphenyl)-7-methyl-3-(3-methyl-4-methylsulfanylphenyl)imidazo[1,2-*a*] pyrimidine; ¹H-NMR: 2.36 (s, 3H), 2.55 (s, 3H), 2.63 (s, 3H), 3.80 (s, 3H), 6.64 (d, 1H, *J*=7), 6.83 (d, 2H, *J*=9), 7.19 (s, 1H), 7.25-7.35 (m, 2H) 7.73 (d, 2H, *J*=9), 8.04 (d, 1H, *J*=7); MS [M+1]⁺: 376 |
| 140 | 3-(3-*tert*-Butyl-4-methylsulfanylphenyl)-2-(4-methoxyphenyl)imidazo[1,2-*a*]pyrimidine; ¹H-NMR: 1.47 (s, 9H), 2.59 (s, 3H), 3.82 (s, 3H), 6.80 (dd, 1H, *J*=7, *J*=4), 6.85 (d, 2H, *J*=9), 7.27 (dd, 1H, *J*=7, *J*=2), 7.42 (d, 1H, *J*=2), 7.43 (d, 1H, *J*=7), 7.73 (d, 2H, *J*=9), 8.27 (dd, 1H, *J*=7, *J*=2), 8.53 (dd, 1H, *J*=4, *J*=2); MS [M+1]⁺: 404 |
| 141 | 3-(3-Chloro-5-methyl-4-methylsulfanylphenyl)-2-(4-methoxyphenyl)imidazo[1,2-*a*]pyrimidine; ¹H-NMR: 2.35 (s, 3H), 2.50 (s, 3H), 3.92 (s, 3H), 6.82 (dd, 1H, *J*=7, *J*=4), 7.10 (d, 2H, *J*=8.5), 7.36 (d, 2H, *J*=8.5), 7.66 (s, 1H), 7.67 (s, 1H), 8.18 (d, 1H, *J*=7, *J*=2), 8.56 (dd, 1H, *J*=4, *J*=2); MS [M/M+2]⁺: 396/398 |
| 142 | 3-(3-Chloro-5-methyl-4-methylsulfanylphenyl)-2-(4-methoxyphenyl)-7-methylimidazo[1,2-*a*]pyrimidine; ¹H-NMR: 2.33 (s, 3H), 2.49 (s, 3H), 2.64 (s, 3H), 3.92 (s, 3H), 6.67 (d, 1H, *J*=7), 7.09 (d, 2H, *J*=8.5), 7.34 (d, 2H, *J*=8.5), 7.66 (s, 1H), 7.67 (s, 1H), 8.02 (d, 1H, *J*=7); MS [M/M+2]⁺: 410/412 |
| 143 | 3-(2,3-Dichloro-4-methylsulfanylphenyl)-2-(4-methoxyphenyl)imidazo[1,2-*a*]pyrimidine; ¹H-NMR: 2.56 (s, 3H), 3.81 (s, 3H), 6.84 (dd, 1H, *J*=7, *J*=4), 6.86 (d, 2H, *J*=9), 7.14 (d, 1H, *J*=8.5), 7.27 (d, 1H, *J*=8.5), 7.65 (d, 2H, *J*=9), 7.89 (dd, 1H, *J*=7, *J*=2), 8.59 (dd, 1H, *J*=4, *J*=2); MS [M/M+2/M+4]⁺: 416/418/420 |
| 144 | 3-(3-Chloro-4-ethylsulfanylphenyl)-2-(4-methoxyphenyl)imidazo[1,2-*a*]pyrimidine; ¹H-NMR: 1.45 (t, 3H, *J*=7.5), 3.06 (q, 2H, *J*=7.5), 3.82 (s, 3H), 6.83 (dd, 1H, *J*=7, *J*=4), 6.86 (d, 2H, *J*=9), 7.28 (dd, 1H, *J*=8, *J*=2), 7.35 (d, 1H, *J*=8), 7.46 (d, 1H, *J*=2), 7.70 (d, 2H, *J*=9), 8.22 (d, *J*=7, *J*=2), 8.54 (dd, 1H, *J*=4, *J*=2); MS [M/M+2]⁺: 396/398 |
| 145 | 3-(3-Chloro-4-ethylsulfanylphenyl)-2-(4-methoxyphenyl)-7-methylimidazo[1,2-*a*]pyrimidine; ¹H-NMR: 1.45 (t, 3H, *J*=7), 2.64 (s, 3H), 3.05 (q, 2H, *J*=7), 3.81 (s, 3H), 6.68 (d, 1H, *J*=7), 6.85 (d, 2H, *J*=9), 7.26 (dd, 1H, *J*=8, *J*=1.5), 7.34 (d, 1H, *J*=8), 7.45 (d, 1H, *J*=1.5), 7.69 (d, 2H, *J*=9), 8.06 (d, *J*=7); MS [M/M+2]⁺: 410/412 |
| 146 | 3-(3-Chloro-4-propylsulfanylphenyl)-2-p-tolylimidazo[1,2-*a*]pyrimidine; ¹H-NMR: 1.13 (t, 3H, *J*=7), 1.77-1.88 (m, 2H), 2.35 (s, 3H), 3.00 (t, 2H, *J*=7), 6.83 (dd, 1H, *J*=7, *J*=4), 7.13 (d, 2H, *J*=8), 7.27 (dd, 1H, *J*=8, *J*=1.5), 7.35 (d, 1H, *J*=8), 7.46 (d, 1H, *J*=1.5), 7.64 (d, 2H, *J*=8), 8.22 (dd, *J*=7, *J*=2), 8.55 (dd, 1H, *J*=4, *J*=2); MS [M/M+2]⁺: 394/396 |
| 147 | 3-(3-Chloro-4-propylsulfanylphenyl)-7-methyl-2-p-tolylimidazo[1,2-*a*]pyrimidine; ¹H-NMR: 1.13 (t, 3H, *J*=7), 1.77-1.88 (m, 2H), 2.34 (s, 3H), 2.64 (s, 3H), 3.00 (t, 2H, *J*=7), 6.69 (d, 1H, *J*=7), 7.12 (d, 2H, *J*=8), 7.26 (dd, 1H, *J*=8, *J*=2), 7.33 (d, 1H, *J*=8), 7.44 (d, 1H, *J*=2), 7.64 (d, 2H, *J*=8), 8.07 (d, *J*=7); MS [M/M+2]⁺: 408/410 |
| 148 | 3-(3-Chloro-4-propylsulfanylphenyl)-2-(4-methoxyphenyl)imidazo[1,2-*a*]pyrimidine; ¹H-NMR: 1.13 (t, 3H, *J*=7.5), 1.76-1.86 (m, 2H), 3.00 (t, 2H, *J*=7.5), 3.82 (s, 3H), 6.83 (dd, 1H, *J*=7, *J*=4), 6.85 (d, 2H, *J*=9), 7.27 (dd, 1H, *J*=8, *J*=2), 7.35 (d, 1H, *J*=8), 7.46 (d, 1H, *J*=2), 7.70 (d, 2H, *J*=9), 8.22 (d, *J*=7, *J*=2), 8.54 (dd, 1H, *J*=4, *J*=2); MS [M/M+2]⁺: 410/412 |
| 149 | 3-(3-Chloro-4-propylsulfanyphenyl)-2-(4-methylsulfanylphenyl)imidazo[1,2-*a*]pyrimidine; ¹H-NMR: 1.13 (t, 3H, *J*=7), 1.76-1.88 (m, 2H), 2.49 (s, 3H), 3.00 (t, 2H, *J*=7), 6.84 (dd, 1H, *J*=7, *J*=4), 7.19 (d, 2H, *J*=8.5), 7.26 (dd, 1H, *J*=8.5, *J*=2), 7.35 (d, 1H, *J*=8.5), 7.46 (d, 1H, *J*=2), 7.68 (d, 2H, *J*=8.5), 8.22 (dd, 1H, *J*=7, *J*=2), 8.56 (dd, 1H, *J*=4, *J*=2); MS [M/M+2]⁺: 426/428 |
| 150 | 3-(3-Chloro-4-propylsulfanylphenyl)-7-methyl-2-(4-methylsulfanylphenyl)imidazo[1,2-*a*]pyrimidine; ¹H-NMR: 1.13 (t, 3H, *J*=7), 1.76-1.87 (m, 2H), 2.48 (s, 3H), 2.64 (s, 3H), 3.00 (t, 2H, *J*=7), 6.70 (d, 1H, J=7), 7.18 (d, 2H, *J*=8.5), 7.25 (dd, 1H, *J*=8, *J*=2), 7.34 (d, 1H, *J*=8), 7.44 (d, 1H, *J*=2), 7.68 (d, 2H, *J*=8.5), 8.06 (d, 1H, *J*=7); MS [M/M+2]⁺: 440/442 |
| 151 | 3-(4-Isopropylsulfanylphenyl)-2-(4-methoxyphenyl)imidazo[1,2-*a*]pyrimidine; ¹H-NMR: 1.37 (d, 6H, *J*=7), 3.51 (m, 1H, *J*=7), 3.78 (s, 3H), 6.77 (dd, 1H, *J=*7, *J*=4), 6.81 (d, 2H, *J*=9), 7.33 (d, 2H, *J*=8), 7.47 (d, 2H, *J*=8), 7.67 (d, 2H, *J=*9), 8.22 (dd, 1H, *J*=7, *J*=2), 8.48 (dd, 1H, *J*=4, *J*=2); MS [M+1]⁺: 376 |
| 152 | 3-(4-Isopropylsulfanylphenyl)-2-(4-methoxyphenyl)-7-methylimidazo[1,2-*a*]pyrimidine; ¹H-NMR: 1.38 (d, 6H, *J*=6.5), 2.62 (s, 3H), 3.41-3.73 (m, 1H), 3.79 (s, 3H), 6.65 (d, 1H, *J*=7), 6.82 (d, 2H, *J*=9), 7.33 (d, 2H, *J*=8.5), 7.48 (d, 2H, *J*=8.5), 7.68 (d, 2H, *J*=9), 8.07 (d, 1H, *J*=7); MS [M+1]⁺: 390 |
| 153 | 3-(3-Chloro-4-isopropylsulfanylphenyl)-2-*p*-tolylimidazo[1,2-*a*]pyrimidine; ¹H-NMR: 1.45 (d, 6H, *J*=6.5), 2.35 (s, 3H) 3.55-3.66 (m, 1H), 6.84 (dd, 1H, *J*=7, *J*=4), 7.14 (d, 2H, *J*=8), 7.28 (dd, 1H, *J*=8, *J*=2), 7.45 (d, 1H, *J*=8), 7.49 (d, 1H, *J*=2), 7.64 (d, 2H, *J*=8), 8.25 (dd, 1H, *J*=7, *J*=2), 8.57 (dd, 1H, *J*=4, *J*=2); MS [M/M+2]⁺: 394/396 |
| 154 | 3-(3-Chloro-4-isopropylsulfanylphenyl)-7-methyl-2-*p*-tolylimidazo[1,2-*a*]pyrimidine; ¹H-NMR: 1.44 (d, 6H, *J*=6.5), 2.34 (s, 3H), 2.64 (s, 3H), 3.39-3.79 (m, 1H), 6.70 (d, 1H, *J*=7), 7.12 (d, 2H, *J*=8), 7.26 (dd, 1H, *J*=8, *J*=2), 7.44 (d, 1H, *J*=8), 7.47 (d, 1H, *J*=2), 7.64 (d, 2H, *J*=8), 8.09 (d, *J*=7); MS [M/M+2]⁺: 408/410 |
| 155 | 3-(3-Chloro-4-isopropylsulfanylphenyl)-2-(4-methoxyphenyl)imidazo[1,2-*a*]pyrimidine; ¹H-NMR: 1.45 (d, 6H, *J*=7), 3.56-3.65 (m, 1H), 3.82 (s, 3H), 6.84 (dd, 1H, *J*=7, *J*=4), 6.86 (d, 2H, *J*=9), 7.28 (dd, 1H, *J*=8, *J*=2), 7.56 (d, 1H, *J*=8), 7.49 (d, 1H, *J*=2), 7.69 (d, 2H, *J*=9), 8.24 (d, *J*=7, *J*=2), 8.55 (dd, 1H, *J*=4, *J*=2); MS [M/M+2]⁺: 410/412 |
| 156 | 3-(3-Chloro-4-isopropylsulfanylphenyl)-2-(4-methoxyphenyl)-7-methylimidazo[1,2-*a*]pyrimidine; ¹H-NMR: 1.44 (d, 6H, *J*=6.5), 2.64 (s, 3H), 3.55-3.64 (m, 1H), 3.81 (s, 3H), 6.69 (d, 1H, *J*=7), 6.85 (d, 2H, *J*=9), 7.27 (dd, 1H, *J*=8, *J*=2), 7.44 (d, 1H, *J*=8), 7.47 (d, 1H, *J*=2), 7.69 (d, 2H, *J*=9), 8.09 (d, *J*=7); MS [M/M+2]⁺: 424/426 |
| 157 | 3-(3-Chloro-4-isopropylsulfanylphenyl)-2-(4-methylsulfanylphenyl)imidazo[1,2-*a*]pyrimidine; ¹H-NMR: 1.45 (d, 6H, *J*=6.5), 2.49 (s, 3H), 3.61 (m, 1H, *J*=6.5), 6.85 (dd, 1H, *J*=7, *J*=4), 7.20 (d, 2H, *J*=9), 7.28 (dd, 1H, *J*=8, *J*=2), 7.46 (d, 1H, *J*=8), 7.49 (d, 1H, *J*=2), 7.68 (d, 2H, *J*=9), 8.25 (dd, *J*=7, *J*=2), 8.58 (d, 1H, *J*=4, *J*=2); MS [M/M+2]⁺: 426/428 |
| 158 | 3-(3-Chloro-4-isopropylsulfanylphenyl)-7-methyl-2-(4-methylsulfanylphenyl)imidazo[1,2-*a*]pyrimidine; ¹H-NMR: 1.45 (d, 6H, *J*=6.5), 2.49 (s, 3H), 2.65 (s, 3H), 3.60 (m, 1H, *J*=6.5), 6.71 (d, 1H, *J*=7), 7.19 (d, 2H, *J*=8.5), 7.26 (dd, 1H, *J*=8, *J*=2), 7.45 (d, 1H, *J*=8), 7.47 (d, 1H, *J*=2), 7.68 (d, 2H, *J*=8.5), 8.08 (d, 1H, *J*=7); MS [M/M+2]⁺. 440/442 |
| 159 | 3-(4-Cyclopropylsulfanylphenyl)-2-(4-methoxyphenyl)imidazo[1,2-*a*]pyrimidine; ¹H-NMR: 0.76 (dt, 2H, *J*=6.5, *J*=5), 1.15 (dt, 2H, *J*=6.5, *J*=5), 2.18-2.26 (m, 1H), 3.80 (s, 3H), 6.78 (dd, 1H, *J*=7, *J*=4), 6.84 (d, 2H, *J*=8.5), 7.34 (d, 2H, *J*=8.5), 7.51 (d, 2H, *J*=8.5), 7.71 (d, 2H, *J*=8.5), 8.22 (dd, 1H, *J*=7, *J*=2), 8.50 (dd, 1H, *J*=4, *J*=2); MS [M+1]⁺: 374 |
| 160 | 3-(4-Cyclopropylsulfanylphenyl)-2-(4-methoxyphenyl)-7-methylimidazo[1,2-*a*]pyrimidine; ¹H-NMR: 0.76 (dt, 2H, *J*=6.5, *J*=5), 1.15 (dt, 2H, *J*=6.5, *J*=5), 2.18-2.26 (m, 1H), 2.62 (s, 3H), 3.80 (s, 3H), 6.65 (d, 1H, *J*=7), 6.83 (d, 2H, *J*=9), 7.33 (d, 2H, *J*=8.5), 7.49 (d, 2H, *J*=8.5), 7.71 (d, 2H, *J*=9), 8.06 (d, 1H, *J*=7); MS [M+1]⁺: 388 |
| 161 | 3-(4-Cyclohexylmethylsulfanylphenyl)-2-(4-methoxyphenyl)imidazo[1,2-*a*]pyrimidine; ¹H-NMR: 0.90-1.35 (m, 5H), 1.55-1.80 (m, 4H), 1.88-2.00 (m, 2H), 2.90 (d, 2H, *J*=7), 3.81 (s, 3H), 6.78 (dd, 1H, *J*=7, *J*=4), 6.84 (d, 2H, *J*=9), 7.33 (d, 2H, *J*=8.5), 7.42 (d, 2H, *J*=8.5), 7.71 (d, 2H, *J*=9), 8.22 (dd, 1H, *J*=7, *J*=2), 8.52 (dd, 1H, *J*=4, *J*=2); MS. [M+1]⁺: 430 |
| 162 | 3-(4-Cyclohexylmethylsulfanylphenyl)-2-(4-methoxyphenyl)-7-methylimidazo[1,2-*a*]pyrimidine; ¹H-NMR: 0.90-1.37 (m, 5H), 1.50-1.82 (m, 4H), 1.85-2.05 (m, 2H), 2.62 (s, 3H), 2.90 (d, 2H, *J*=6.5), 3.80 (s, 3H), 6.65 (d, 1H, *J*=7), 6.83 (d, 2H, *J*=8.5), 7.31 (d, 2H, *J*=8), 7.41 (d, 2H, *J*=8.5), 7.70 (d, 2H, *J*=8.5), 8.06 (dd, 1H, *J*=7); MS [M+1]⁺: 444 |
| 163 | 3-(4-Cyclohexylmethylsulfanylphenyl)-2-(4-methylsulfanylphenyl)imidazo[1,2-*a*]pyrimidine; ¹H-NMR: 0.90-1.35 (m, 5H), 1.55-1.80 (m, 4H), 1.88-2.00 (m, 2H), 2.48 (s, 3H), 2.90 (d, 2H, *J*=7), 6.81 (dd, 1H, *J*=7, *J*=4), 7.17 (d, 2H, *J*=8.5), 7.33 (d, 2H, *J*=8.5), 7.42 (d, 2H, *J*=8.5), 7.69 (d, 2H, *J*=8.5), 8.23 (dd, 1H, *J*=7, *J*=2), 8.54 (dd, 1H, *J*=4, *J*=2); MS [M+1]⁺: 446 |
| 164 | 3-(4-Cyclohexylmethylsulfanylphenyl)-7-methyl-2-(4-methylsulfanylphenyl)imidazo[1,2-*a*]pyrimidine; ¹H-NMR: 0.90-1.40 (m, 5H), 1.45-2.05 (m, 6H), 2.47 (s, 3H), 2.63 (s, 3H), 2.90 (d, 2H, *J*=6.5), 6.67 (d, 1H, *J*=7), 7.16 (d, 2H, *J*=8), 7.31 (d, 2H, *J*=8), 7.41 (d, 2H, *J*=8.5), 7.68 (d, 2H, *J*=8.5), 8.07 (dd, 1H, *J*=7); MS [M+1]⁺: 460 |
| 165 | 2-[3-Chloro-4-(propane-1-sulfinyl)phenyl]-3-*p*-tolylimidazo[1,2-*a*]pyrimidine; ¹H-NMR: 1.07 (t, 3H, *J*=7.5), 1.61-1.98 (m, 2H), 2.49 (s, 3H), 2.72-2.86 (m, 1H), 3.03 (ddd, 1H, *J*=7, *J*=9.5, *J*=13), 6.85 (dd, 1H, *J*=7, *J*=4), 7.32 (d, 1H, *J*=8), 7.39 (d, 2H, *J*=8), 7.69 (dd, 1H, *J*=8.5, *J*=1.5), 7.74 (d, 2H, *J*=8.5), 8.03 (d, 1H, *J*=2), 8.22 (dd, 1H, *J*=7, *J*=2), 8.59 (dd, 1H, *J*=4, *J*=2); MS [M/M+2]⁺: 410/412 |
| 166 | 2-(4-Methanesulfinylphenyl)-7-methyl-3-phenylimidazo[1,2-*a*]pyrimidine; ¹H-NMR: 2.64 (s, 3H), 2,71 (s, 3H), 6.70 (d, 1H, *J*=7), 7.40-7.43 (m, 2H,), 7.52-7.56 (m, 5H), 7.90 (d, 2H, *J*=8.5), 8.08 (d, 1H, *J*=7); MS [M+1]⁺: 348 |
| 167 | 3-(4-Chlorophenyl)-2-(4-methanesulfinylphenyl)imidazo[1,2-*a*]pyrimidine; ¹H-NMR: 2.73 (s, 3H), 6.88 (dd, 1H, *J*=7, *J*=4), 7.40 (d, 2H, *J*=8.5), 7.56 (d, 2H, *J*=8.5), 7.59 (d, 2H, *J*=8.5), 7.89 (d, 2H, *J*=8.5), 8.23 (dd, 1H, *J*=7, *J*=2), 8.61 (dd, 1H, *J*=4, *J*=2); MS [M/M+2]⁺: 368/370 |
| 168 | 3-(4-Bromophenyl)-2-(4-methanesulfinylphenyl)-7-methylimidazo[1,2-*a*]pyrimidine; ¹H-NMR: 2.66 (s, 3H), 2.73 (s, 3H), 6.74 (d, 1H, *J*=7), 7.31 (d, 2H, *J*=8.5), 7.58 (d, 2H, *J*=8.5), 7.70 (d, 2H, *J*=8.5), 7.88 (d, 2H, *J*=8.5), 8.07 (d, 1H, *J*=7); MS [M/M+2]⁺: 426/428 |
| 169 | 3-(3-Fluoro-4-methylsulfanylphenyl)-2-(4-methanesulfinylphenyl)imidazo[1,2-*a*]pyrimidine; ¹H-NMR: 2.57 (s, 3H), 2.74 (s, 3H), 6.89 (dd, 1H, *J*=7, *J*=4), 7.13 (dd, 1H, *J*=10, *J*=2), 7.20 (dd, 1H, *J*=8, *J*=2), 7.39 (t, 1H, *J*=8), 7.60 (d, 2H, *J*=9), 7.91 (d, 2H, *J*=9), 8.27 (dd, 1H, *J*=7, *J*=2), 8.61 (dd, 1H, *J*=4, *J*=2); MS [M+1]⁺: 398 |
| 170 | 3-(3-Fluoro-4-methylsulfanylphenyl)-2-(4-methanesulfinylphenyl)-7-methylimidazo[1,2-*a*]pyrimidine; ¹H-NMR: 2.56 (s, 3H), 2.66 (s, 3H), 2.74 (s, 3H), 6.74 (d, 1H, *J*=7), 7.11 (dd, 1H, *J*=10, *J*=2), 7.18 (dd, 1H, *J*=8, *J*=2), 7.38 (t, 1H, *J*=8), 7.58 (d, 2H, *J*=8.5), 7.90 (d, 2H, *J*=8.5), 8.11 (d, 2H, *J*=7); MS [M+1]⁺: 412 |
| 171 | 3-(3-Chloro-4-methylsulfanylphenyl)-2-(4-methanesulfinylphenyl)imidazo[1,2-*a*]pyrimidine; ¹H-NMR: 2.56 (s, 3H), 2.73 (s, 3H), 6.89 (d, 2H, *J*=1), 7.45 (s, 1H), 7.59 (d, 2H, *J*=8.5), 7.90 (d, 2H, *J*=8.5), 8.25 (dd, 1H, *J*=7, *J*=2), 8.60 (dd, 2H, *J*=4, *J*=2); MS [M/M+2]⁺: 414/416 |
| 172 | 3-(3-Chloro-4-methylsulfanylphenyl)-2-(4-methanesulfinylphenyl)-7-methylimidazo[1,2-*a*]pyrimidine; ¹H-NMR: 2.55 (s, 3H), 2.64 (s, 3H), 2.71 (s, 3H), 6.73 (d, 1H, *J*=7), 7.27 (d, 1H, *J*=8.5), 7.30 (dd, 1H, *J*=8.5, *J*=1.5), 7.42 (d, 1H, *J*=1.5), 7.56 (d, 2H, *J*=8.5), 7.88 (d, 2H, *J*=8.5), 8.08 (d, 2H, *J*=7); MS [M/M+2]⁺: 428/430 |
| 173 | 3-(3-Chloro-4-methanesulfinylphenyl)-2-(4-methanesulfinylphenyl)-7-methylimidazo[1,2-*a*]pyrimidine; ¹H-NMR: 2.67 (s, 3H), 2.74 (s, 3H), 2.94 (s, 3H), 6.79 (d, 1H, *J*=7), 7.47 (dd, 1H, *J*=4, *J*=1.5), 7.60 (d, 2H, *J*=8.5), 7.63 (m, 1H), 7.85 (d, 2H, *J*=8.5), 8.13 (dd, 1H, *J*=8, *J*=1.5), 8.15 (d, 2H, *J*=7); MS [M/M+2]⁺: 444/446 |
| 174 | 3-(3-Fluoro-4-methoxyphenyl)-2-(4-methanesulfinylphenyl)-7-methylimidazo[1,2-*a*]pyrimidine; ¹H-NMR: 2.64 (s, 3H), 2.73 (s, 3H), 3.99 (s, 3H), 6.73 (d, 1H, *J*=7), 7.12-7.19 (m, 3H), 7.57 (d. 2H, *J*=8.5), 7.90 (d, 2H, *J*=8.5), 8.06 (d, 1H, *J*=7); MS [M+1]⁺: 396 |
| 175 | 2-(3-Chloro-4-methanesulfinylphenyl)-3-(4-methoxyphenyl)imidazo[1,2-*a*]pyrimidine; ¹H-NMR: 2.81 (s, 3H), 3.92 (s, 3H), 6.85 (dd, 1H, *J*=7, *J*=4), 7.11 (d, 1H, *J*=8.5), 7.36 (d, 2H, *J*=8.5), 7.73 (dd, 1H, *J*=8.5, *J*=2), 7.79 (d, 1H, *J*=8.5), 8.03 (d, 1H, *J*=2), 8.20 (dd, 1H, *J*=7, *J*=2), 8.59 (dd, 1H, *J*=4, *J*=2); MS [M/M+2]⁺: 398/400 |
| 176 | 3-[3-Chloro-4-(propane-1-sulfinyl)phenyl]-2-(4-methoxyphenyl)imidazo[1,2-*a*]pyrimidine; ¹H-NMR: 1.13 (t, 3H, *J*=7.5), 1.71-1.87 (m, 1H), 1.92-2.07 (m, 1H), 2.82-2.94 (m, 1H), 3.14 (ddd, 1H, *J*=7, *J*=9.5, *J*=13), 3.82 (s, 3H), 6.86 (d, 2H, *J*=9), 6.87 (dd, 1H, *J*=7, *J*=4), 7.49 (d, 1H, *J*=1.5), 7.61 (dd, 1H, *J*=8, *J*=1.5), 7.63 (d, 2H, *J*=8), 8.06 (d, 1H, *J*=8), 8.28 (d, 1H, *J*=7, *J*=2), 8.58 (dd, 1H, *J*=4, *J*=2); MS [M/M+2]⁺: 426/428 |
| 177 | 3-[3-Chloro-4-(propane-1-sulfinyl)phenyl]-2-(4-methoxyphenyl)-7-methylimidazo[1,2-*a*]pyrimidine; ¹H-NMR: 1.13 (t, 3H, *J*=7), 1.72-1.85 (m, 1H), 1.91-2.07 (m, 1H), 2.65 (s, 3H), 2.83-2.93 (m, 1H), 3.13 (ddd, 1H, *J*=7, *J*=9, *J*=13), 3.81 (s, 3H), 6.73 (d, 1H, *J*=7), 6.85 (d, 2H, *J*=9), 7.47 (d, 1H, *J*=1.5), 7.59 (dd, 1H, *J*=8, *J*=1.5), 7.62 (d, 2H, *J*=9), 8.05 (d, 1H, *J*=8), 8.13 (d, 1H, *J*=7); MS [M/M+2]⁺: 440/442 |
| 178 | 3-(4-Methanesulfinylphenyl)-7-methyl-2-phenylimidazo[1,2-*a*]pyrimidine; ¹H-NMR: 2.66 (s, 3H), 2.85 (s, 3H), 6.73 (d, 1H, *J*=7), 7.28-7.32 (m, 3H), 7.62 (d, 2H, *J*=8.5), 7.69-7.72 (m, 2H), 7.82 (d, 2H, *J*=8.5), 8.12 (d, 1H, *J*=7); MS [M+1]⁺: 348 |
| 179 | 3-(4-Methanesulfinylphenyl)-2-(4-methoxyphenyl)-7-methylimidazo[1,2-*a*]pyrimidine; ¹H-NMR: 2.65 (s, 3H), 2.84 (s, 3H), 3.81 (s, 3H), 6.71 (d, 1H, *J*=7), 6.84 (d, 2H, *J*=9), 7.62 (d, 2H, *J*=8.5), 7.64 (d, 2H, *J*=9), 7.81 (d, 2H, *J*=8.5), 8.12 (d, 1H, *J*=7); MS [M+1]⁺: 378 |
| 180 | 3-(4-Methanesulfinylphenyl)-2-(4-methoxy-3-methylphenyl)-7-methylimidazo[1,2-a]pyrimidine; ¹H-NMR: 2.16 (s, 3H), 2.64 (s, 3H), 2.83 (s, 3H), 3.81 (s, 3H), 6.70 (d, 1H, *J*=7), 6.72 (d, 1H, *J*=8.5), 7.40 (dd, 1H, *J*=8.5, *J*=2), 7.61(d, 1H, *J*=2), 7.62 (d, 2H, *J*=8.5), 7.81 (d, 2H, *J*=8.5), 8.12 (d, 1H, *J*=7); MS [M+1]⁺: 392 |
| 181 | 3-(4-Methanesulfinylphenyl)-2-(6-methoxybiphenyl-3-yl)-7-methylimidazo[1,2-*a*]pyrimidine; ¹H-NMR: 2.57 (s, 3H), 2.77 (s, 3H), 3.79 (s, 3H), 6.65 (d, 1H, *J*=7), 6.87 (d, 1H, *J*=8.5), 7.20-7.44 (m, 5H), 7.58 (d, 2H, *J*=8.5), 7.61 (dd, 1H, *J*=8.5, *J*=2), 7.75 (d, 2H, *J*=8.5), 7.76 (d, 1H, *J*=2.5), 8.08 (d, 1H, *J*=7); MS [M+1]⁺: 354 |
| 182 | 2-(4-Ethoxy-3-fluorophenyl)-3-(4-methanesulfinylphenyl)imidazo[1,2-*a*]pyrimidine; ¹H-NMR: 1.45 (t, 3H, *J*=7), 2.85 (s, 3H), 4.12 (q, 2H, *J*=7), 6.85 (dd, 1H, *J*=7, *J*=4), 6.88 (t, 1H, *J*=9), 7.39-7.46 (m, 2H), 7.64 (d, 2H, *J*=8.5), 7.85 (d, 2H, *J*=8.5), 8.25 (dd, 1H, *J*=7, *J*=2), 8.59 (dd, 1H, *J*=4, *J*=2); MS [M+1]⁺: 396 |
| 183 | 2-(4-Ethoxy-3-fluorophenyl)-3-(4-methanesulfinylphenyl)-7-methylimidazo[1,2-*a*]pyrimidine; ¹H-NMR: 1.43 (t, 3H, *J*=7), 2.63 (s, 3H), 2.84 (s, 3H), 4.09 (q, 2H, *J*=7), 6.71 (d, 1H, *J*=7), 6.85 (t, 1H, *J*=9), 7.37-7.44 (m, 2H), 7.61 (d, 2H, *J*=8.5), 7.82 (d, 2H, *J*=8.5), 8.09 (d, 1H, *J*=7); MS [M+1]⁺: 410 |
| 184 | 2-(4-Fluorophenyl)-3-(4-methanesulfinylphenyl)-7-methylimidazo[1,2-*a*]pyrimidine; ¹H-NMR: 2.66 (s, 3H), 2.85 (s, 3H), 6.74 (d, 1H, *J*=7), 6,98 (d, 1H, *J*=8.5), 7.01 (d, 1H, *J*=8.5), 7.61 (d, 1H, *J*=8), 7.66 (d, 1H, *J*=8.5) 7.68 (d, 1H, *J*=8.5), 7.83 (d, 2H, *J*=8), 8.13 (d, 1H, *J*=7); MS [M+1]⁺: 366 |
| 185 | 2-(3-tert-Butyl-4-methoxyphenyl)-3-(4-methanesulfinylphenyl)-7-methylimidazo[1,2-*a*]pyrimidine; ¹H-NMR: 1.21 (s, 9H), 2.64 (s, 3H), 2.80 (s, 3H), 3.83 (s, 3H), 6.70 (d, 1H, *J*=7), 6.84 (d, 1H, *J*=8.5), 7.50 (d, 1H, *J*=2), 7.63 (d, 2H, *J*=8.5), 7.67 (dd, 1H, *J*=8.5, *J*=2), 7.82 (d, 2H, *J*=8.5), 8.10 (d, 1H, *J*=7); MS [M+1]⁺: 334 |
| 186 | 3-(4-Methanesulfinylphenyl)-2-(6-methoxybiphenyl-3-yl)imidazo[1,2-*a*]pyrimidine; ¹H-NMR: 2.80 (s, 3H), 3.81 (s, 3H), 6.85 (dd, 1H, *J*=7, *J*=4), 6.93 (d, 1H, *J*=8.5), 7.26-7.41 (m, 7H), 7.60 (d, 1H, *J*=2.5), 7.67 (d, 2H, *J*=8.5), 7.70 (dd, 1H, *J*=8.5, *J*=2.5), 8.28 (dd, 1H, *J*=7, *J*=2), 8.56 (dd, 1H, *J*=4, *J*=2); MS [M+1]⁺: 440 |
| 187 | 3-(3-Fluoro-4-methanesulfinylphenyl)-2-(4-methoxyphenyl)imidazo[1,2-*a*]pyrimidine; ¹H-NMR: 2.95 (s, 3H), 3.83 (s, 3H), 6.88 (d, 2H, *J*=9), 6.89 (dd, 1H, *J*=7, *J*=4), 7.23 (dd, 1H, *J*=10, *J*=1.5), 7.53 (dd, 1H, *J*=8, *J*=1.5), 7.64 (d, 2H, *J*=9), 8.04 (t, 1H, *J*=8), 8.32 (dd, 1H, *J*=7, *J*=2), 8.60 (dd, 2H, *J*=4, *J*=2) ; MS [M+1]⁺: 382 |
| 188 | 3-(3-Fluoro-4-methanesulfinylphenyl)-2-(4-methoxyphenyl)-7-methylimidazo[1,2-*a*]pyrimidine; ¹H-NMR: 2.66 (s, 3H), 2.94 (d, 3H, *J*=1), 3.82 (s, 3H), 6.74 (d, 1H, *J*=7), 6.87 (d, 2H, *J*=9), 7.21 (dd, 2H, *J*=10, *J*=1.5), 7.50 (dd, 1H, *J*=8, *J*=2), 7.63 (d, 2H, *J*=9), 8.02 (t, 1H, *J*=8), 8.16 (d, 2H, *J*=7); MS [M+1]⁺: 396 |
| 189 | 3-(3-Chloro-4-methanesulfinylphenyl)-2-(4-methoxyphenyl)imidazo[1,2-*a*]pyrimidine; ¹H-NMR: 2.93 (s, 3H), 3.82 (s, 3H), 6.87 (dd, 1H, *J*=7, *J*=4), 6.87 (d, 2H, *J*=9), 7.50 (d, 1H, *J*=1.5), 7.62-7.65 (m, 1H), 7.63 (d, 2H, *J*=9), 8.11 (d, 1H, *J*=8.5), 8.30 (dd, 1H, *J*=7, *J*=2), 8.58 (dd, 2H, *J*=4, *J*=2); MS [M/M+2]⁺: 398/400 |
| 190 | 3-(3-Chloro-4-methanesulfinylphenyl)-2-(4-methoxyphenyl)-7-methylimidazo[1,2-*a*]pyrimidine; ¹H-NMR: 2.65 (s, 3H), 2.93 (s, 3H), 3.82 (s, 3H), 6.74 (d, 1H, *J*=7), 6.86 (d, 2H, *J*=8.5), 7.48 (s, 1H), 7.63 (m, 3H), 8.10 (d, 1H, *J*=8), 8.14 (d, 2H, *J*=7); MS [M/M+2]⁺: 412/414 |
| 191 | 3-(3-Chloro-4-methanesulfinylphenyl)-2-(3-chloro-4-methoxyphenyl)-7-methylimidazo[1,2-*a*]pyrimidine; ¹H-NMR: 2.67 (s, 3H), 2.94 (s, 3H), 3.91 (s, 3H), 6.76 (d, 1H, *J*=7), 6.87 (d, 1H, *J*=8.5), 7.48 (d, 1H, *J*=1.5), 7.51 (dd, 1H, *J*=8.5, *J*=2), 7.63 (dd, 1H, *J*=8.5, *J*=2), 7.77 (d, 1H, *J*=2), 8.13 (d, 1H, *J*=8), 8.14 (d, 1H, *J*=7); MS [M/M+2/M+4]⁺: 446/448/450 |
| 192 | 3-(3-Chloro-4-methanesulfinyl-5-methylphenyl)-2-(4-methoxyphenyl)imidazo[1,2-*a*]pyrimidine; ¹H-NMR: 2.63 (s, 3H), 2.95 (s, 3H), 3.92 (s, 3H), 6.85 (dd, 1H, *J*=7, *J*=4), 7.11 (d, 2H, *J*=9), 7.36 (d, 2H, *J*=9), 7.61 (s, 1H), 7.62 (s, 1H), 8.20 (dd, 1H, *J*=7, *J*=2), 8.59 (dd, 1H, *J*=4, *J*=2); MS [M/M+2]⁺: 412/414 |
| 193 | 3-(3-Chloro-4-methanesulfinyl-5-methylphenyl)-2-(4-methoxyphenyl)-7-methylimidazo[1,2-*a*]pyrimidine; ¹H-NMR: 2.62 (s, 3H), 2.65 (s, 3H), 2.94 (s, 3H), 3.92 (s, 3H), 6.70 (d, 1H, *J*=7), 7.10 (d, 2H, *J*=9), 7.34 (d, 2H, *J*=9), 7.60 (m, 1H), 7.61 (s, 3H), 8.03 (d, 1H, *J*=7); MS [M/M+2]⁺: 420/422 |
| 194 | 3-(3-tert-Butyl-4-methanesulfinylphenyl)-2-(4-methoxyphenyl)imidazo[1,2-*a*]pyrimidine; ¹H-NMR: 1.40 (s, 9H), 2.85 (s, 3H), 3.82 (s, 3H), 6.84 (dd, 1H, *J*=7, *J*=4), 6.85 (d, 2H, *J*=9), 7.45 (d, 1H, *J*=1.5), 7.62 (dd, 1H, *J*=8, *J*=1.5), 7.65 (d, 2H, *J*=9), 8.33 (dd, 1H, *J*=7, *J*=2), 8.41 (d, 1H, *J*=8), 8.57 (dd, 1H, *J*=4, *J*=2); MS [M+1]⁺: 420 |
| 195 | 3-[3-Chloro-4-(propane-1-sulfinyl)phenyl]-2-*p*-tolylimidazo[1,2-*a*]pyrimidine; ¹H-NMR: 1.14 (t, 3H, *J*=7), 1.71-1.87 (m, 1H), 1.93-2.07 (m, 1H), 2.36 (s, 3H), 2.82-2.92 (m, 1H), 3.13 (ddd, 1H, *J*=7, *J*=9, *J*=13), 6.89 (dd, 1H, *J*=7, *J*=4), 7.14 (d, 2H, *J*=8), 7.49 (d, 1H, *J*=1.5), 7.58 (d, 2H, *J*=8), 7.61 (dd, 1H, *J*=8, *J*=1.5), 8.06 (d, 1H, *J*=8), 8.30 (dd, *J*=7, *J*=2), 8.59 (dd, 1H, *J*=4, *J*=2); MS [M/M+2]⁺: 410/412 |
| 196 | 3-(3-Chloro-4-methoxyphenyl)-2-(4-methanesulfonylphenyl)imidazo[1,2-*a*]pyrimidine; ¹H-NMR: 3.05 (s, 3H), 4.01 (s, 3H), 6.90 (dd, 1H, *J*=7, *J*=4), 7.12 (d, 1H, *J*=8.5), 7.29 (dd, 1H, *J*=8.5, *J*=2), 7.48 (d. 1H, *J*=2), 7.86 (d, 2H, *J*=8.5), 7.95 (d, 2H, *J*=8.5), 8.21 (dd, 1H, *J*=7, *J*=2), 8.62 (dd, 1H, *J*=4, *J*=2); MS [M/M+2]⁺: 414/416 |
| 197 | 2-(4-Methoxyphenyl)-3-[4-(propane-2-sulfinyl) phenyl] imidazo [1, 2-*a*] pyrimidine; ¹H-NMR: 1.22 (d, 3H, *J*=6.5), 1.32 (d, 6H, *J*=6.5), 2.93 (m, 1H), 3.81 (s, 3H), 6.83 (d, 2H, *J*=9), 6.85 (dd, 1H, *J*=7, *J*=4), 7.61 (d, 2H, *J*=8), 7.62 (d, 2H, *J*=9), 7.77 (d, 2H, *J*=8), 8.26 (dd, 1H, *J*=7, *J*=2), 8.57 (dd, 1H, *J*=4, *J*=2); MS [M+1]⁺: 392 |
| 198 | 2-(4-Methoxyphenyl)-7-methyl-3-[4-(propane-2-sulfinyl)phenyl]imidazo[1,2-*a*]pyrimidine; ¹H-NMR: 1.23 (d, 3H, *J*=7), 1.32 (d, 6H, *J*=7), 2.65 (s, 3H), 2.93 (m, 1H), 3.81 (s, 3H), 6.71 (d, 1H, *J*=7), 6.83 (d, 2H, *J*=9), 7.60 (d, 2H, *J*=8), 7.62 (d, 2H, *J*=8.5), 7.75 (d, 2H, *J*=8.5), 8.11 (d, 1H, *J*=7); MS [M+1]⁺: 406 |
| 199 | 3-[3-Chloro-4-(propane-2-sulfinyl)phenyl]-2-(4-methoxyphenyl)imidazo[1,2-*a*]pyrimidine; ¹H-NMR: 1.12 (d, 3H, *J*=7), 1.52 (d, 6H, *J*=7), 3.27 (m, 1H), 3.83 (s, 3H), 6.86 (d, 2H, *J*=9), 6.87 (dd, 1H, *J*=7, *J*=4), 7.49 (d, 2H, *J*=1.5), 7.60 (dd, 1H, *J*=8, *J*=1.5), 7.62 (d, 2H, *J*=9), 7.98 (d, 1H, *J*=8), 8.28 (dd, 1H, *J*=7, *J*=2), 8.59 (dd, 1H, *J*=4 , *J*=2) ; MS [M/M+2]⁺: 426/428 |
| 200 | 3-(4-Cyclohexylmethanesulfinylphenyl)-2-(4-methoxyphenyl)imidazo[1,2-*a*]pyrimidine; ¹H-NMR: 1.10-1.43 (m, 5H), 1.70-1.81 (m, 4H), 1.98-2.09 (m, 1H), 2.10-2.20 (m, 1H), 2.61 (dd, 1H, *J*=13, *J*=9.5), 2.91 (dd, 1H, *J*=13, *J*=4.5), 3.81 (s, 3H), 6.82 (dd, 1H, *J*=7, *J*=4), 6.85 (d, 2H, *J*=9), 7.62 (d, 2H, *J*=8), 7.64 (d, 2H, *J*=8), 7.81 (d, 2H, *J*=8), 8.27 (dd, 1H, *J*=7, *J*=4), 8.57 (dd, 1H, *J*=4, *J*=2); MS [M+1]⁺: 446 |
| 201 | 3-(4-Cyclohexylmethanesulfinylphenyl)-2-(4-methoxyphenyl)-7-methylimidazo[1,2-*a*]pyrimidine; ¹H-NMR: 1.04-1.37 (m, 5H), 1.69-1.81 (m, 4H), 1.90-2.19 (m, 2H), 2.59 (dd, 1H, *J*=13. *J*=9.5), 2.64 (s, 3H), 2.90 (dd, 1H, *J*=13, *J*=4.5), 3.80 (s, 3H), 6.70 (d, 1H, *J*=7), 6.83 (d, 2H, *J*=9), 7.60 (d, 2H, *J*=8.5), 7.63 (d, 2H, *J*=9), 7.79 (d, 2H, *J*=8), 8.11 (d, 1H, *J*=7); MS [M+1]⁺: 460 |
| 202 | 2-[3-Chloro-4-(propane-1-sulfonyl)phenyl]-3-*p*-tolylimidazo[1,2-a]pyrimidine; ¹-H-NMR: 1.04 (t, 3H, *J*=7.5), 1.67-1.80 (m, 2H), 2.50 (s, 3H), 3.35-3.40 (m, 2H), 6.87 (dd, 1H, *J*=7, *J*=4), 7.32 (d, 1H, *J*=8), 7.41 (d, 2H, *J*=8), 7.67 (dd, 1H, *J*=8.5, *J*=2), 7.96 (d, 2H, *J*=8.5), 8.14 (d, 1H, *J*=2), 8.22 (dd, 1H, *J*=7, *J*=2), 8.61 (dd, 1H, *J*=4, *J*=2); MS [M/M+2]⁺: 426/428 |
| 203 | 2-(4-Ethoxy-3-fluorophenyl)-3-(4-methanesulfonylphenyl)imidazo[1,2-*a*]pyrimidine; ¹H-NMR: 1.45 (t, 3H, *J*=7), 3.17 (s, 3H), 4.11 (q, 2H, *J*=7), 6.88 (dd, 1H, *J*=7, *J*=4), 6.89 (t, 1H, *J*=8.5), 7.36 (ddd, 1H, *J*=8.5, *J*=2, *J*=1), 7.42 (dd, 1H, *J*=12.5, *J*=2), 7.69 (d, 2H, *J*=8.5), 8.12 (d, 2H, *J*=8.5), 8.29 (dd, 1H, *J*=7, *J*=2), 8.60 (dd, 1H, *J*=4, *J*=2); MS [M+1]⁺: 412 |
| 204 | 2-(4-Ethoxy-3-fluorophenyl)-3-(4-methanesulfonylphenyl)-7-methylimidazo[1,2-*a*]pyrimidine; ¹H-NMR: 1.45 (t, 3H, *J*=7), 2.66 (s, 3H), 3.17 (s, 3H), 4.12 (q, 2H, *J*=7), 6.75 (d, 1H, *J*=7), 6.89 (t, 1H, *J*=8.5), 7.39 (ddd, 1H, *J*=8.5 *J*=2, *J*=1), 7.42 (dd, *J*=8.5, *J*=2), 7.66 (d, 2H, *J*=8.5), 8.11 (d, 2H, *J*=2), 8.13 (d, 1H, *J*=7); MS [M+1]⁺: 418 |
| 205 | 3-(2-Chloro-4-methanesulfonylphenyl)-2-(4-methoxyphenyl)imidazo[1,2-*a*]pyrimidine; ¹H-NMR: 3.20 (s, 3H), 3.81 (s, 3H), 6.86 (d, 2H, *J*=9), 6.91 (dd, 1H, *J*=7, *J*=4), 7.57 (d, 2H, *J*=9), 7.62 (d, 1H, *J*=8), 7.90 (dd, 1H, *J*=7, *J*=2), 7.93 (dd, 1H, *J*=8, *J*=2), 8.24 (d, 1H, *J*=2), 8.63 (dd, 2H, *J*=4, *J*=2); MS [M/M+2]⁺: 414/416 |
| 206 | 3-(3-Chloro-4-methanesulfonylphenyl)-2-(4-methoxyphenyl)imidazo[1,2-*a*]pyrimidine; ¹H-NMR: 3.37 (s, 3H), 3.83 (s, 3H), 6.91 (d, 2H, *J*=9), 6.92 (dd, 1H, *J*=7, *J*=4), 7.57 (dd, 1H, *J*=8, *J*=2), 7.62 (d, 2H, *J*=9), 7.68 (d, 1H, *J*=2), 8.29 (d, 1H, *J*=8), 8.35 (dd, 1H, *J*=7, *J*=2), 8.61 (dd, 2H, *J*=4, *J*=2); MS [M/M+2]⁺: 414/416 |
| 207 | 3-(3-Chloro-4-methanesulfonylphenyl)-2-(3-fluoro-4-methoxyphenyl)imidazo[1,2-*a*]pyrimidine; ¹H-NMR: 3.39 (s, 3H), 3.92 (s, 3H), 6.93 (d, 1H, *J*=8.5), 6.94 (dd, 1H, *J*=7, *J*=4), 7.58 (dd, 1H, *J*=2, *J*=1), 7.47 (dd, 1H, *J*=8.5, *J*=2), 7.57 (dd, 1H, *J*=8.5, *J*=1), 7.68 (d, 1H, *J*=1.5), 8.33 (dd, 1H, *J*=7, *J*=2), 8.32 (d, 1H, *J*=8.5), 8.64 (dd, 1H, *J*=4, *J*=2); MS [M/M+2]⁺: 400/402 |
| 208 | 3-(3-Chloro-4-methanesulfonyl-5-methylphenyl)-2-(4-methoxyphenyl)imidazo[1,2-*a*]pyrimidine; ¹H-NMR: 2.67 (s, 3H), 3.28 (s, 3H), 3.93 (s, 3H), 6.86 (dd, 1H, 208 J=7, *J*=4), 7.12 (d, 2H, *J*=9), 7.36 (d, 2H, *J*=9), 7.71 (d, 1H, *J*=1.5), 7.78 (d, 1H, *J*=1.5), 8.20 (dd, 1H, *J*=7, *J*=2), 8.61 (dd, 1H, *J*=4, *J*=2); MS [M/M+2]⁺: 428/430 |
| 209 | 3-(3-Chloro-4-methanesulfonyl-5-methylphenyl)-2-(4-methoxyphenyl)-7-methylimidazo[1,2-*a*]pyrimidine; ¹H-NMR: 2.66 (s, 6H), 3.28 (s, 3H), 3.92 (s, 3H), 6.72 (d, 1H, *J*=7), 7.11 (d, 2H, *J*=9), 7.34 (d, 2H, *J*=9), 7.71 (d, 1H, *J*=2), 7.77 (d, 1H, *J*=2), 8.04 (d, 1H, *J*=7); MS [M/M+2]⁺: 442/444 |
| 210 | 3-[3-Chloro-4-(propane-1-sulfonyl)phenyl]-2-*p*-tolylimidazo[1,2-*a*]pyrimidine; ¹H-NMR: 1.10 (t, 3H, *J*=7.5), 1.81-91 (m, 2H), 2.37 (s, 3H), 3.44-3.50 (m, 2H), 6.93 (dd, 1H, *J*=7, *J*=4), 7.16 (d, 2H, *J*=8), 7.56 (d, 2H, *J*=8), 7.66 (d, 1H, *J*=2), 7.68 (dd, 1H, *J*=8.5, *J*=1.5), 8.25 (d, 1H, *J*=8.5), 8.35 (dd, *J*=7, *J*=2), 8.63 (dd, 1H, *J*=4, *J*=2); MS [M/M+2]⁺: 471/473 |
| 211 | 2-(4-Methoxyphenyl)-3-[4-(propane-2-sulfonyl)phenyl]imidazo[1,2-*a*]pyrimidine; ¹H-NMR: 1.38 (d, 6H, *J*=7), 3.29 (m, 1H), 3.82 (s, 3H), 6.87 (d, 2H, *J*=8.5), 6.88 (dd, 1H, *J*=7, *J*=4), 7.60 (d, 2H, *J*=9), 7.67 (d, 2H, *J*=8), 8.04 (d, 2H, *J*=8.5), 8.32 (dd, 1H, *J*=7, *J*=2), 8.59 (dd, 1H, *J*=4, *J*=2); MS [M+1]⁺: 408 |
| 212 | 2-(4-Methoxyphenyl)-7-methyl-3-[4-(propane-2-sulfonyl)phenyl]imidazo[1,2-*a*]pyrimidine; ¹H-NMR: 1.38 (d, 6H, *J*=6.5), 2.65 (s, 3H), 3.29 (m, 1H), 3.82 (s, 3H), 6.73 (d, 1H, *J*=7), 6.83 (d, 2H, *J*=8.5), 7.59 (d, 2H, *J*=8.5), 7.64 (d, 2H, *J*=8), 8.01 (d, 2H, *J*=8), 8.16 (dd, 1H, *J*=7); MS [M+1]⁺: 422 |
| 213 | 3-(4-Cyclohexylmethanesulfonylphenyl)-2-(4-methoxyphenyl)imidazo[1,2-*a*]pyrimidine; ¹H-NMR: 1.05-1.34 (m, 5H), 1.64-1.75 (m, 4H), 1.89-1.94 (m, 2H), 3.08 (d, 2H, *J=*6), 3.81 (s, 3H), 6.84 (d, 2H, *J*=8.5), 6.88 (dd, 1H, *J*=7, *J*=4), 7.59 (d, 2H, *J*=8.5), 7.67 (d, 2H, *J*=8.5), 8.06 (d, 2H, *J*=8.5), 8.30 (dd, 1H, *J*=7, *J*=2), 8.59 (dd, 1H, *J*=4, *J*=2); MS [M+1]⁺: 462 |
| 214 | 3-(4-Cyclohexylmethanesulfonylphenyl)-2-(4-methoxyphenyl)-7-methylimidazo[1,2-*a*]pyrimidine; ¹H-NMR: 1.03-1.39 (m, 5H), 1.69-1.74 (m, 4H), 1.85-1.94 (m, 2H), 2.66 (s, 3H), 3.07 (d, 2H, *J*=6), 3.81 (s, 3H), 6.73 (d, 1H, *J*=7), 6.84 (d, 2H, *J*=8.5), 7.60 (d, 2H, *J*=9), 7.64 (d, 2H, *J*=8.5), 8.04 (d, 2H, *J*=8.5), 8.15 (d, 1H, *J*=7); MS [M+1]⁺: 476 |
| 215 | 3-(4-Cyclopropanesulfonylphenyl)-2-(4-methoxyphenyl)-7-methylimidazo[1,2-*a*]pyrimidine; ¹H-NMR (d₆-DMSO): 1.09-1.17 (m, 2H), 1.41-1.47 (m, 2H), 2.54-2.59 (m, 1H), 2.65 (s, 3H), 3.82 (s, 3H), 6.74 (d, 1H, *J*=7), 6.85 (d, 2H, *J*=9), 7.62 (d, 2H, *J*=8.5), 7.65 (d, 2H, *J*=8.5), 8.04 (d, 2H, *J*=8.5), 8.17 (d, 1H, *J*=7); MS [M+1]⁺: 420 |
| 216 | 4-(7-Methyl-3-phenylimidazo[1,2-*a*]pyrimidin-2-yl)benzenesulfinic acid; ¹H-NMR (d₆-DMSO) : 2.54 (s, 3H), 6.89 (d, 1H, *J*=7), 7.37 (d, 2H, *J*=8.5), 7.47-7.60 (m, 7H), 8.32 (d, 1H, *J*=7); MS [M+1]⁺: 350 |
| 217 | 2-Chloro-4-[2-(4-methoxyphenyl)-7-methylimidazo[1,2-*a*]pyrimidin-3-yl]benzenesulfonamide; ¹H-NMR (d₆-DMSO) : 2.55 (s, 3H), 3.85 (s, 3H), 6.91 (d, 1H, *J*=7), 7.15 (d, 2H, *J*=9), 7.30-7.35 (m, 3H), 7.42 (d, 2H, *J*=9), 7.48-7.52 (m, 1H), 7.66-7.67 (m, 1H), 8.26 (d, 1H, *J*=7) |
| 218 | 4-[2-(4-Methoxyphenyl)imidazo[1,2-a]pyrimidin-3-yl]-2-methylbenzenesulfonamide; ¹H-NMR (d₆-DMSO): 2.63 (s, 3H), 3.75 (s, 3H), 6.92 (d, 2H, *J*=9), 7.02 (dd, 1H, *J*=7, *J*=4), 7.47 (dd, 1H, *J*=8, *J*=1.5), 7.51 (s, 2H), 7.54 (d, 2H, *J*=9),7.57 (d, 1H, *J*=1.5), 7.98 (d, 1H, *J*=8), 8.54 (dd, 1H, *J*=7, *J*=2), 8.56 (dd, 2H, *J*=4, *J*=2) ; MS [M+1]⁺: 395 |
| 219 | 4-[2-(4-Methoxyphenyl)-7-methylimidazo[1,2-*a*]pyrimidin-3-yl]-2-methylbenzenesulfonamide; ¹H-NMR (d₆-DMSO): 2.55 (s, 3H), 2.62 (s, 3H), 3.74 (s, 3H), 6.91 (d, 2H, *J*=9), 6.91 (d, 1H, *J*=7), 7.44 (dd, 1H, *J*=8, *J*=1.5), 7.49 (s, 2H), 7.51 (d, 2H, *J*=9), 7.52 (d, 1H, *J*=1.5), 7.97 (d, 1H, *J*=8), 8.41 (d, 1H, *J*=7); MS [M+1]⁺: 409 |
| 220 | Acetic acid 4-(7-methyl-3-phenylimidazo[1,2-*a*]pyrimidin-2-yl)phenylsulfanylmethyl ester; ¹H-NMR: 2.10 (s, 3H), 2.64 (s, 3H), 5.40 (s, 2H), 6.68 (d, 1H, *J*=7), 7.35 (d, 2H, *J*=9), 7.42-7.45 (m, 2H), 7.51-7.57 (m, 3H), 7.73 (d, 2H, *J*=9), 8.07 (d, 1H, *J*=7); MS [M+1]⁺: 390 |
| 221 | Acetic acid 4-(7-methyl-3-phenylimidazo[1,2-*a*]pyrimidin-2-yl)benzenesulfonylmethyl ester; ¹H-NMR: 2.07 (s, 3H), 2.67 (s, 3H), 5.12 (s, 2H), 6.73 (d, 1H, *J*=7), 7.41-7.44 (m, 2H), 7.55-7.60 (m, 3H), 7.81 (d, 2H, *J*=9), 7.96 (d, 2H, *J*=9), 8.08 (d, 1H, *J*=7); MS [M+1]⁺: 422 |
| 222 | 3-(3-Methoxyphenyl)-2-(4-methoxyphenyl)-7-methylimidazo[1,2-*a*]pyrimidine; ¹H-NMR: 2.63 (s, 3H), 3.80 (s, 3H), 3.81 (s, 3H), 6.65 (d, 1H, *J*=7), 6.83 (d, 2H, *J*=9), 6.95 (dd, 1H, *J*=2.5, *J*=1), 7.00-7.05 (m, 2H), 7.45 (dd, 1H, *J*=8.5, *J*=7.5), 7.72 (d, 2H, *J*=9), 8.09 (d, 1H, *J*=7); MS [M+1]⁺: 346 |
| 223 | Acetic acid 4-[3-(4-bromophenyl)imidazo[1,2-*a*]pyrimidin-2-yl]phenylsulfanylmethyl ester; ¹H-NMR: 2.16 (s, 3H), 5.53 (s, 2H), 6.91 (dd, 1H, *J*=7, *J*=4), 7.41 (d, 2H, *J*=8.5), 7.45 (d, 2H, *J*=8.5), 7.62 (d, 2H, *J*=8.5), 7.63 (d, 2H, *J*=8.5), 8.27 (dd, 1H, *J*=7, *J*=4), 8.62 (dd, 1H, *J*=4, *J*=2); MS [M/M+2]⁺: 454/456 |
| 224 | Acetic acid 4-[3-(4-bromophenyl)imidazo[1,2-*a*]pyrimidin-2-yl]benzenesulfinylmethyl ester; ¹H-NMR: 2.15 (s, 3H), 4.82 (d, 1H, *J*=10), 5.08 (d, 1H, *J*=10), 6.91 (dd, 1H, *J*=7, *J*=4), 7.34 (d, 2H, *J*=8.5), 7.63 (d, 2H, *J*=8.5), 7.73 (d, 2H, *J*=8.5), 7.92 (d, 2H, *J*=8.5), 8.24 (d, 1H, *J*=7, *J*=2), 8.63 (d, 1H, *J*=4, *J*=2); MS [M/M+2]⁺: 470/472 |
| 225 | Acetic acid 4-[2-(4-bromophenyl)imidazo[1,2-*a*]pyrimidin-3-yl]benzenesulfinylmethyl ester; ¹H-NMR: 2.19 (s, 3H), 4.97 (d, 1H, *J*=10), 5.20 (d, 1H, *J*=10), 6.90 (dd, 1H, *J*=7, *J*=4), 7.44 (d, 2H, *J*=8.5), 7.55 (d, 2H, *J*=8.5), 7.65 (d, 2H, *J*=8.5), 7.88 (d, 2H, *J*=8.5), 8.28 (d, 1H, *J*=7, *J*=2), 8.61 (d, 1H, *J*=4, *J*=2); MS [M/M+2]⁺: 470/472 |
| 226 | Acetic acid 4-[3-(4-bromophenyl)imidazo[1,2-*a*]pyrimidin-2-yl]benzenesulfonylmethyl ester; ¹H-NMR: 2.07 (s, 3H), 5.12 (s, 2H), 6.89 (dd, 1H, *J*=7, *J*=4), 7.33 (d, 2H, *J*=8.5), 7.73 (d, 2H, *J*=8.5), 7.83 (d, 2H, *J*=9), 7.93 (d, 2H, *J*=9), 8.22 (dd, 1H, *J*=7, *J*=2), 8.61 (dd, 1H, *J*=4, *J*=2); MS [M/M+2]⁺: 486/488 |
| 227 | Acetic acid 4-[2-(4-bromophenyl)imidazo[1,2-*a*]pyrimidin-3-yl]benzenesulfonylmethyl ester; ¹H-NMR: 2.12 (s, 3H), 5.23 (s, 2H), 6.91 (dd, 1H, *J*=7, *J*=4), 7.44 (d, 2H, *J*=8.5), 7.50 (d, 2H, *J*=8.5), 7.68 (d, 2H, *J*=8.5), 8.08 (d, 2H, *J*=8.5), 8.32 (dd, 1H, *J*=7, *J*=2), 8.56 (dd, 1H, *J*=4, *J*=2); MS [M/M+2]⁺: 486/488 |
| 228 | 4-[2-(4-Methanesulfinylphenyl)-7-methylimidazo[1,2-*a*]pyrimidin-3-yl]benzenesulfonamide; ¹H-NMR (d₆-DMSO): 2.59 (s, 3H), 2.74 (s, 3H), 7.00 (d, 1H, *J*=7), 7.45 (s, 2H), 7.65 (d, 2H, *J*=8.5), 7.75-7.80 (m, 4H), 7.96-8.02 (m, 2H), 8.37 (d, 2H, *J*=7); MS [M+1]⁺: |
| 229 | 4-[2-(4-Hydroxy-3-methylphenyl)-7-methylimidazo[1,2-*a*]pyrimidin-3-yl]benzenesulfonamide; ¹H-NMR (d₆-DMSO): 2.06 (s, 3H), 2.58 (s, 3H), 6.68 (d, 1H, *J*=8.5), 6.91 (d, 1H, *J*=7), 7.11 (d, 1H, *J*=8.5), 7.39 (s, 1H), 7.48 (s, 2H), 7.68 (d, 2H, *J*=8), 7.96 (d, 2H, *J*=8), 8.44 (d, 2H, *J*=7), 9.44 (s, 1H); MS [M+1]⁺: 395 |
| 230 | *N*-Acetyl-4-(7-methyl-2-phenylimidazo[1,2-*a*]pyrimidin-3-yl)benzenesulfonamide; ¹H-NMR (d₆-DMSO): 1.95 (s, 3H), 2.57 (s, 3H), 6.97 (d, 1H, *J*=7), 7.31-7.35 (m, 3H), 7.51-7.54 (m, 2H), 7.74 (d, 2H, *J*=8), 8.04 (d, 2H, *J*=8), 8.51 (d, 2H, *J*=7), 12.23 (br., 1H) |
| 231 | *N*-Acetyl-4-[2-(4-methoxyphenyl)-7-methylimidazo[1,2-*a*]pyrimidin-3-yl]benzenesulfonamide; ¹H-NMR (d₆-DMSO): 2.03 (s, 3H), 2.62 (s, 3H), 3.78 (s, 3H), 6.70 (d, 1H, *J*=7), 6.82 (d, 2H, *J*=9), 7.56 (d, 2H, *J*=8.5), 7.58 (d, 2H, *J*=9), 8.14 (d, 2H, *J*=8.5), 8.15 (d, 2H, *J*=7); MS [M+1]⁺: 437 |
| 232 | *N*-Acetyl-4-[2-(3-chloro-4-methoxyphenyl)-7-methylimidazo[1,2-*a*]pyrimidin-3-yl]benzenesulfonamide; ¹H-NMR (d₆-DMSO) 1.97 (s, 3H), 2.57 (s, 3H), 3.85 (s, 3H), 6.97 (d, 1H, *J*=7), 7.13 (d, 1H, *J*=8.5), 7.40 (dd, 1H, *J*=8.5, *J*=2.5), 7.57 (d, 1H, *J*=2.5), 7.77 (d, 2H, *J*=8.5), 8.06 (d, 2H, *J*=8.5), 8.48 (d, 2H, *J*=7); MS [M/M+2]⁺: 471/473 |
| 233 | *N*-Acetyl-4-[2-(4-methoxy-3-methylphenyl)-7-methylimidazo[1,2-*a*]pyrimidin-3-yl]benzenesulfonamide; ¹H-NMR (d₆-DMSO): 2.06 (s, 3H), 2.16 (s, 3H), 2.64 (s, 3H), 3.82 (s, 3H), 6.73 (d, 1H, *J*=8.5), 6.74 (d, 1H, *J*=7), 7.35 (ddd, 1H, *J*=8.5, *J*=2, *J*=1), 7.57 (dd, 1H, *J*=2, *J*=1), 7.60 (d, 2H, *J*=8.5), 8.17 (d, 2H, *J*=8.5), 8.20 (d, 2H, *J*=7); MS [M+1]⁺: 451 |
| 234 | 4-[2-(4-Fluorophenyl)imidazo[1,2-*a*]pyrimidin-3-yl]benzonitrile; ¹H-NMR: 6.92 (dd, 1H, *J*=7, *J*=4), 7.02 (t, 2H, *J*=8.5), 7.59 (d, 2H, *J*=8.5), 7.62 (d, 1H, *J*=8.5), 7.64 (d, 1H, *J*=8.5), 7.84 (d, 2H, *J*=8.5), 8.31 (dd, *J*=7, *J*=2), 8.62 (dd, 1H, *J*=4, *J*=2); MS [M+1]⁺: 315 |
| 235 | 4-[3-(4-Fluorophenyl)imidazo[1,2-*a*]pyrimidin-2-yl]benzonitrile; ¹H-NMR: 6.94 (dd, 1H, *J*=7, *J*=4), 7.31 (t, 2H, *J*=8.5), 7.43 (d, 1H, *J*=9), 7.46 (d, 1H, *J*=9), 7.61 (d, 2H, *J*=8.5), 7.83 (d, 2H, *J*=8.5), 8.22 (dd, *J*=6.5, *J*=1), 8.64 (dd, 1H, *J*=4, *J*=1); MS [M+1]⁺: 315 |
| 236 | 2-(4-Fluorophenyl)-3-(4-nitrophenyl)imidazo[1,2-a]pyrimidine; MS [M+1]⁺: 335 |
| 237 | 237 3-(4-Fluorophenyl)-2-(4-nitrophenyl)imidazo[1,2-a]pyrimidine; MS [M+1]⁺: 3.35 |
| 238 | 3-(3-Chloro-4-methylsulfanylphenyl)-2-(4-chlorophenyl)imidazo[1,2-*a*]pyrimidine; MS [M/M+2/M+4]⁺: 386/388/390 |
| 239 | 3-(3-Chloro-4-methylsulfanylphenyl)-2-(4-chlorophenyl)-7-methylimidazo[1,2-*a*]pyrimidine; MS [M/M+2/M+4]⁺: 400/402/404 |
| 240 | 3-(3-Chloro-4-methylsulfanylphenyl)-2-(4-fluorophenyl)imidazo[1,2-*a*]pyrimidine; MS [M/M+2]⁺: 370/372 |
| 241 | 3-(3-Chloro-4-methylsulfanylphenyl)-2-(4-fluorophenyl)-7-methylimidazo[1,2-*a*]pyrimidine; MS [M/M+2]⁺: 384/386 |
| 242 | 3-Benzo[1,3]dioxol-5-yl-2-(4-methoxyphenyl)imidazo[1,2-*a*]pyrimidine; ¹H-NMR: 3.81 (s, 3H), 6.08 (s, 2H), 6.80 (dd, 1H, *J*=7, *J*=4), 6.85 (d, 2H, *J*=9), 6.85 (d, 1H, *J*=2), 6.91 (dd, 1H, *J*=8, *J*=2), 6.98 (d, 1H, *J*=8), 7.72 (d, 2H, *J*=9), 8.20 (dd, 1H, *J*=7, *J*=2), 8.51 (dd, 1H, *J*=4, *J*=2); MS [M+1]⁺: 346 |
| 243 | 3-(4,4-Dimethylthiochroman-6-yl)-7-methyl-2-(4-methylsulfanylphenyl)imidazo[1,2-*a*]pyrimidine; ¹H-NMR: 1.27 (s, 6H), 1.99-2.03 (m, 2H), 2.47 (s, 3H), 2.63 (s, 3H), 3.07-3.11 (m, 2H), 6.66 (d, 1H, *J*=7), 7.09 (dd, 1H, *J*=8, *J*=2), 7.17 (d, 2H, *J*=8.5), 7.25 (d, 1H, *J*=8), 7.38 (d, 1H, *J*=2), 7.73 (d, 2H, *J*=8.5), 8.08 (d, 1H, *J*=7); MS [M+1]⁺: 432 |
| 244 | 3-(4,4-Dimethylthiochroman-6-yl)-2-(4-methoxyphenyl)imidazo[1,2-*a*]pyrimidine; ¹H-NMR: 1.27 (s, 6H), 1.99-2.03 (m, 2H), 3.08-3.12 (m, 2H), 3.81 (s, 3H), 6.79 (dd, 1H, *J*=7, *J*=4), 6.85 (d, 2H, *J*=9), 7.12 (dd, 1H, *J*=8, *J*=2), 7.26 (d, 1H, *J*=8), 7.40 (d, 1H, *J*=2), 7.75 (d, 2H, *J*=9), 8.25 (dd, 1H, *J*=7, *J*=4), 8.52 (dd, 1H, *J*=4, *J*=2); MS [M+1]⁺: 402 |
| 245 | 3-(4,4-Dimethylthiochroman-6-yl)-2-(4-methoxyphenyl)-7-methylimidazo[1,2-*a*]pyrimidine; ¹H-NMR: 1.26 (s, 6H), 1.98-2.02 (m, 2H), 2.61 (s, 3H), 3.06-3.10 (m, 2H), 3.79 (s, 3H), 6.65 (d, 1H, *J*=7), 6.82 (d, 2H, *J*=9), 7.09 (dd, 1H, *J*=8, *J*=2), 7.23 (d, 1H, *J*=8), 7.38 (d, 1H, *J*=2), 7.73 (d, 2H, *J*=9), 8.09 (d, 1H, *J*=7); MS [M+1]⁺:416 |
| 246 | 4-[3-(4-Bromophenyl)imidazo[1,2-*a*]pyrimidin-2-yl]benzenesulfonamide; ¹H-NMR (d₆-DMSO): 7.06 (dd, 1H, *J*=7, *J*=4), 7.31 (s, 2H), 7.50 (d, 2H, *J*=8.5), 246 7.74 (d, 2H, *J*=8.5), 7.79 (d, 2H, *J*=9), 7.80 (d, 2H, *J*=9), 8.54 (dd, 1H, *J*=7, *J*=2), 8.62 (dd, 1H, *J*=4, *J*=2); MS [M/M+2]⁺: 429/431 |

### Determination of apoptosis

To stablish whether the antiproliferative activity of the compounds of the present invention is due to an apoptotic process, and not merely to a necrotic process, the generation of DNA fragments associated to histones (mono- and oligonucleosomes) was determined after incubation of the human colon cancer cell lines HCT-116 with the compounds of the present invention at different concentrations. DNA fragmentation into nucleosomes, which is an indicator of apoptosis phenomena, was quantified by a sandwich inmunoassay using monoclonal antibodies directed against DNA and histones (Cell Death Detection ELISA^{PLUS}, Roche Diagnostics, cat #1920685). The quantity of fragmented DNA was expressed with the *Enrichment Factor* (EF) parameter, which is a coefficient of absorbance of nucleosomes liberated in the cytosol of the cells cultured in the presence of the products compared with control cells.

### Determination of cell proliferation inhibition

The inhibitory capacity of cell proliferation of the compounds was determined in two human colon adenocarcinoma cell lines (HCT-116) obtained at the ATCC (American Type Collection). The cells were seeded in 96-well plates and kept at 37°C in a CO₂ heater for 24 hours to allow cell-substrate-adhesion. Subsequently, cells were treated with the products under study at concentrations comprised between 1 and 100 µM for 48 hours. After the treatment period, the medium was removed and cells were dyed with Sulforodamine B. Finally, decolouration of the dyed cells was carried out with Tris base 10 mM and the plates were read at 493-530 nm in a plate reader. IC₅₀ was calculated as the product concentration inducing a growth inhibition of the 50% compared with control cells not treated.

The Table 2 below shows biological results of the two mentioned assays for some of the examples of the present invention.

**TABLE 2**

| EXAMPLES | IC₅₀ | EF | COX-2 |
|---|---|---|---|
| Celecoxib | + | + | +++ |
| Exisulind | + | + | + |
| 8 | +++ | +++ | ++ |
| 13 | +++ | +++ | + |
| 86 | +++ | +++ | ND |
| 124 | +++ | +++ | + |
| 125 | ++ | +++ | ND |
| 132 | +++ | +++ | + |
| 136 | +++ | +++ | + |
| 138 | +++ | +++ | + |
| 148 | +++ | +++ | + |
| 151 | +++ | +++ | + |
| 153 | +++ | +++ | + |
| 157 | +++ | +++ | + |
| 189 | ++ | +++ | + |
| 238 | +++ | +++ | + |
| 240 | ++ | ++ | + |
| 244 | +++ | +++ | + |

Proliferation inhibition and COX-2 inhibition:
+++:IC₅₀ <5µM, ++: 5µM<IC₅₀<25 µM, +: IC₅₀ >25µM
ND: not determined

Apoptosis induction (EF) at 10 µM:
+: EF<3, ++: 3<EF<6, +++: EF>6.

The compounds of the present invention are more potent than celecoxib as antiproliferative and pro-apoptotic agents, but much less potent than celecoxib as COX-2 inhibitory agents. These results and the fact that the human cancer cell line HCT-116 does not express COX-2 isoenzyme show that their mechanism of action is independent of their COX-2 inhibitory properties.

## Claims

1. A compound of general formula (I), 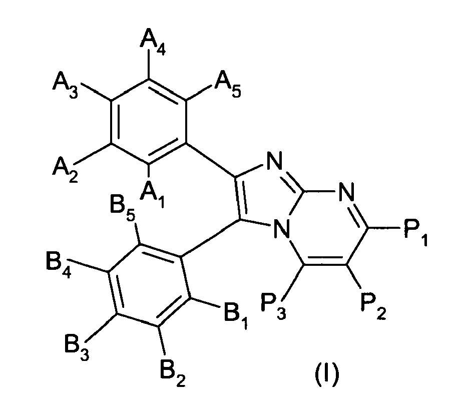 stereoisomers and mixtures thereof, polymorphs and mixtures thereof, and pharmaceutically acceptable solvates and addition salts of them all,
wherein:
and A₁, A₂, A₃, A₄, A₅, B₁, B₂, B₃, B₄ B₅ are radicals independently selected from the group consisting of H, (C₁-C₄)-alkyl, (C₃-C₇)-cycloalkyl, CF₃, OCF₃, CN, (CH₂)ₙOR1, (CH₂)ₙNR1R2, CONR1R2, F, Cl, Br, I, NR1R2, NR2COR1, OR1, COR1, COOR1, COSR1, OCOR1, SR1, SOR1, S(O)OH, SO₂R1, SO₂NR2R3, SO₂NHCOR1, and SCOR1; wherein
n is an integer from 1 to 3;
R1 is a radical selected from H, CH₂OCOR2, CF₃, (C₁-C₄)-alkyl, and (C₃-C₇)-cycloalkylmethyl and (C₃-C₇)-cycloalkyl;
R2 is a radical selected from H, and (C₁-C₄)-alkyl;
R3 is a radical selected from COR1, and SO₂R1;
alternatively, either A₂ and A₃, or B₂ and B₃ may be forming a R4-(C₁-C₃)-alkyl-R5 biradical, wherein R4 and R5 are independently selected from CR1R2, O, NR1, S; and
P₁, P₂, and P₃ are radicals independently selected from the group consisting of H, NR1R2, NR2COR1, CF₃, F, Cl, Br, OH, SH, (C₁-C₄)-alkyl, (C₁-C₄)-alkoxyl and (C₁-C₄)-alkylsulfanyl,
with the proviso that formula (I) does not fulfill any of the following circumstances:
simultaneously B₃ is SO₂NH₂ or SO₂CH₃, A₃, A₄ or A₅ are H, F, Cl, Br, (C₁-C₃)-alkyl, CF₃, (C₁-C₃)-alkoxyl or OCF₃, and P₁ or P₂ are H, CH₃, Cl, Br or CH₃O;
simultaneously B₃ is CH₃O or H, A₃ is CH₃O or H, and P₁, P₂ and P₃ are H; simultaneously B₃ is F, A₃ is SO₂CH₃, and P₁ is methyl;
simultaneously A₁, A₂, A₃, A₄, A₅, B₁, B₂, B₃, B₄, B₅ are H, P₁ is methyl, P₂ is H and P₃ is OH;
simultaneously A₃ and B₃ are CH₃O, A₁, A₂, A₄, A₅, B₁, B₂, B₄ and B₅ are H, and one of the groups P₁, P₂ or P₃ are H, OH, (C₁-C₄)-alkyl or (C₁-C₄)-alkoxyl, being the remaining two groups P₁, P₂ or P₃ representing H; or
simultaneously A₃ and B₃ are CH₃O, A₁, A₂, A₄, A₅, B₁, B₂, B₄ and B₅ are H, P₁ is OH or (C₁-C₄)-alkoxyl, P₂ is H and P₃ is (C₁-C₄)-alkyl.

2. The compound according to claim 1, wherein A₃ and B₃ are radicals selected from H, (C₁-C₄)-alkyl or (C₃-C₇)-cycloalkyl, CF₃, OCF₃, CN, CONR1R2, F, CI, Br, I, NR1R2, NR2COR1, OR1, COR1, COOR1, COSR1, OCOR1, SR1, SOR1, and SCOR1.

3. The compound according to claim 2, wherein B₃ is a radical selected from SR1, and SOR1.

4. The compound according to claim 1 selected from the group consisting of:
2-(4-methoxyphenyl)-3-(4-methylsulfanylphenyl)imidazo[1,2-a]pyrimidine;
2-(4-bromophenyl)-3-(4-methylsulfanylphenyl)imidazo[1,2-*a*]pyrimidine;
2-(4-methoxyphenyl)-7-methyl-3-(4-methylsulfanylphenyl)imidazo[1,2-*a*]pyrimidine;
2-(4-methanesulfonylphenyl)-7-methyl-3-*p*-tolylimidazo[1,2-a]pyrimidine;
3-(3-chloro-4-methylsulfanylphenyl)-2-(4-methylsulfanylphenyl)imidazo[1,2-*a*]pyrimidine;
3-(3-methyl-4-methylsulfanylphenyl)-2-(4-methylsulfanylphenyl)imidazo[1,2-*a*]pyrimidine;
3-(3-chloro-4-methylsulfanylphenyl)-2-(4-methoxyphenyl)imidazo[1,2-*a*]pyrimidine;
3-(3-chloro-4-methylsulfanylphenyl)-2-(4-methoxyphenyl)-7-methylimidazo[1,2-*a*]pyrimidine;
3-(3-bromo-4-methylsulfanylphenyl)-2-m-tolylimidazo[1,2-a]pyrimidine;
3-(3-bromo-4-methylsulfanylphenyl)-2-(4-chlorophenyl)imidazo[1,2-*a*]pyrimidine;
2-(4-methoxyphenyl)-3-(3-methyl-4-methylsulfanylphenyl)imidazo[1,2-*a*]pyrimidine;
3-(3-chloro-4-propylsulfanylphenyl)-2-(4-methoxyphenyl)imidazo[1,2-*a*]pyrimidine;
3-(4-isopropylsulfanylphenyl)-2-(4-methoxyphenyl)imidazo[1,2-a]pyrimidine;
3-(3-chloro-4-isopropylsulfanylphenyl)-2-*p*-tolylimidazo[1,2-a]pyrimidine;
3-(3-chloro-4-isopropylsulfanylphenyl)-2-(4-methylsulfanylphenyl)imidazo[1,2-*a*]pyrimidine; and
3-(3-chloro-4-methanesulfinylphenyl)-2-(4-methoxyphenyl)imidazo[1,2-*a*]pyrimidine.

5. A pharmaceutical composition comprising, as an active ingredient, a therapeutically effective amount of the compound according to any one of the claims 1 to 4 together with appropriate amounts of pharmaceutically acceptable excipients.

6. Use of the compound as defined in any one of claims 1 to 4 for the manufacture of a medicament for the chemoprevention and/or treatment of a precancerous lesion.

7. Use according to claim 6, wherein the precancerous lesion is familial adenomatous polyposis or an actinic keratosis.

8. Use of the compound as defined in any one of claims 1 to 4 for the manufacture of a medicament for the chemoprevention and/or treatment of a cancer.

9. Use according to claim 8, wherein the cancer is colorectal, prostate, breast, bladder, or skin cancer.

10. Use according to any one of the claims 6 to 9, wherein the medicament is administered orally, parenterally or topically.

## Patentansprüche

1. Verbindung der allgemeinen Formel (I), 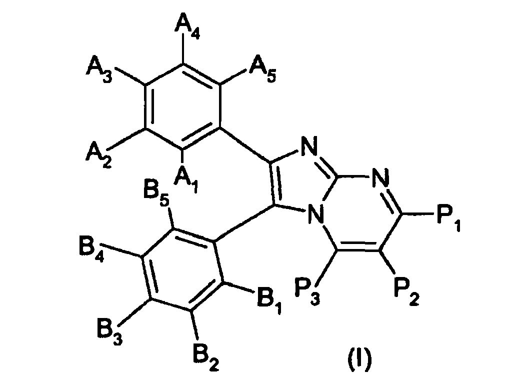 Stereoisomere und Mischungen derselben, Polymorphe und Mischungen derselben und pharmazeutisch akzeptable Solvate und Zusatzsalze aller genannten,
wobei:
A₁, A₂, A₃, A₄, A₅, B₁, B₂, B₃, B₄ und B₅ Radikale sind, die unabhängig aus der Gruppe bestehend aus H, (C₁-C₄)-Alkyl, (C₃-C₇)-Cycloalkyl, CF₃, OCF₃, CN, (CH₂)ₙOR1, (CH₂)ₙNR1R2, CONR1R2, F, Cl, Br, I, NR1R2, NR2COR1, OR1, COR1, COOR1, COSR1, OCOR1, SR1, SOR1, S(O)OH, SO₂R1, SO₂NR2R3, SO₂NHCOR1 und SCOR1 ausgewählt sind; wobei n eine Ganzzahl von 1 bis 3 ist;
R1 ein Radikal ist, das aus H, CH₂OCOR2, CF₃, (C₁-C₄)-Alkyl und (C₃-C₇)-Cycloalkylmethyl und (C₃-C₇)-Cycloalkyl ausgewählt ist;
R2 ein Radikal ist, das aus H und (C₁-C₄)-Alkyl ausgewählt ist;
R3 ein Radikal ist, das aus COR1 und SO₂R1 ausgewählt ist;
wobei als Alternative entweder A₂ und A₃ oder B₂ und B₃ ein R4-(C₁-C₃)-Alkyl-R5-Biradikal bilden kann, wobei R4 und R5 unabhängig aus CR1R2, O, NR1, S ausgewählt sind; und
wobei P₁, P₂ und P₃ Radikale sind, die unabhängig aus der Gruppe bestehend aus H, NR1R2, NR2COR1, CF₃, F, Cl, Br, OH, SH, (C₁-C₄)-Alkyl, (C₁-C₄)-Alkoxyl und (C₁-C₄)-Alkylsulfanyl ausgewählt sind, unter der Voraussetzung, dass die Formel (I) keine der nachstehenden Umstände erfüllt:
gleichzeitig ist B₃ SO₂NH₂ oder SO₂CH₃, A₃, A₄ oder A₅ sind H, F, Cl, Br, (C₁-C₃)-Alkyl, CF₃, (C₁-C₃)-Alkoxyl oder OCF₃ und P₁ oder P₂ sind H, CH₃, Cl, Br oder CH₃O;
gleichzeitig ist B₃ CH₃O oder H, A₃ ist CH₃O oder H und P₁, P₂ und P₃ sind H;
gleichzeitig ist B₃ F, A₃ ist SO₂CH₃ und P₁ ist Methyl;
gleichzeitig sind A₁, A₂, A₃, A₄, A₅, B₁, B₂, B₃, B₄, B₅ H, P₁ ist Methyl, P₂ ist H und P₃ ist OH;
gleichzeitig sind A₃ und B₃ CH₃O, A₁, A₂, A₄, A₅, B₁, B₂, B₄ und B₅ sind H und eine aus den Gruppen P₁, P₂ oder P₃ sind H, OH, (C₁-C₄)-Alkyl oder (C₁-C₄)-Alkoxyl, wobei die restlichen zwei Gruppen P₁, P₂ oder P₃ H repräsentieren; oder
gleichzeitig sind A₃ und B₃ CH₃O, A₁, A₂, A₄, A₅, B₁, B₂, B₄ und B₅ sind H, P₁ ist OH oder (C₁-C₄)-Alkoxyl, P₂ ist H und P₃ ist (C₁-C₄)-Alkyl.

2. Verbindung gemäß Anspruch 1, wobei A₃ und B₃ Radikale sind, die aus der Gruppe H, (C₁-C₄)-Alkyl oder (C₃-C₇)-Cycloalkyl, CF₃, OCF₃, CN, CONR1R2, F, C1, Br, I, NR1R2, NR2COR1, OR1, COR1, COOR1, COSR1, OCOR1, SR1, SOR1 und SCOR1 ausgewählt sind.

3. Verbindung gemäß Anspruch 2, wobei B₃ ein Radikal ist, das aus SR1 und SOR1 ausgewählt ist.

4. Verbindung gemäß Anspruch 1, ausgewählt aus der Gruppe bestehend aus:
2-(4-Methoxyphenyl)-3-(4-methylsulfanylphenyl)imidazo[1,2-a]pyrimidin;
2-(4-Bromphenyl)-3-(4-methylsulfanylphenyl)imidazo[1,2-a]pyrimidin;
2-(4-methoxyphenyl)-7-methyl-3-(4-methylsulfanylphenyl)imidazo[1,2-*a*]pyrimidin;
2-(4-Methansulfonylphenyl)-7-methyl-3-*p*-tolylimidazo[1,2-a]pyrimidin;
3-(3-Chloro-4-methylsulfanylphenyl)-2-(4-methylsulfanylphenyl)imidazo[1,2-*a*]pyrimidin;
3-(3-Methyl-4-methylsulfanylphenyl)-2-(4-methylsulfanylphenyl)imidazo[1,2-*a*]pyrimidin;
3-(3-Chloro-4-methylsulfanylphenyl)-2-(4-methoxyphenyl)imidazo[1,2-*a*]pyrimidin;
3-(3-Chloro-4-methylsulfanylphenyl)-2-(4-methoxyphenyl)-7-methylimidazo[1,2-*a*] pyrimidin;
3-(3-Bromo-4-methylsulfanylphenyl)-2-*m*-tolylimidazo[1,2-*a*]pyrimidin;
3-(3-Bromo-4-methylsulfanylphenyl)-2-(4-chlorophenyl)imidazo[1,2-*a*]pyrimidin;
2-(4-Methoxyphenyl)-3-(3-methyl-4-methylsulfanylphenyl)imidazo[1,2-*a*]pyrimidin;
3-(3-Chloro-4-propylsulfanylphenyl)-2-(4-methoxyphenyl)imidazo[1,2-*a*] pyrimidin;
3-(4-Isopropylsulfanylphenyl)-2-(4-methoxyphenyl)imidazo[1,2-a]pyrimidin;
3-(3-Chloro-4-isopropylsulfanylphenyl)-2-*p*-tolylimidazo[1,2-a]pyrimidin;
3-(3-Chloro-4-isopropylsulfanylphenyl)-2-(4-methylsulfanylphenyl)imidazo[1,2-*a*]pyrimidin; und
3-(3-Chloro-4-methansulfinylphenyl)-2-(4-methoxyphenyl)imidazo[1,2-*a*]pyrimidin.

5. Pharmazeutische Zusammensetzung, die als Wirkstoff eine therapeutisch wirksame Menge der Verbindung gemäß einem der Ansprüche 1 bis 4 zusammen mit geeigneten Mengen pharmazeutisch akzeptabler Trägerstoffe umfasst.

6. Verwendung der Verbindung gemäß einem der Ansprüche 1 bis 4 zur Herstellung eines Medikaments für die Chemoprävention und/oder Behandlung einer präkanzerösen Läsion.

7. Verwendung gemäß Anspruch 6, wobei die präkanzeröse Läsion eine familiäre adenomatöse Polyposis oder eine aktinische Keratose ist.

8. Verwendung der Verbindung gemäß einem der Ansprüche 1 bis 4 zur Herstellung eines Medikaments für die Chemoprävention und/oder Behandlung eines Krebses.

9. Verwendung gemäß Anspruch 8, wobei der Krebs ein Kolorektal-, Prostata-, Brust-, Blasen- oder Hautkrebs ist.

10. Verwendung gemäß einem der Ansprüche 6 bis 9, wobei das Medikament oral, parenteral oder topisch verabreicht wird.

## Revendications

1. Composé de formule générale (I), 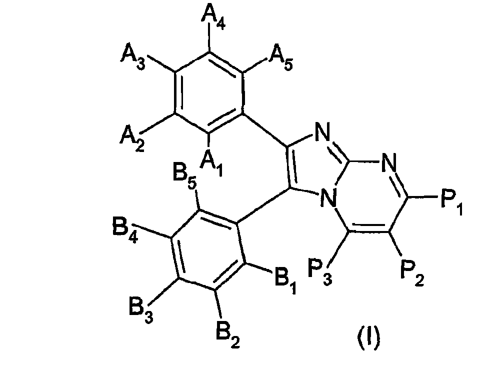 stéréoisomères et mélanges de ceux-ci, polymorphes et mélanges de ceux-ci, et solvates et sels d'addition pharmaceutiquement acceptables de tous ceux-ci,
dans laquelle :
A₁, A₂, A₃, A₄, A₅, B₁, B₂, B₃, B₄ et B₅ sont des radicaux indépendamment choisis dans le groupe constitué par H, un groupe alkyle en C₁-C₄, cycloalkyle en C₃-C₇, CF₃, OCF₃, CN, (CH₂)ₙOR1, (CH₂)ₙNR1R2, CONR1R2, F, C1, Br, I, NR1R2, NR2COR1, OR1, COR1, COOR1, COSR1, OCOR1, SR1, SOR1, S(O)OH, SO₂R1, SO₂NR2R3, SO₂NHCOR1, et SCOR1 ; dans laquelle
n est un nombre entier de 1 à 3 ;
R1 est un radical choisi parmi H, CH₂OCOR2, CF₃, un groupe alkyle en C₁-C₄, et cycloalkylméthyle en C₃-C₇ et cycloalkyle en C₃-C₇ ;
R2 est un radical choisi parmi H, et un groupe alkyle en C₁-C₄ ;
R3 est un radical choisi parmi COR1, et SO₂R1 ;
en variante, soit A₂ et A₃, soit B₂ et B₃ peuvent former un biradical R4- (alkyl en C₁-C₃)-R5, où R4 et R5 sont indépendamment choisis parmi CR1R2, O, NR1, S ; et
P₁, P₂, et P₃ sont des radicaux indépendamment choisis dans le groupe constitué par H, NR1R2, NR2COR1, CF₃, F, Cl, Br, OH, SH, un groupe alkyle en C₁-C₄, alcoxyle en C₁-C₄ et alkyl-sulfanyle en C₁-C₄,
à condition que la formule (I) ne réponde à aucune des circonstances suivantes :
simultanément B₃ est SO₂NH₂ ou SO₂CH₃, A₃, A₄ ou A₅ sont H, F, Cl, Br, un groupe alkyle en C₁-C₃, CF₃, un groupe alcoxyle en C₁-C₃ ou OCF₃, et P₁ ou P₂ sont H, CH₃, CI, Br ou CH₃O ;
simultanément B₃ est CH₃O ou H, A₃ est CH₃O ou H, et P₁, P₂ et P₃ sont H ;
simultanément B₃ est F, A₃ est SO₂CH₃, et P₁ est un groupe méthyle ;
simultanément A₁, A₂, A₃, A₄, A₅, B₁, B₂, B₃, B₄, B₅ sont H, P₁ est un groupe méthyle, P₂ est H et P₃ est OH ;
simultanément A₃ et B₃ sont CH₃O, A₁, A₂, A₄, A₅, B₁, B₂, B₄ et B₅ sont H, et l'un des groupes P₁, P₂ ou P₃ est H, OH, un groupe alkyle en C₁-C₄ ou alcoxyle en C₁-C₄, les deux groupes restants de P₁, P₂ ou P₃ représentant H ; ou
simultanément A₃ et B₃ sont CH₃O, A₁, A₂, A₄, A₅, B₁, B₂, B₄ et B₅ sont H, P₁ est OH ou un alcoxyle en C₁-C₄, P₂ est H et P₃ est un groupe alkyle en C₁-C₄.

2. Composé selon la revendication 1, dans lequel A₃ et B₃ sont des radicaux choisis parmi H, un groupe alkyle en C₁-C₄ ou cycloalkyle en C₃-C₇, CF₃, OCF₃, CN, CONR1R2, F, Cl, Br, I, NR1R2, NR2COR1, OR1, COR1, COOR1, COSR1, OCOR1, SR1, SOR1, et SCOR1.

3. Composé selon la revendication 2, dans lequel B₃ est un radical choisi parmi SR1 et SOR1.

4. Composé selon la revendication 1, choisi dans le groupe constitué par :
la 2-(4-méthoxyphényl)-3-(4-méthylsulfanylphényl)-imidazo[1,2-a]pyrimidine ;
la 2-(4-bromophényl)-3-(4-méthylsulfanylphényl)-imidazo[1,2-a]pyrimidine ;
la 2-(4-méthoxyphényl)-7-méthyl-3-(4-méthyl-sulfanylphényl)imidazo[1,2-*a*]pyrimidine ;
la 2-(4-méthanesulfonylphényl)-7-méthyl-3-p-tolyl-imidazo[1,2-*a*]pyrimidine ;
la 3-(3-chloro-4-méthylsulfanylphényl)-2-(4-méthylsulfanylphényl)imidazo[1,2-*a*]pyrimidine ;
la 3-(3-méthyl-4-méthylsulfanylphényl)-2-(4-méthylsulfanylphényl)imidazo[1,2-*a*]pyrimidine ;
la 3-(3-chloro-4-méthylsulfanylphényl)-2-(4-méthoxyphényl)imidazo[1,2-*a*]pyrimidine ;
la 3-(3-chloro-4-méthylsulfanylphényl)-2-(4-méthoxyphényl)-7-méthylimidazo[1-,2-*a*]pyrimidine ;
la 3-(3-bromo-4-méthylsulfanylphényl)-2-*m*-tolylimidazo[1,2-*a*]pyrimidine ;
la 3-(3-bromo-4-méthylsulfanylphényl)-2-(4-chlorophényl)imidazo[1,2-*a*]pyrimidine ;
la 2-(4-méthoxyphényl)-3-(3-méthyl-4-méthylsulfanylphényl)imidazo[1,2-*a*]pyrimidine ;
la 3-(3-chloro-4-propylsulfanylphényl)-2-(4-méthoxyphényl)imidazo[1,2-*a*]pyrimidine ;
la 3-(4-isopropylsulfanylphényl)-2-(4-méthoxyphényl)imidazo[1,2-*a*]pyrimidine ;
la 3-(3-chloro-4-isopropylsulfanylphényl)-2-p-tolylimidazo[1,2-*a*]pyrimidine ;
la 3-(3-chloro-4-isopropylsulfanylphényl)-2-(4-méthylsulfanylphényl)imidazo[-1,2-*a*]pyrimidine; et
la 3-(3-chloro-4-méthanesulfinylphényl)-2-(4-méthoxyphényl)imidazo[1,2-*a*]pyrimidine.

5. Composition pharmaceutique comprenant, en tant qu'un principe actif, une quantité thérapeutiquement efficace du composé selon l'une quelconque des revendications 1 à 4, conjointement avec des quantités adaptées d'excipients pharmaceutiquement acceptables.

6. Utilisation du composé tel que défini dans l'une quelconque des revendications 1 à 4, pour la fabrication d'un médicament destiné à la chimioprévention et/ou au traitement d'une lésion précancéreuse.

7. Utilisation selon la revendication 6, dans laquelle la lésion précancéreuse est la polypose adénomateuse familiale ou une kératose actinique.

8. Utilisation du composé tel que défini dans l'une quelconque des revendications 1 à 4, pour la fabrication d'un médicament destiné à la chimioprévention et/ou au traitement d'un cancer.

9. Utilisation selon la revendication 8, dans laquelle le cancer est le cancer colorectal, de la prostate, du sein, de la vessie, ou de la peau.

10. Utilisation selon l'une quelconque des revendications 6 à 9, dans laquelle le médicament est administré par voie orale, parentérale ou topique.
